# EUROPEAN PATENT APPLICATION

(11) **EP 4 108 659 A1**
(43) Date of publication of application: **28.12.2022**
(21) Application number: 21212393.9
(22) Date of filing: 07.06.2017
(51) Int. Cl.: C07D 401/14, C07D 405/14, C07D 413/14, C07D 401/04, C07D 401/12, C07D 471/04, C07D 471/20, C07D 491/107, C07D 519/00, A61K 31/506, A61K 31/537, A61P 35/00, A61P 9/00, A61P 19/00, A61P 37/00

(54) **NOVEL HETEROCYCLIC DERIVATIVES USEFUL AS SHP2 INHIBITORS**

(30) Priority: 07.06.2016 WO PCT/CN2016/085122
(62) Divisional of application: 17809742.4
(71) Applicant: Jacobio Pharmaceuticals Co., Ltd., Beijing 101111 (CN)
(72) Inventor: MA, Cunbo, Daxing, 101111 (CN); GAO, Panliang, Daxing, 101111 (CN); CHU, Jie, Daxing, 101111 (CN); WU, Xinping, Daxing, 101111 (CN); WEN, Chunwei, Daxing, 101111 (CN); KANG, Di, Daxing, 101111 (CN); BAI, Jinlong, Daxing, 101111 (CN); PEI, Xiaoyan, Daxing, 101111 (CN)
(74) Representative: Arch, Peter Jonathan Sanders

(57) **Abstract**

Provided are certain pyrazine derivatives (I) as SHP2 inhibitors which is shown as formula (I), their synthesis and their use for treating a SHP2 medicated disorder. More particularly, provided are fused heterocyclic derivatives useful as inhibitors of SHP2, methods for producing such compounds and methods for treating a SHP2-medicated disorder.

## Description

### Technical Field

This invention relates to certain novel pyrazine derivatives (Formula I) as SHP2 inhibitors which is shown as formula I, their synthesis and their use for treating a SHP2 mediated disorder. More particularly, this invention is directed to fused heterocyclic derivatives useful as inhibitors of SHP2, methods for producing such compounds and methods for treating a SHP2-mediated disorder.

### Background Art

SHP2 (The Src Homolgy-2 phosphatease) is a non-receptor protein tyrosine phosphatase encoded by the PTPN11 gene that harbors a classical tyrosine phosphatase domain and two N-terminal Src homology 2 (SH2) domains and a C-terminal tail. The two SH2 domains control the subcellular localization and functional regulation of SHP2. In its inactive state, the N-terminal SH2 domain blocks the PTP domain and this autoinhibition is relieved by binding of the SH2 domains to specific phosphotyrosine sites on receptors or receptor-associated adaptor proteins. The stimulation, for example, by cytokines or growth factors leads to exposure of the catalytic site resulting in enzymatic activation of SHP2.

SHP2 is widely expressed and participated in multiple cell signaling processes, such as the Ras-Erk, PI3K-Akt, Jak-Stat, Met, FGFR, EGFR, and insulin receptors and NF-kB pathways, in which plays an important role in proliferation, differentiation, cell cycle maintenance and migration.

The hyperactivation of SHP2 catalytic activity caused by either germline or somatic mutations in PTPN11 has been identified in patients with Noonan syndrome, Leopard syndrome, juvenile myelomonocytic leukemias, myelodysplastic syndrome, B cell acute lymphoblastic leukemia/lymphoma, and acute myeloid leukemia. In addition, activating mutations of PTPN11 have been found in solid tumors as well, such as lung cancer, colon cancer, melanoma, neuroblastoma, and hepatocellular carcinoma. Therefore, the presence of the activated or up-regulated SHP2 protein in human cancers and other disease make SHP2 an excellent target for development of novel therapies. The compounds of the present invention fulfill the need of small molecules in order to inhibit the activity of SHP2.

### Summary of Invention

The present invention relates to heterocyclic pyrazine compounds useful as SHP2 inhibitors and for the treatment of conditions mediated by SHP2. The compounds of the invention have the general structure as Formula I or a pharmaceutically acceptable salt: 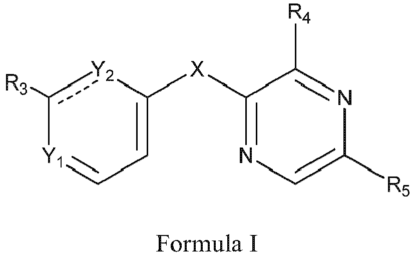 and
X is absent, O, S, SO, S(O)₂, C(O), C(O)R₁₁, CR₁₁R₁₂, or -NR₁₁; and each R₁₁ and R₁₂ is independently -H, halogen, -NH₂, -CN, -OH, -NO₂, carbonyl, =O, oxo, carboxyl, substituted or unsubstituted C₁₋₆alkoxy, or substituted or unsubstituted C₁₋₆alkyl;
Y₁ is Nor CR₁;
Y₂ is N or CR₂;
each R₁ and R₂ is independently -H, halogen, -CN, -OH, -NH₂, -N₃, -NO₂, substituted or unsubstituted C₁₋₆alkoxy, or substituted or unsubstituted C₁₋₆alkyl; or
R₁ combines with R₃, or R₂ combines with R₃, to form a 5-10 member heteroaryl, 5-10 member carbocyclic or 5-10 member heterocyclic ring, wherein each of the ring systems is independently optionally substituted with halogen, -CN, -OH, -NR₈R₉, -N₃, -NO₂, carbonyl, =O, oxo, substituted or unsubstituted C₁₋₆alkyl, substituted or unsubstituted C₁₋₆alkoxy, or C(O)R₈; or
R₃ is -H, halogen, -CN, -OH, -N₃, -NO₂, -NR₈R₉, -N(R₈)(CH₂)ₚNR₈R₉, -N(R₈)(CH₂)ₚR₈, -N(R₈)GₚR₈, -N(R₈)GₚNR₈R₉, -N(R₈)(C=O)_{q}R₈, -N(R₈)(C=O)_{q}NR₈R₉, -N(R₈)(C=O)_{q}GₚR₈, -N(R₈)(C=O)_{q}GₚNR₈R₉, -N(R₈)(C=O)_{q}Gₚ(C=O)_{q}R₈, -N(R₈)(C=O)_{q}Gₚ(C=O)_{q}NR₈R₉, -N(R₈) (C=O)_{q}N(R₈)(C=O)_{q}R₈, -N(R₈)(C=O)_{q}N(R₈)(C=O)_{q}NR₈R₉, -N(R₈)(C=O)_{q}N(R₈)G_{q}(C=O)ₚR₈, -N(R₈)(C=O)_{q}N(R₈)Gₚ(C=O)_{q}NR₈R₉, C(O)_{q}R₈, C(O)OR₈, C(O)NH₂, C(O)NHR₈, C(O)NR₈R₉, C₁₋₆alkyl, C₆₋₁₀aryl, arylalkyl, alkoxy, heteroaryl, heterocyclic, or carbocyclic; and each of which may be optionally substituted; and each p and q is independently 0, 1, 2 or 3;
each G is independently C₆₋₁₀ary, C₃₋₈carbocyclic or C₅₋₁₀heteroaryl; and each of which may be optionally substituted;
R₄ is -H, halogen, -CN, -OH, -NR₈R₉, -N₃, -NO₂, substituted or unsubstituted C₁₋₆alkoxy, substituted or unsubstituted C₁₋₆alkyl, C₅₋₁₈heterocyclic or C₅₋₁₈carbocyclic; wherein each of the ring systems is independently optionally substituted with halogen, -CN, -OH, -NO₂, carbonyl, =O, oxo, substituted or unsubstituted C₁₋₆alkyl, substituted or unsubstituted C₁₋₆alkoxy, -NR₈R₉, or -CH₂NR₈R₉;
R₅ is -H, halogen, -CN, -OH, -NR₈R₉, -N₃, -NO₂, C₁₋₆alkyl, C₁₋₆alkoxy, C₆₋₁₀aryl, C₆₋₁₀arylalkyl, C₆₋₁₀heteroaryl, C₅₋₁₈heterocyclic or C₅₋₁₈carbocyclic; and each of which is independently optionally substituted;
each R₈ and R₉ is independently -H, halogen, -CN, -OH, -N₃, -NO₂, C₁₋₆alkyl, C₁₋₆alkoxy, C₂₋₆alkenyl, NH(C₁₋₆alkyl), N(C₁₋₆alkyl)₂, C₅₋₁₀heterocyclic or C₅₋₁₀carbocyclic; and each of which may be independently optionally substituted.

The present invention further provides some preferred technical solutions with regard to compound of Formula (I).

In some embodiments of Formula (I), R₁ is -H, -F, -Cl, -Br, -I, -CN, -OH, -NH₂, substituted or unsubstituted C₁₋₃alkoxy, or substituted or unsubstituted C₁₋₃alkyl.

In some embodiments of Formula (I), R₁ is -H, -F, -Cl, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.

In some embodiments of Formula (I), R₂ is -H, -F, -Cl, -Br, -I, -CN, -OH, -NH₂, substituted or unsubstituted C₁₋₃alkoxy, or substituted or unsubstituted C₁₋₃alkyl.

In some embodiments of Formula (I), R₂ is -H, -F, -Cl, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.

In some embodiments of Formula (I), R₃ is -H, -F, -Cl, -Br, -CN, -OH, -NO₂, -NR₈R₉, -N(R₈)(CH₂)ₚNR₈R₉, -N(R₈)(CH₂)ₚR₈, -N(R₈)GₚR₈, -N(R₈)GₚNR₈R₉, -N(R₈)(C=O)_{q}R₈, -N(R₈)(C=O)_{q}NR₈R₉, -N(R₈)(C=O)_{q}GₚR₈, -N(R₈)(C=O)_{q}GₚNR₈R₉, -N(R₈)(C=O)_{q}Gₚ(C=O)_{q}R₈, -N(R₈)(C=O)_{q}Gₚ(C=O)_{q}NR₈R₉, -N(R₈)(C=O)_{q}N(R₈)(C=O)_{q}R₈, -N(R₈)(C=O)_{q}N(R₈) (C=O)_{q}NR₈R₉, -N(R₈)(C=O)_{q}N(R₈)G_{q}(C=O)ₚR₈, -N(R₈)(C=O)_{q}N(R₈)Gₚ(C=O)_{q}NR₈R₉, C(O)_{q}R₈, C(O)OR₈, C(O)NH₂, C(O)NHR₈, C(O)NR₈R₉, C₁₋₆alkyl or C₆₋₁₀aryl; and each of which may be optionally substituted with halogen, -CN, -OH, -NH₂, -N₃, -NO₂, substituted or unsubstituted C₁₋₆alkyl, or substituted or unsubstituted C₁₋₆alkoxy.

In some embodiments of Formula (I), R₃ is -H, -F, -Cl, -Br, -NR₈R₉, -N(R₈)(CH₂)ₚNR₈R₉, -N(R₈)(CH₂)ₚR₈, -N(R₈)GₚR₈, -N(R₈)GₚNR₈R₉, -N(R₈)(C=O)_{q}R₈, -N(R₈)(C=O)_{q}NR₈R₉, -N(R₈)(C=O)_{q}GₚR₈, -N(R₈)(C=O)_{q}GₚNR₈R₉, -N(R₈)(C=O)_{q}Gₚ(C=O)_{q}R₈, -N(R₈) (C=O)_{q}Gₚ(C=O)_{q}NR₈R₉, -N(R₈)(C=O)_{q}N(R₈)(C=O)_{q}R₈, -N(R₈)(C=O)_{q}N(R₈) (C=O)_{q}NR₈R₉, -N(R₈)(C=O)_{q}N(R₈)G_{q}(C=O)_{P}R₈, -N(R₈)(C=O)_{q}N(R₈)Gₚ(C=O)_{q}NR₈R₉ or C(O)_{q}R₈, and each of which may be optionally substituted with -F, -Cl, -Br, -NHmethyl, -NHethyl, -NHpropyl, -NHisopropyl, -NHOCH₃, -NHOCH₂CH₃, -NHCH₂OCH₃, -NHOCH₂CH₂CH₃, -NHCH₂OCH₂CH₃, -NHCH₂CH₂OCH₃, -NHOCH(CH₃)₂, -NHCH(OCH₃)₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with -F, -Cl, -Br or -I.

In some embodiments of Formula (I), each G is independently 6-membered aryl, 7-membered aryl, 8-membered aryl, 3-membered carbocyclic, 4-membered carbocyclic, 5-membered carbocyclic, 6-membered carbocyclic or 7-membered carbocyclic; and each of which may be optionally substituted with halogen, -CN, -OH, -NH₂, -N₃, -NO₂, substituted or unsubstituted C₁₋₆alkyl, or substituted or unsubstituted C₁₋₆alkoxy.

In some embodiments of Formula (I), each G is independently 6-membered aryl, 7-membered aryl, 8-membered aryl, 3-membered carbocyclic, 4-membered carbocyclic, 5-membered carbocyclic or 6-membered carbocyclic; and each of which may be optionally substituted with -F, -Cl, -Br, -CN, -OH, -NH₂, -NO₂, substituted or unsubstituted C₁₋₃alkyl, or substituted or unsubstituted C₁₋₃alkoxy.

In some embodiments of Formula (I), each G is independently phenyl, and which may be optionally substituted with -F, -Cl, -OH, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with -F, -Cl, -Br or -I.

In some embodiments of Formula (I), each G is independently 5-membered heteroaryl, 6-membered heteroaryl, 7-membered heteroaryl or 8-membered heteroaryl; and each of which contains 1, 2, 3 or 4 heteroatoms select from N, O or S, and may be optionally substituted with halogen, -CN, -OH, -NH₂, -N₃, -NO₂, substituted or unsubstituted C₁₋₆alkyl, or substituted or unsubstituted C₁₋₆alkoxy.

In some embodiments of Formula (I), p is 0 or 1.

In some embodiments of Formula (I), q is 1 or 2.

In some embodiments of Formula (I), R₃ is -NH₂,

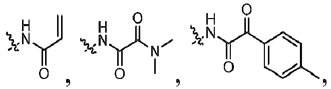

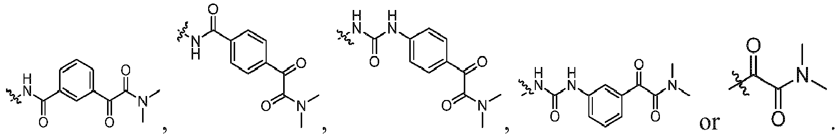

In some embodiments of Formula (I), R₁ is combines with R₃, or R₂ is combines with R₃, to form a 5-10 member heteroaryl or 5-10 member heterocyclic ring, wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy, or C(O)R₈.

In some embodiments of Formula (I), R₁ is combines with R₃, or R₂ is combines with R₃, to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring; wherein each of the ring systems contains 1, 2, 3 or 4 heteroatoms select from N, O or S, and is optionally substituted with -F, -Cl, -Br, -I, -CN, -OH, -NH₂, carbonyl, =O, oxo, substituted or unsubstituted C₁₋₃alkyl, substituted or unsubstituted C₁₋₃alkoxy, or C(O)R₈.

In some embodiments of Formula (I), R₁ is combines with R₃ to form a 5-10 member heteroaryl or a 5-10 member heterocyclic ring; wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy, or C(O)R₈.

In some embodiments of Formula (I), R₁ is combines with R₃ to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms select from N, O or S, and is optionally substituted with -F, -Cl, -Br, -I, -CN, -OH, -NH₂, carbonyl, =O, oxo, substituted or unsubstituted C₁₋₃alkyl, substituted or unsubstituted C₁₋₃alkoxy, or C(O)R₈.

In some embodiments of Formula (I), R₁ is combines with R₃ to form a 5-membered heteroaryl, 6-membered heteroaryl, 5-membered heterocyclic ring or 6-membered heterocyclic, wherein each of the ring systems contains 1, 2 or 3 heteroatoms select from N, O or S, and is optionally substituted with -F, -Cl, -Br, -I, -CN, -OH, -NH₂, -NHmethyl, -NHethyl, -NHpropyl, -NHisopropyl, -NHOCH₃, carbonyl, =O, oxo, methyl, ethyl, propyl, isopropyl, methoxy, CHF₂, CH₂F, CF₃ or C(O)R₈; and each methyl, ethyl, propyl, isopropyl, methoxy, CHF₂, CH₂F or C(O)R₈ is independently optionally substituted with -F, -Cl, -Br or -I.

In some embodiments of Formula (I), R₂ is combines with R₃ to form a 5-10 member heteroaryl or 5-10 member heterocyclic ring, wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy or C(O)R₈.

In some embodiments of Formula (I), R₂ is combines with R₃ to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms select from N, O or S, and is optionally substituted with -F, -Cl, -Br, -I, -CN, -OH, -NH₂, carbonyl, =O, oxo, substituted or unsubstituted C₁₋₃alkyl, substituted or unsubstituted C₁₋₃alkoxy, or C(O)R₈.

In some embodiments of Formula (I), R₄ is -H, -F, -Cl, -Br, -CN, -OH, -NR₈R₉, substituted or unsubstituted C₁₋₆alkyl, substituted or unsubstituted C₁₋₆alkoxy, C₅₋₁₈heterocyclic or C₅₋₁₀carbocyclic, wherein each of the ring systems is optionally substituted with -F, -Cl, -Br, -CN, -OH, -NO₂, carbonyl, =O, oxo, substituted or unsubstituted C₁₋₆alkyl, -NH-C₁₋₆alkyl, -NH-C₁₋₆alkoxy, -C₁₋₆alkylene-NH₂, or -C₁₋₆alkylene-NH-C₁₋₆alkyl.

In some embodiments of Formula (I), R₄ is H, -F, -Cl, -CN, -OH, -NH₂, substituted or unsubstituted C₁₋₃alkyl, substituted or unsubstituted C₁₋₃alkoxy, 5-membered heterocyclic containing 1, 2 or 3 heteroatoms select from N or O, 6-membered heterocyclic containing 1, 2 or 3 heteroatoms select from N or O, 5-membered carbocyclic or 6-membered carbocyclic; wherein each of the ring systems is optionally substituted with -F, -Cl, -Br, -CN, -OH, carbonyl, =O, oxo, substituted or unsubstituted C₁₋₃alkyl, substituted or unsubstituted C₁₋₃alkoxy, -NH₂, -NH-C₁₋₃alkyl, -NH-C₁₋₃alkoxy, -C₁₋₃alkylene-NH₂ or -C₁₋₃alkylene-NH-C₁₋₃alkyl.

In some embodiments of Formula (I), R₄ is -Cl, -NH₂, methyl or piperidinyl, wherein the ring system is optionally substituted with methyl, -NH₂ or -CH₂NH₂.

In some embodiments of Formula (I), R₅ is -H, -F, -Cl, -Br, -I, -NR₈R₉, C₁₋₃alkyl, C₁₋₃alkoxy, C₆₋₉aryl, C₆₋₉arylalkyl, C₆₋₉heteroaryl, C₆₋₁₇heterocyclic or C₆₋₁₇carbocyclic, wherein each of which is independently optionally substituted with halogen, -CN, -OH, -N₃, -NO₂, -NH₂, carbonyl, =O, oxosubstituted or unsubstituted C₁₋₆alkyl, substituted or unsubstituted C₁₋₆alkoxy, substituted or unsubstituted (CH₂)ₖNR₈R₉, substituted or unsubstituted (CH₂)ₖNHC(O)OR₈ or C(O)R₈; and k is 0, 1 or 2.

In some embodiments of Formula (I), the C₆₋₉heteroaryl contains 1, 2, 3 or 4 heteroatoms select from N, O or S, and the C₆₋₉heteroaryl is 6-membered heteroaryl, 7-membered heteroaryl, 8-membered heteroaryl or 9-membered heteroaryl; the C₆₋₁₇heterocyclic contains 1, 2, 3, 4, 5, or 6 heteroatoms select from N, O, or S, and the C₆₋₁₇heterocyclic is 6-membered heterocyclic, 7-membered heterocyclic, 8-membered heterocyclic, 9-membered heterocyclic, 10-membered heterocyclic, 11-membered heterocyclic, 12-membered heterocyclic, 13-membered heterocyclic, 14-membered heterocyclic, 15-membered heterocyclic, 16-membered heterocyclic or 17-membered heterocyclic.

In some embodiments of Formula (I), R₅ is -F, -Cl, -Br, -NR₈R₉, methyl, ethyl, propyl, isopropyl, methoxy, ethoxy, propoxy, isopropoxy, 6-membered aryl, 7-membered aryl, 8-membered aryl, 9-membered aryl, 6-membered arylalkyl, 7-membered arylalkyl, 8-membered arylalkyl, 9-membered arylalkyl, 6-membered heteroaryl, 7-membered heteroaryl, 8-membered heteroaryl, 9-membered heteroaryl, 6-membered heterocyclic, 7-membered heterocyclic, 8-membered heterocyclic, 9-membered heterocyclic 10-membered heterocyclic, 11-membered heterocyclic, 12-membered heterocyclic, 13-membered heterocyclic, 14-membered heterocyclic, 15-membered heterocyclic, 16-membered heterocyclic, 6-membered carbocyclic, 7-membered carbocyclic, 8-membered carbocyclic, 9-membered carbocyclic, 10-membered carbocyclic, 11-membered carbocyclic, 12-membered carbocyclic, 13-membered carbocyclic, 14-membered carbocyclic, 15-membered carbocyclic or 16-membered carbocyclic; and each heteroaryl contains 1, 2 or 3 heteroatoms select from N, O or S, and each heterocyclic contains 1, 2, 3, or 4 heteroatoms select from N, O or S; wherein each of which is independently optionally substituted with -F, -Cl, -Br, -I, -CN, -OH, -NO₂, -NH₂, carbonyl, =O, oxo, substituted or unsubstituted C₁₋₃alkyl, substituted or unsubstituted C₁₋₃alkoxy, substituted or unsubstituted (CH₂)ₖNR₈R₉, substituted or unsubstituted (CH₂)ₖNHC(O)OR₈, or substituted or unsubstituted C(O)R₈; and k is 0, 1 or 2.

In some embodiments of Formula (I), R₅ is 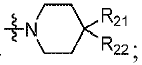
each R₂₁ and R₂₂ is independently halogen, C₁₋₃alkyl, -NH₂, -C₁₋₃alkylene-NH₂, -C₁₋₃alkylene-NH-C₁₋₃alkyl, -C₁₋₃alkylene-N(C₁₋₃alkyl)₂, -NHBoc or -CH₂NHBoc;
or R₂₁ and R₂₂ together with the carbon atom to which they are both attached form a 5-10 member heteroaryl, 5-10 member carbocyclic or a 5-10 member heterocyclic ring, wherein each of the ring system is optionally substituted with halogen, -CN, -OH, carbonyl, =O, oxo, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH₂, C₁₋₃alkoxy or C₁₋₃alkyl.

In some embodiments of Formula (I), R₂₁ and R₂₂ together with the carbon atom to which they are both attached form a 5-membered heteroaryl, 6-membered heteroaryl, 7-membered heteroaryl, 8-membered heteroaryl, 9-membered heteroaryl, 10-membered heteroaryl, 5-membered heterocyclic ring, 6-membered heterocyclic ring, 7-membered heterocyclic ring, 8-membered heterocyclic ring, 9-membered heterocyclic ring or 10-membered heterocyclic ring; wherein each of the ring system contains 1, 2 or 3 heteroatoms select from N, O or S, and is independently optionally substituted with halogen, -CN, -OH, carbonyl, =O, oxo, -NH₂, C₁₋₃alkoxy or C₁₋₃alkyl.

In some embodiments of Formula (I), X is O, S or absent.

In some embodiments of Formula (I), Y₁ is N and Y₂ is CR₂.

In some embodiments of Formula (I), Y₂ is N and Y₁ is CR₁.

In some embodiments of Formula (I), R₄ is -NH₂. In some embodiments of Formula (I), R₅ is -NH₂,

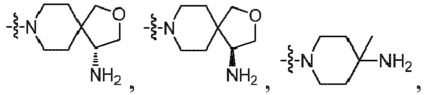

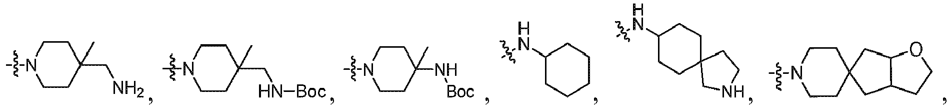

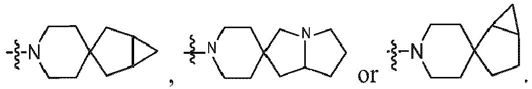

In some embodiments of Formula (I), each R₈ and R₉ is independently -H, halogen, CN, -OH, -NO₂, -NH₂, -C₁₋₆alkylene-NH₂, -C₁₋₆alkylene-NH-C₁₋₆alkyl, -C₁₋₆alkylene-N(C₁₋₆alkyl)₂, -NHBoc, -CH₂NHBoc; -NH-C₁₋₆alkyl, -N(C₁₋₆alkyl)₂, -NH-C₁₋₆alkoxy, -N(C₁₋₆alkoxy)₂, C₁₋₆alkyl, C₁₋₆alkoxy, C₂₋₆alkenyl, C₂₋₆alkylnyl, C₅₋₁₀heterocyclic or C₅₋₁₀carbocyclic; each of which may be optionally substituted.

In some embodiments of Formula (I), each R₈ and R₉ is independently -H, -F, -Cl, -CN, -OH, -NO₂, -NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, C₁₋₄alkyl, C₁₋₃alkoxy, C₂₋₃alkenyl, C₂₋₃alkylnyl, 5-membered heterocyclic, 6-membered heterocyclic, 7-membered heterocyclic, 8-membered heterocyclic, 9-membered heterocyclic, 10-membered heterocyclic, 5-membered carbocyclic, 6-membered carbocyclic, 7-membered carbocyclic, 8-membered carbocyclic, 9-membered carbocyclic or 10-membered carbocyclic; and each of which may be independently optionally substituted with halogen, -CN, -OH, -NH₂, -N₃, -NO₂, -C₁₋₆alkylene-NH₂, -C₁₋₆alkylene-NH-C₁₋₆alkyl, -C₁₋₆alkylene-N(C₁₋₆alkyl)₂, -NHBoc, -CH₂NHBoc; -NH-C₁₋₆alkyl, -N(C₁₋₆alkyl)₂, -NH-C₁₋₆alkoxy, -N(C₁₋₆alkoxy)₂, substituted or unsubstituted C₁₋₆alkyl, or substituted or unsubstituted C₁₋₆alkoxy; and each heterocyclic contains 1, 2, 3 or 4 heteroatoms select from N, O or S.

In some embodiments of Formula (I), each R₈ and R₉ is independently -H, methyl, tert-butyl, -CH=CH₂, N(CH₃)₂,

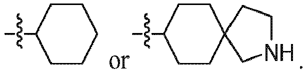

In some embodiments of Formula (I), the compound is of Formula II: 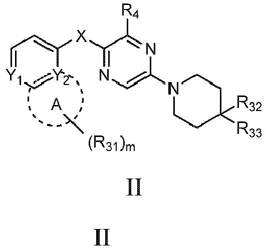 and
X is absent or S;
Y₁ is N or CR₂₅;
Y₂ is N or C;
R₂₅ is H, halogen, C₁₋₃alkyl, C₁₋₃alkoxy, C₂₋₃alkenyl or C₂₋₃alkylnyl;
ring 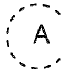 is 5-8 member heteroaryl containing 1,2,3 or 4 heteroatoms select form N, O or S, 5-8 member carbocyclic or 5-8 member heterocyclic ring containing 1,2,3 or 4 heteroatoms select form N, O or S;
R₃₁ is -H, halogen, -OH, -NH₂, -(C=O)C₁₋₃alkyl, -CN, -NO₂, carbonyl, =O, oxo, carboxyl, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy;
m is 0, 1, 2, 3 or 4;
R₄ is -H, halogen, -NH₂, substituted or unsubstituted C₁₋₃alkoxy, or substituted or unsubstituted C₁₋₃alkyl;
each R₃₂ and R₃₃ is independently -H, halogen, -OH, -NH₂, -CN, -NO₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy;
or R₃₂ and R₃₃ together with the carbon atom to which they are both attached form a 5-8 member heteroaryl containing 1, 2 or 3 heteroatoms select from N, O or S, or 5-8 member heterocyclic ring containing 1, 2 or 3 heteroatoms select from N, O or S, wherein each of the ring systems is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy.

The present invention further provides some preferred technical solutions with regard to compound of Formula (II).

In some embodiments of Formula (II), ring 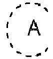 is 5-membered heteroaryl, 6-membered heteroaryl, 7-membered heteroaryl, 5-membered heterocyclic ring, 6-membered heterocyclic ring, 7-membered heterocyclic ring, 5-membered carbocyclic, 6-membered carbocyclic, 7-membered carbocyclic or 8-membered carbocyclic; and each the ring systems contains 1, 2 or 3 heteroatoms select form N, O or S.

In some embodiments of Formula (II), ring 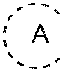 is 5-membered heterocyclic ring containing 1, 2 or 3 heteroatoms select form N or O, 6-membered heterocyclic ring containing 1 or 2 heteroatoms select form N or O or 5-membered carbocyclic.

In some embodiments of Formula (II), R₃₁ is -F, -COCH₃, carbonyl, =O, oxo, -CH₃ or -CF₃.

In some embodiments of Formula (II), R₃₂ and R₃₃ together with the carbon atom to which they are both attached to form a 5-membered heteroaryl, 6-membered heteroaryl, 7-membered heteroaryl, 5-membered heterocyclic ring, 6-membered heterocyclic ring, or 7-membered heterocyclic ring; wherein each of the ring systems contains 1,2, or 3 heteroatoms select from N, O or S, and is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy.

In some embodiments of Formula (II), R₃₂ and R₃₃ together with the carbon atom to which they are both attached form a 5-membered heterocyclic ring; 6-membered heterocyclic ring or 7-membered heterocyclic ring; wherein each of the ring system containing 1 or 2 heteroatoms independently select from O or N, and is optionally substituted with halogen, -CN, -OH, -NH₂, carbonyl, =O, oxo, carboxyl, -N₃, -NO₂, C₁₋₃alkyl or C₁₋₃alkoxy.

In some embodiments of Formula (II), R₃₂ and R₃₃ together with the carbon atom to which they are both attached to form a 5-membered heterocyclic ring; and the heterocyclic ring contains 1 heteroatoms selected from O or N, and is optionally substituted with -F, -Cl, -OH, -NH₂, carbonyl, =O, oxo, methyl or methoxy.

In some embodiments of Formula (II), each R₃₂ and R₃₃ is independently -CH₂NH₂, -CH₂NHBoc or methyl.

In some embodiments of Formula (II), Y₁ is N.

In some embodiments of Formula (II), Y₂ is C.

In some embodiments of Formula (II), is -H or -Cl.

In some embodiments of Formula (II), R₄ is -NH₂.

In some embodiments of Formula (I), the compound is of Formula III: 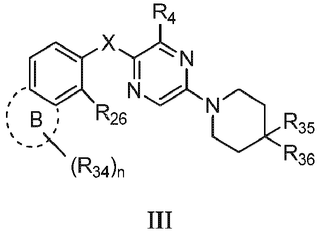 and
X is absent or S;
R₂₆ is -H, halogen, substituted or unsubstituted C₁₋₃alkyl, or substituted or unsubstituted C₁₋₃alkoxy;
ring 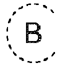 is 5-8 member heteroaryl or 5-8 member heterocyclic ring; and each the ring system independently contains 1,2,3 or 4 heteroatoms select form N, O or S;
R₃₄ is -H, halogen, -OH, -NR₃₅R₃₆, -CN, -NO₂, carbonyl, =O, oxo, substituted or unsubstituted C₁₋₃alkyl, or substituted or unsubstituted C₁₋₃alkoxy;
n is 0, 1, 2 or 3_{;}
R₄ is -H, halogen, -NH₂, ubstituted or unsubstituted C₁₋₆alkoxy, or substituted or unsubstituted C₁₋₆ alkyl;
each R₃₅ and R₃₆ is independently -H, halogen, -OH, -NH₂, -CN, -NO₂, -CH₂NH₂, substituted or unsubstituted C₁₋₃alkyl, or substituted or unsubstituted C₁₋₃alkoxy;
or R₃₅ and R₃₆ together with the carbon atom to which they are both attached to form a 5-8 member heteroaryl or 5-8 member heterocyclic ring, wherein each of the ring system independently contains 1, 2 or 3 heteroatoms select from N, O or S, and is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NHC₁₋₃alkoxy, -N(C₁₋₃alkoxy)₂, substituted or unsubstituted C₁₋₃alkyl, or substituted or unsubstituted C₁₋₃alkoxy.

The present invention further provides some preferred technical solutions with regard to compound of Formula (III).

In some embodiments of Formula (III), ring 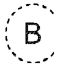 is 5-membered heteroaryl, 6-membered heteroaryl, 7-membered heteroaryl, 5-membered heterocyclic ring, 6-membered heterocyclic ring or 7-membered heterocyclic ring; and each of the ring system independently contains 1, 2 or 3 heteroatoms select form N, O or S.

In some embodiments of Formula (III), ring 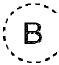 is 5-membered heterocyclic ring or 6-membered heterocyclic ring; and each of the ring system independently contains 1 or 2 heteroatoms select form N or O.

In some embodiments of Formula (III), R₃₅ and R₃₆ together with the carbon atom to which they are both attached to form a 5-membered heteroaryl, 6-membered heteroaryl, 7-membered heteroaryl, 5-membered heterocyclic ring, 6-membered heterocyclic ring or 7-membered heterocyclic ring; wherein each of the ring system is independently contains 1,2, or 3 heteroatoms select from N, O or S, and is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NHC₁₋₃alkoxy, -N(C₁₋₃alkoxy)₂, substituted or unsubstituted C₁₋₃alkyl, or substituted or unsubstituted C₁₋₃alkoxy.

In some embodiments of Formula (III), R₃₅ and R₃₆ together with the carbon atom to which they are both attached to form a 5-membered heterocyclic ring; 6-membered heterocyclic ring or 7-membered heterocyclic ring; wherein each of the ring system is independently contains 1 or 2 heteroatoms independently select from O or N, and is optionally substituted with halogen, -CN, -OH, -NH₂, carbonyl, =O, oxo, carboxyl, -N₃, -NO₂, C₁₋₃alkyl or C₁₋₃alkoxy.

In some embodiments of Formula (III), R₃₅ and R₃₆ together with the carbon atom to which they are both attached to form a 5-membered heterocyclic ring; wherein the ring system contains 1 heteroatom independently select from O or N, and is optionally substituted with -F, -Cl, -OH, -NH₂, carbonyl, =O, oxo, methyl or methoxy.

In some embodiments of Formula (III), R₂₆ is -H or -Cl

In some embodiments of Formula (III), R₄ is -NH₂.

In some embodiments of Formula (III), R₃₄ is -F, -COCH₃, carbonyl, =O, oxo, -CH₃ or -CF₃.

In some embodiments of Formula (I), Formula (II) or Formula (III), each substituted or unsubstituted C₁₋₆alkyl is independently C₁₋₆alkyl, or C₁₋₆alkyl substituted with halogen, -OH, -CN, NH₂, -NO₂, carbonyl, =O, oxo, -C₁₋₆alkylene-NH₂, -C₁₋₆alkylene-NH-C₁₋₆alkyl, or -C₁₋₆alkylene-N(C₁₋₆alkyl)₂; each substituted or unsubstituted C₁₋₆alkoxy is independently C₁₋₆alkoxy, or C₁₋₆alkoxy substituted with halogen, -OH, -CN, NH₂, -NO₂, carbonyl, =O, oxo, -C₁₋₆alkylene-NH₂, -C₁₋₆alkylene-NH-C₁₋₃alkyl, or -C₁₋₆alkylene-N(C₁₋₆alkyl)₂.

In some embodiments of Formula (I), Formula (II) or Formula (III), each substituted or unsubstituted C₁₋₃alkyl is independently C₁₋₃alkyl, or C₁₋₃alkyl substituted with halogen, -OH, -CN, NH₂, -NO₂, carbonyl, =O, oxo, -C₁₋₃alkylene-NH₂, -C₁₋₃alkylene-NH-C₁₋₃alkyl, or -C₁₋₃alkylene-N(C₁₋₃alkyl)₂; each substituted or unsubstituted C₁₋₃alkoxy is independently C₁₋₃alkoxy, or C₁₋₃alkoxy substituted with halogen, -OH, -CN, NH₂, -NO₂, carbonyl, =O, oxo, -C₁₋₃alkylene-NH₂, -C₁₋₃alkylene-NH-C₁₋₃alkyl, or -C₁₋₃alkylene-N(C₁₋₃alkyl)₂.

In some embodiments of Formula (I), Formula (II) or Formula (III), each C₁₋₆alkyl is independently methyl, ethyl, propyl, isopropyl, cyclopropyl, n-butyl, isobutyl, tert-buyl, n-pentyl, neopentyl, isopentyl, cyclopentyl, n-hexyl or cyclohexyl.

In some embodiments of Formula (I), Formula (II) or Formula (III), each C₁₋₃alkyl is independently methyl, ethyl, propyl, isopropyl or cyclopropyl.

In some embodiments of Formula (I), Formula (II) or Formula (III), each C₁₋₃alkoxy is independently methoxy, ethoxy, propoxy, isopropoxy or cyclopropyloxy.

In some embodiments of Formula (I), Formula (II) or Formula (III), each C₂₋₃alkenyl is independently -CH=CH₂, -CH₂-CH=CH₂, or -CH=CH-CH₃.

In some embodiments of Formula (I), Formula (II) or Formula (III), each C₂₋₃alkylnyl is independently -C≡CH, -CH₂-C≡CH, or -C≡C-CH₃.

In some embodiments of Formula (I), Formula (II) or Formula (III), each halogen is independently -F, -Cl, -Br or -I.

In some embodiments of Formula (I), Formula (II) or Formula (III), each heterocyclic group and each carbocyclic group includes single ring, spiral ring, bridge ring, fused ring and all various combinations of spiral ring, bridge ring, and/or fused ring.

In some embodiments of Formula (I), Formula (II) or Formula (III), the said single ring includes the said spiral ring includes 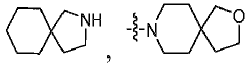 ; and the said various combinations of spiral ring, bridge ring, and/or fused ring include

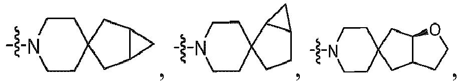

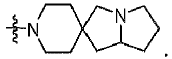

The present invention further provides some preferred technical solutions with regard to compound of Formula (I) or Formula (II), compound is
1) (S)-N1-(3-((3-amino-5-(4-amino-2-oxa-8-azaspiro[4.5]decan-8-yl)pyrazin-2-yl)thio)-2-chl orophenyl)-N2,N2-dimethyloxalamide hydrochloride;
2) N¹-(4-((3-amino-5-(4-amino-4-methylpiperidin-1-yl)pyrazin-2-yl)thio)-3-chloropyridin-2-y 1)-N²,N²-dimethyloxalamide;
3) N-(3-((3-amino-5-(4-amino-4-methylpiperidin-1-yl)pyrazin-2-yl)thio)-2-chlorophenyl)-4-( 2-(dimethylamino)-2-oxoacetyl)benzamide;
4) 6-((3-amino-5-(4-(aminomethyl)-4-methylpiperidin-1-yl)pyrazin-2-yl)thio)-2H-benzo[b][1, 4]oxazin-3(4H)-one;
5) 6-(3-amino-5-(4-(aminomethyl)-4-methylpiperidin-1-yl)pyrazin-2-yl)-2H-benzo[b][1,4]oxa zin-3 (4H)-one;
6) 5 -((2-amino-3 -chloropyridin-4-yl)thio)-N2-(2-azaspiro[4.5]dec an-8-yl)pyrazine-2 ,6-diamin e;
7) (S)-1-(4-((3-amino-5-(4-amino-2-oxa-8-azaspiro[4.5]decan-8-yl)pyrazin-2-yl)thio)-3,3-difl uoroindolin-1-yl)ethanone;
8) (S)-4-((3-amino-5-(4-amino-2-oxa-8-azaspiro[4.5]decan-8-yl)pyrazin-2-yl)thio)-3,3-difluor oindolin-2-one;
9) 4-((3-amino-S-(4-(aminomethyl)-4-methylpiperidin-1-yl)pyrazin-2-yl)thio)indolin-2-one;
10) (S)-4-((3-amino-5-(4-amino-2-oxa-8-azaspiro[4.5]decan-8-yl)pyrazin-2-yl)thio)-3,3-difluor o-1H-pyrrolo[2,3-b]pyridin-2(3H)-one;
11) (R)-N-((S)-8-(6-amino-5-((3,3-difluoro-1-methyl-2-oxoindolin-4-yl)thio)pyrazin-2-yl)-2-o xa-8-azaspiro[4.5]decan-4-yl)-2-methylpropane-2-sulfinamide;
12) (S)-4-((3-amino-5-(4-amino-2-oxa-8-azaspiro[4.5]decan-8-yl)pyrazin-2-yl)thio)indoline-2, 3-dione hydrochloride;
13) tert-butyl ((1-(5-((1-acetyl-3,3-difluoroindolin-4-yl)thio)-6-aminopyrazin-2-yl)-4-methylpiperidin-4-yl)methyl)carbamate;
14) 4-((3-amino-5-(4-(aminomethyl)-4-methylpiperidin-1-yl)pyrazin-2-yl)thio)indoline-2,3-dio ne;
15) 5-((2-amino-3-chloropyridin-4-yl)thio)-6-(4-(aminomethyl)-4-methylpiperidin-1-yl)pyrazin -2-amine;
16) N¹-(4-((3-amino-5-(4-(aminomethyl)-4-methylpiperidin-1-yl)pyrazin-2-yl)thio)-3-chloropy ridin-2-yl)-N2,N2-dimethyloxalamide ;
17) tert-butyl ((1-(6-amino-5-((2-amino-3-chloropyridin-4-yl)thio)pyrazin-2-yl)-4-methylpiperidin-4-yl)methyl)carbamate;
18) (S)-N1-(4-((3-amino-5-(4-amino-2-oxa-8-azaspiro[4.5]decan-8-yl)pyrazin-2-yl)thio)-3-chl oropyridin-2-yl)-N2,N2-dimethyloxalamide;
19) (S)-N¹-(3-((3-amino-5-(4-amino-2-oxa-8-azaspiro[4.5]decan-8-yl)pyrazin-2-yl)thio)-2-chlo rophenyl)-N²,N²-dimethyloxalamide;
20) N¹-(3-((3-amino-5-(4-(aminomethyl)-4-methylpiperidin-1-yl)pyrazin-2-yl)thio)-2-chloroph enyl)-N²,N²-dimethyloxalamide;
21) N-(3-((3-amino-5-(4-(aminomethyl)-4-methylpiperidin-1-yl)pyrazin-2-yl)thio)-2-chlorophe nyl)-3-(2-(dimethylamino)-2-oxoacetyl)benzamide;
22) 2-(3-(3-(3-((3-amino-5-(4-(aminomethyl)-4-methylpiperidin-1-yl)pyrazin-2-yl)thio)-2-chlo rophenyl)ureido)phenyl)-N,N-dimethyl-2-oxoacetamide;
23) 2-(4-(3-(3-((3-amino-5-(4-(aminomethyl)-4-methylpiperidin-1-yl)pyrazin-2-yl)thio)-2-chlo rophenyl)ureido)phenyl)-N,N-dimethyl-2-oxoacetamide;
24) 6-(4-(aminomethyl)-4-methylpiperidin-1-yl)-3-((3,4-dihydro-2H-benzo[b][1,4]oxazin-6-yl) thio)pyrazin-2-amine;
25) tert-butyl (1-(6-amino-5-((2-amino-3-chloropyridin-4-yl)thio)pyrazin-2-yl)-4-methylpiperidin-4-yl)carbamate;
26) tert-butyl (1-(5-((2-acrylamido-3-chloropyridin-4-yl)thio)-6-aminopyrazin-2-yl)-4-methylpiperidin-4-yl)carbamate;
27) tert-butyl (1-(6-amino-5-((3-chloro-2-(2-(dimethylamino)-2-oxoacetamido)pyridin-4 -yl)thio)pyrazin-2-yl)-4-methylpiperidin-4-yl)carbamate;
28) tert-butyl (1-(6-amino-5-((2-chloro-3-(2-(dimethylamino)-2-oxoacetamido)phenyl) thio)pyrazin-2-yl)-4-methylpiperidin-4-yl)carbamate;
29) N¹-(3-((3-amino-5-(4-amino-4-methylpiperidin-1-yl)pyrazin-2-yl)thio)-2-chlorophenyl)-N² ,N²-dimethyloxalamide;
30) tert-butyl (1-(6-amino-5-((3-amino-2-chlorophenyl)thio)pyrazin-2-yl)-4-methylpiperidin-4-yl)carbamate;
31) N-(3-((3-amino-5-(4-amino-4-methylpiperidin-1-yl)pyrazin-2-yl)thio)-2-chlorophenyl)-2-o xo-2-(p-tolyl)acetamide;
32) tert-butyl (1-(5-((3-acrylamido-2-chlorophenyl)thio)-6-aminopyrazin-2-yl)-4-methylpiperidin-4-yl)carbamate;
33) 6-(3-amino-5-(4-amino-4-methylpiperidin-1-yl)pyrazin-2-yl)-2H-benzo[b][1,4]oxazin-3(4 H)-one;
34) N-(4-((3-amino-5-(4-amino-4-methylpiperidin-1-yl)pyrazin-2-yl)thio)-3-chloropyridin-2-yl )-4-(2-(dimethylamino)-2-oxoacetyl)benzamide;
35) N-(4-((3-amino-5-(4-(aminomethyl)-4-methylpiperidin-1-yl)pyrazin-2-yl)thio)-3-chloropyr idin-2-yl)-4-(2-(dimethylamino)-2-oxoacetyl)benzamide;
36) N-(3-((3-amino-5-(4-(aminomethyl)-4-methylpiperidin-1-yl)pyrazin-2-yl)thio)-2-chlorophe nyl)-4-(2-(dimethylamino)-2-oxoacetyl)benzamide;
37) 5-((2-amino-3-chloropyridin-4-yl)thio)-N2-cyclohexylpyrazine-2,6-diamine;-3062-B
38) (S)-8-(5-((1H-pyrrolo[2,3-b]pyridin-4-yl)thio)-6-aminopyrazin-2-yl)-2-oxa-8-azaspiro[4.5] decan-4-amine;
39) (S)-8-(6-amino-5-((3,3-dimethylindolin-4-yl)thio)pyrazin-2-yl)-2-oxa-8-azaspiro[4.5]decan -4-amine;
40) (S)-8-(6-amino-5-((3-fluoro-1H-indol-4-yl)thio)pyrazin-2-yl)-2-oxa-8-azaspiro[4.5]decan-4 -amine;
41) 5-((2-amino-3-chloropyridin-4-yl)thio)-N2-(4-(aminomethyl)-4-methylcyclohexyl)pyrazine -2,6-diamine;
42) (S)-8-(5-((1H-indol-4-yl)thio)-6-aminopyrazin-2-yl)-2-oxa-8-azaspiro[4.5]decan-4-amine;
43) (S)-1-(4-((3-amino-5-(4-amino-2-oxa-8-azaspiro[4.5]decan-8-yl)pyrazin-2-yl)thio)-1H-ind ol-1-yl)ethanone;
44) 5-((2-amino-3-chloropyridin-4-yl)thio)-N2-(4-amino-4-methylcyclohexyl)pyrazine-2,6-dia mine;
45)
   (S)-6-((3-amino-5-(4-amino-2-oxa-8-azaspiro[4.5]decan-8-yl)pyrazin-2-yl)thio)-2H-benzo[ b][1,4]oxazin-3(4H)-one;
46) (S)-4-((3-amino-5-(4-amino-2-oxa-8-azaspiro[4.5]decan-8-yl)pyrazin-2-yl)thio)-1,3,3-trim ethylindolin-2-one;
47) (4S)-8-(6-amino-5-((3-fluoroindolin-4-yl)thio)pyrazin-2-yl)-2-oxa-8-azaspiro[4.5]decan-4-amine;
48) 1-(4-((3-amino-5-((S)-4-amino-2-oxa-8-azaspiro[4.5]decan-8-yl)pyrazin-2-yl)thio)-3-fluor o-3-methylindolin-1-yl)ethanone;
49) (S)-8-(6-amino-5-((3,3-difluoroindolin-4-yl)thio)pyrazin-2-yl)-2-oxa-8-azaspiro[4.5]decan-4-amine;
50) 1-(4-((3-amino-5-((S)-4-amino-2-oxaspiro[4.5]decan-8-yl)pyrazin-2-yl)thio)-3-methylindol in-1-yl)ethanone;
51) (S)-8-(6-amino-S-((8-chloro-4,4-difluoro-1,2,3,4-tetrahydroquinolin-5-yl)thio)pyrazin-2-yl) -2-oxa-8-azaspiro[4.5]decan-4-amine;
52) (4S)-8-(6-amino-5-((8-chloro-4-fluoro-1,2,3,4-tetrahydroquinolin-5-yl)thio)pyrazin-2-yl)-2 -oxa-8-azaspiro[4.5]decan-4-amine;
53) (S)-8-(6-amino-5-((3,3-difluoro-1-methylindolin-4-yl)thio)pyrazin-2-yl)-2-oxa-8-azaspiro[ 4.5]decan-4-amine;
54) (S)-6-((3-amino-5-(4-amino-2-oxa-8-azaspiro[4.5]decan-8-yl)pyrazin-2-yl)thio)-3,3-difluor oindolin-2-one;
55) 4-((3-amino-5-((S)-4-amino-2-oxa-8-azaspiro[4.5]decan-8-yl)pyrazin-2-yl)thio)-3-fluoroin dolin-2-one;
56) (S)-8-(6-amino-5-((3,3-difluoro-2,3-dihydrobenzofuran-4-yl)thio)pyrazin-2-yl)-2-oxa-8-az aspiro[4.5]decan-4-amine;
57) (S)-8-(6-amino-5-((4,4-difluorochroman-5-yl)thio)pyrazin-2-yl)-2-oxa-8-azaspiro[4.5]deca n-4-amine;
58) 4-((3-amino-5-((S)-4-amino-2-oxa-8-azaspiro[4.5]decan-8-yl)pyrazin-2-yl)thio)-3-fluoro-1 -methyl-3-(trifluoromethyl)indolin-2-one;
59) (S)-6-((3-amino-5-(4-amino-2-oxa-8-azaspiro[4.5]decan-8-yl)pyrazin-2-yl)thio)-7-chloroin dolin-2-one;
60) (S)-8-(6-amino-5-((5-chloro-3,4-dihydro-2H-benzo[b][1,4]oxazin-6-yl)thio)pyrazin-2-yl)-2 -oxa-8-azaspiro[4.5]decan-4-amine;
61) (S)-7-((3-amino-5-(4-amino-2-oxa-8-azaspiro[4.5]decan-8-yl)pyrazin-2-yl)thio)-8-chloro-3 ,4-dihydroquinolin-2(1H)-one;
62) (S)-6-((3-amino-5-(4-amino-2-oxa-8-azaspiro[4.5]decan-8-yl)pyrazin-2-yl)thio)-5-chloro-2 H-benzo[b][1,4]oxazin-3(4H)-one;
63) (S)-2-(3-((3-amino-5-(4-amino-2-oxa-8-azaspiro[4.5]decan-8-yl)pyrazin-2-yl)thio)-2-chlor ophenyl)-N,N-dimethyl-2-oxoacetamide;
64) 4-((3-amino-5-(4-(aminomethyl)-4-methylpiperidin-1-yl)pyrazin-2-yl)thio)-3,3-difluoro-1 H-pyrrolo[2,3-b]pyridin-2(3H)-one;
65) (S)-4-((3-amino-5-(4-amino-2-oxa-8-azaspiro[4.5]decan-8-yl)pyrazin-2-yl)thio)-1H-benzo[ d]imidazol-2(3H)-one;
66) (S)-4-((3-amino-5-(4-amino-2-oxa-8-azaspiro[4.5]decan-8-yl)pyrazin-2-yl)thio)-1,3-dimeth yl-1H-benzo[d]imidazol-2(3H)-one;
67) (S)-8-(6-amino-5-((2,2-difluoro-2,3-dihydro-1H-benzo[d]imidazol-4-yl)thio)pyrazin-2-yl)-2-oxa-8-azaspiro[4.5]decan-4-amine;
68) (S)-8-(6-amino-5-((2,2-difluoro-1,3-dimethyl-2,3-dihydro-1H-benzo[d]imidazol-4-yl)thio) pyrazin-2-yl)-2-oxa-8-azaspiro[4.5]decan-4-amine;
69) (4S)-8-(6-amino-5-((1-amino-3,3-difluoro-2,3-dihydro-1H-inden-4-yl)thio)pyrazin-2-yl)-2-oxa-8-azaspiro[4.5]decan-4-amine;
70) (S)-5-((3-amino-5-(4-amino-2-oxa-8-azaspiro[4.5]decan-8-yl)pyrazin-2-yl)thio)-2-methyl-[ 1,2,4]triazolo[4,3-a]pyridin-3(2H)-one;
71) (S)-8-(6-amino-5-((3,3-difluoro-2-methyl-2,3-dihydro-[1,2,4]triazolo[4,3-a]pyridin-5-yl)thi o)pyrazin-2-yl)-2-oxa-8-azaspiro[4.5]decan-4-amine;
72) (S)-1-(4-((3-amino-5-(4-amino-2-oxa-8-azaspiro[4.5]decan-8-yl)pyrazin-2-yl)thio)indolin-1-yl)ethanone;
73) 1'-(6-amino-5-((2-amino-3-chloropyridin-4-yl)thio)pyrazin-2-yl)hexahydrospiro[cyclopent a [b]furan-5,4'-piperidin]-4-amine;
74) 1'-(6-amino-5-((2-amino-3-chloropyridin-4-yl)thio)pyrazin-2-yl)spiro[bicyclo[3.1.0]hexane -3,4'-piperidin]-2-amine;
75) 1'-amino-1-(6-amino-5-((2-amino-3-chloropyridin-4-yl)thio)pyrazin-2-yl)tetrahydrospiro[p iperidine-4,2'-pyrrolizin]-3'(1'H)-one;
76) 1'-(6-amino-5-((2-amino-3-chloropyridin-4-yl)thio)pyrazin-2-yl)spiro[bicyclo[3.1.0]hexane -2,4'-piperidin]-3-amine.

The present invention also provides a pharmaceutical composition comprising at least one compound described herein and at least one pharmaceutically acceptable excipient. In composition, the said compound in a weight ratio to the said excipient within the range from about 0.0001 to about 10. any one of Formula (I), Formula (II) or Formula (III)

The present invention additionally provided a use of the pharmaceutical composition of as described herein for the preparation of a medicament.

In some embodiments, a medicament thus prepared can be used for the treatment or prevention of cancer, cancer metastasis, cardiovascular disease, an immunological disorder or an ocular disorder.

The present invention additionally provided a use of at least one compound described herein to prepare of a medicament.

In some embodiments, a medicament thus prepared can be used for the treatment or prevention of cancer, cancer metastasis, cardiovascular disease, an immunological disorder or an ocular disorder.

At least one compound for use described herein which is for use in the treatment of cancer, the prevention of cancer metastasis or the treatment of cardiovascular disease, an immunological disorder or an ocular disorder.

Use, in the manufacture of a medicament for use as an inhibitor of SHP2, of at least one compound described herein.

A method of treating a patient having a condition which is mediated by the activity of SHP2, said method comprising administering to the patient a therapeutically effective amount of at least one compound described herein, or a pharmaceutically acceptable salt thereof.

In some embodiments, the condition mediated by the activity of SHP2 is cancer.

In some embodiments, the condition mediated by the activity of SHP2 is noonan syndrome, leopard syndrome, juvenile myelomonocytic leukemias, liver cancer, neuroblastoma, melanoma, squamous-cell carcinoma of the head and neck, acute myeloid leukemia, breast cancer, esophageal cancer, lung cancer, colon cancer, head cancer, gastric carcinoma,neuroblastoma, anaplastic large-cell lymphoma and glioblastoma.

At least one compound described herein or a pharmaceutically acceptable salt thereof for use as a medicament.

At least one compound described herein or a pharmaceutically acceptable salt thereof for use in the treatment of cancer.

A method of treating cancer selected from the group consisting of noonan syndrome, leopard syndrome, juvenile myelomonocytic leukemias, liver cancer, neuroblastoma, melanoma, squamous-cell carcinoma of the head and neck, acute myeloid leukemia, breast cancer, esophageal cancer, lung cancer, colon cancer, head cancer, gastric carcinoma,neuroblastoma, anaplastic large-cell lymphoma and glioblastoma in a mammal comprising administering to a mammal in need of such treatment an effective amount of at least one compound described herein or a pharmaceutically acceptable salt thereof

The term "halogen", as used herein, unless otherwise indicated, means fluoro, chloro, bromo or iodo. The preferred halogen groups include F, Cl and Br. The terms "haloC₁₋₆alkyl", "haloC₂₋₆alkenyl", "haloC₂₋₆alkynyl" and "haloC₁₋₆alkoxy" mean a C₁₋₆alkyl, C₂₋₆alkenyl, C₂₋₆alkynyl or C₁₋₆alkoxy in which one or more (in particular, 1 to 3) hydrogen atoms have been replaced by halogen atoms, especially fluorine or chlorine atoms. In some embiment, preferred are fluoroC₁₋₆alkyl, fluoroC₂₋₆alkenyl, fluoroC₂₋₆alkynyl and fluoroC₁₋₆alkoxy groups, in particular fluoroC₁₋₃alkyl, for example, CF₃, CHF₂, CH₂F, CH₂CH₂F, CH₂CHF₂, CH₂CF₃ and fluoroC₁₋₃alkoxy groups, for example, OCF₃, OCHF₂, OCH₂F, OCH₂CH₂F, OCH₂CHF₂ or OCH₂CF₃, and most especially CF₃, OCF₃ and OCHF₂.

As used herein, unless otherwise indicated, alkyl includes saturated monovalent hydrocarbon radicals having straight, branched or cyclic moieties. For example, alkyl radicals include methyl, ethyl, propyl, isopropyl, cyclcopropyl, n-butyl, isobutyl, sec-butyl, t-butyl, cyclcobutyl, n-pentyl, 3- (2-methyl) butyl, 2-pentyl, 2-methylbutyl, neopentyl, cyclcopentyl, n- hexyl, 2-hexyl, 2-methylpentyl and cyclohexyl. Similary, C₁₋₈, as in C₁₋₈alkyl is defined to identify the group as having 1, 2, 3, 4, 5, 6, 7 or 8 carbon atoms in a linear or branched arrangement.

Alkylene means a difunctional group obtained by removal of a hydrogen atom from an alkyl group that is defined above. For example, methylene (i.e., -CH₂-), ethylene (i.e., -CH₂-CH₂- or -CH(CH₃)-) and propylene (i.e., -CH₂-CH₂-CH₂-, -CH(-CH₂-CH₃)- or -CH₂-CH(CH₃)-).

Alkenyl and alkynyl groups include straight, branched chain or cyclic alkenes and alkynes. Likewise, "C₂₋₈alkenyl" and "C₂₋₈alkynyl"means an alkenyl or alkynyl radicals having 2, 3, 4, 5, 6, 7 or 8 carbon atoms in a linear or brached arrangement.

Alkoxy radicals are oxygen ethers formed from the previously described straight, branched chain or cyclic alkyl groups.

The term"aryl", as used herein, unless otherwise indicated, refers to an unsubstituted or substituted mono- or polycyclic ring system containing carbon ring atoms. The preferred aryls are mono cyclic or bicyclic 6-10 membered aromatic ring systems. Phenyl and naphthyl are preferred aryls. The most preferred aryl is phenyl.

The term"heterocyclic", as used herein, unless otherwise indicated, refers to unsubstituted and substituted mono- or polycyclic non-aromatic ring system containing one or more heteroatoms. Preferred heteroatoms include N, O, and S, including N-oxides, sulfur oxides, and dioxides. Preferably the ring is three to eight membered and is either fully saturated or has one or more degrees of unsaturation. Multiple degrees of substitution, preferably one, two or three, are included within the present definition.

Examples of such heterocyclic groups include, but are not limited to azetidinyl, pyrrolidinyl, piperidinyl, piperazinyl, oxopiperazinyl, oxopiperidinyl, oxoazepinyl, azepinyl, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiamorpholinyl sulfoxide, thiamorpholinyl sulfone and oxadiazolyl.

The term"heteroaryl", as used herein, unless otherwise indicated, represents an aromatic ring system containing carbon(s) and at least one heteroatom. Heteroaryl may be monocyclic or polycyclic, substituted or unsubstituted. A monocyclic heteroaryl group may have 1 to 4 heteroatoms in the ring, while a polycyclic heteroaryl may contain 1 to 10 hetero atoms. A polycyclic heteroaryl ring may contain fused, spiro or bridged ring junction, for example, bycyclic heteroaryl is a polycyclic heteroaryl. Bicyclic heteroaryl rings may contain from 8 to 12 member atoms. Monocyclic heteroaryl rings may contain from 5 to 8 member atoms (cabons and heteroatoms). Examples of heteroaryl groups include, but are not limited to thienyl, furanyl, imidazolyl, isoxazolyl, oxazolyl, pyrazolyl, pyrrolyl, thiazolyl, thiadiazolyl, triazolyl, pyridyl, pyridazinyl, indolyl, azaindolyl, indazolyl, benzimidazolyl, benzofuranyl, benzothienyl, benzisoxazolyl, benzoxazolyl, benzopyrazolyl, benzothiazolyl, benzothiadiazolyl, benzotriazolyl adeninyl, quinolinyl or isoquinolinyl.

The term "cycloalkyl" refers to a substituted or unsubstituted monocyclic, bicyclic or polycyclic non-aromatic saturated ring, which optionally includes an alkylene linker through which the cycloalkyl may be attached. Examplary " cycloalkyl" groups includes but not limited to, cyclopropyl, cyclobutyl, cyclopentyl, cyclohexyl and so on.

The term"carbonyl, =O or oxo"refers to the group C(O).

Whenever the term "alkyl" or "aryl" or either of their prefix roots appear in a name of a substituent (e.g., aralky or dialkylamino) it shall be interpreted as including those limitations given above for"alkyl"and"aryl."Designated numbers of carbon atoms (e.g., C₁₋₆) shall refer independently to the number of carbon atoms in an alkyl moiety or to the alkyl portion of a larger substituent in which alkyl appears as its prefix root.

The term"composition", as used herein, is intended to encompass a product comprising the specified ingredients in the specified amounts, as well as any product which results, directly or indirectly, from combinations of the specified ingredients in the specified amounts. Accordingly, pharmaceutical compositions containing the compounds of the present invention as the active ingredient as well as methods of preparing the instant compounds are also part of the present invention. Furthermore, some of the crystalline forms for the compounds may exist as polymorphs and as such are intended to be included in the present invention. In addition, some of the compounds may form solvates with water (i.e., hydrates) or common organic solvents and such solvates are also intended to be encompassed within the scope of this invention.

The compounds of the present invention may also be present in the form of pharmaceutically acceptable salts. For use in medicine, the salts of the compounds of this invention refer to non-toxic "pharmaceutically acceptable salts". The pharmaceutically acceptable salt forms include pharmaceutically acceptable acidic/anionic or basic/cationic salts. The pharmaceutically acceptable acidic/anionic salt generally takes a form in which the basic nitrogen is protonated with an inorganic or organic acid. Representative organic or inorganic acids include hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicylic, saccharinic or trifluoroacetic. Pharmaceutically acceptable basic/cationic salts include, and are not limited to aluminum, calcium, chloroprocaine, choline, diethanolamine, ethylenediamine, lithium, magnesium, potassium, sodium and zinc.

The present invention includes within its scope the prodrugs of the compounds of this invention. In general, such prodrugs will be functional derivatives of the compounds that are readily converted in vivo into the required compound. Thus, in the methods of treatment of the present invention, the term "administering" shall encompass the treatment of the various disorders described with the compound specifically disclosed or with a compound which may not be specifically disclosed, but which converts to the specified compound in vivo after administration to the subject. Conventional procedures for the selection and preparation of suitable prodrug derivatives are described, for example, in "Design of Prodrugs", ed. H. Bundgaard, Elsevier, 1985.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques know in the art as well as those methods set forth herein.

The present invention includes compounds described can contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof.

The above Formula I is shown without a definitive stereochemistry at certain positions. The present invention includes all stereoisomers of Formula I and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of Formula (I) exists, the present invention includes any possible tautomers and pharmaceutically acceptable salts thereof, and mixtures thereof, except where specifically stated otherwise.

When the compound of Formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. When the compound of the present invention is basic, its corresponding salt can be conveniently prepared from pharmaceutically acceptable non-toxic acids, including inorganic and organic acids. Since the compounds of Formula (I) are intended for pharmaceutical use they are preferably provided in substantially pure form, for example at least 60% pure, more suitably at least 75% pure, especially at least 98% pure (% are on a weight for weight basis).

The pharmaceutical compositions of the present invention comprise a compound represented by Formula I (or a pharmaceutically acceptable salt thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula I, or a prodrug, or a metabolite, or pharmaceutically acceptable salts thereof, of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in- oil liquid emulsion. In addition to the common dosage forms set out above, the compound represented by Formula I, or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound, or a pharmaceutically acceptable salt, of Formula I. The compounds of Formula I, or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen. In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound represented by Formula I of this invention, or a pharmaceutically acceptable salt thereof, via conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants) and the like. Furthermore, other adjuvants can be included to render the formulation isotonic with the blood of the intended recipient. Compositions containing a compound described by Formula I, or pharmaceutically acceptable salts thereof, may also be prepared in powder or liquid concentrate form.

Generally, dosage levels on the order of from about 0.01mg/kg to about 150mg/kg of body weight per day are useful in the treatment of the above-indicated conditions, or alternatively about 0.5mg to about 7g per patient per day. For example, inflammation, cancer, psoriasis, allergy/asthma, disease and conditions of the immune system, disease and conditions of the central nervous system (CNS), may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

These and other aspects will become apparent from the following written description of the invention.

### Examples

The following Examples are provided to better illustrate the present invention. All parts and percentages are by weight and all temperatures are degrees Celsius, unless explicitly stated otherwise. The following abbreviations have been used in the examples:
- DAST:: Diethylaminosulfur trifluoride;
- DCM:: Dichloromethane;
- DIEA:: N,N-Diisopropylethylamine;
- DMF:: N,N-Dimethylformamide;
- DMSO:: Dimethyl sulfoxide;
- EA:: Ethyl acetate;
- EtOH:: Ethanol;
- NMP:: N-methyl-2-pyrrolidone;
- TEA:: Triethylamine;
- THF:: Tetrahydrofuran
- TFA:: Trifluoroacetic acid;
- Xantphos:: Dimethylbisdiphenylphosphinoxanthene;
- min:: Minute;
- rt or RT:: room temperature;
- TLC:: Thin layer chromatography;
- Pre-TLC:: Preparation by thin layer chromatogaraphy.

### Example 1 Synthesis of compound 1

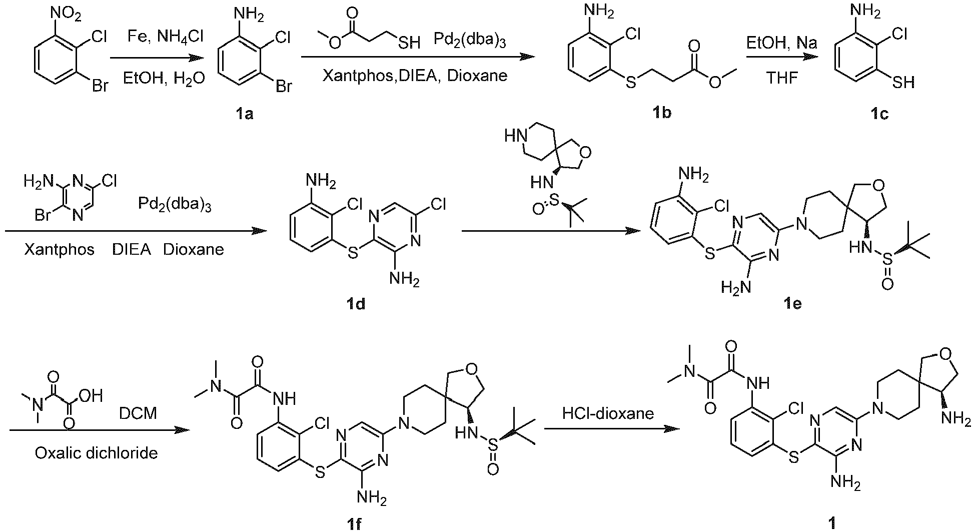

A mixture of 1-bromo-2-chloro-3-nitrobenzene (36.61g, 154.83mmol), iron powder (43.35g, 774.16mmol), NH₄Cl (8.28g, 154.83mmol), EtOH (100mL) and H₂O (50mL) was heated to 60°C for 4 hours, then cooled to 10°C. The reaction mixture was filtered through a pad of Celite and the filtrate was concentrated to remove the EtOH. The residual solution was extracted with EA (100mL×2). The combined organic extracts were washed with brine (100mL), dried over anhydrous Na₂SO₄ and concentrated under reduced pressure to afford the compound **1a** (30.01g,93.88%). MS: 206 (M+H)⁺.

A mixture of the compound **1a** (30.00g, 0.15mol), methyl 3-mercaptopropanoate (27.60g, 0.23mol), Pd₂(dba)₃ (1.37g, 1.5mmol), Xantphos (1.73g, 3.00mmol), DIEA (38.75g, 0.30mol) in dioxane (200mL) was stirred at 95°C under N₂ for 18 hours. After completion of the reaction, the reaction mixture was concentrated under reduced pressure and purified by column chromatography to afford the compound **1b** (10.00g, 27.13%). MS: 246 (M+H)⁺.

A mixture of Na (1.22g, 52.90mmol) and EtOH (25mL) was stirred at 20°C until the Na dissolved completely. The compound **1b** (10.00g, 40.70mmol) in THF (30mL) was added dropwise at -30°C∼-20°C, then the mixture was stirred at 20°C for 3.5 hours, and concentrated under reduced pressure. The residue was added water (50mL), extracted with EA (50mL×2). The water phase was adjusted pH=2~3 with HCl solution (1mol/L) and extracted with EA (50mL×2). The combined organic extracts were washed with brine (50mL), dried over anhydrous Na₂SO₄ and filtered and concentrated under reduced pressure to afford the compound **1c** (6.02g, 92.66%). MS: 160 (M+H)⁺.

A mixture of the compound **1c** (6.02g, 37.71mol), 3-bromo-6-chloropyrazin-2-amine (7.86g, 37.71mol), Pd₂(dba)₃ (0.35g, 0.38mmol), Xantphos (0.43g, 0.75mmol), DIEA (9.74g, 75.42mol) in dioxane (70mL) was stirred at 95°C under N₂ for 17 hours. After completion of the reaction, the mixture was filtered and the filtrate was concentrated under reduced pressure. The residue was added EA (50mL) and stirred for 0.5 hour, then filtered to afford the compound **1d** (8.45, 78.03%). MS: 287 (M+H)⁺.

A solution of the compound **1d** (0.81g, 2.82mmol) , (R)-2-methyl-N-((S)-2-oxa-8-azaspiro [4.5]decan-4-yl)propane-2-sulfinamide (TFA salt, 1.26g, 3.38mmol) and K₂CO₃ (1.17g, 8.46mmol) in NMP (10mL) was stirred for 14 hours at 130°C. After cooling to RT, the resulting residue was dissolved in EA (100mL), washed with H₂O (30mL), dried over anhydrous Na₂SO₄, filtered and the volatiles were removed under reduced pressure and purified by column chromatography to afford the compound **1e** (0.55g, 38%). MS: 511(M+H)⁺.

Oxalyl chloride was added dropwise to a solution of 2-(dimethylamino)-2-oxoacetic acid (0.26 g, 2.15 mmol) in DCM (10mL),and stirred at RT for 2 hours, the volatiles were removed under reduced pressure, the residue was dissloved in DCM (10mL) and added dropwise to a solution of the compound **1e** (0.55g, 1.08mmol) in DCM (10mL), after completion of the reaction, the reaction mixture was quenched by addition of ice-water (20mL), washed with brine, dried over anhydrous Na₂SO₄, filtered and the volatiles were removed under reduced pressure and purified by column chromatography to afford the compound **1f** (0.42g, 64%). MS: 610(M+H)⁺.

The compound **1f** (0.41g, 0.67mmol) and HCl (4M in dioxane, 5mL) in DCM (10mL) was stirred for 20 min at 40°C. After cooling to RT, HCl (1M in H₂O) was added and the resulting aqueous mixture was extracted with DCM. The aqueous phase was basified with NH₄OH (28% in H₂O) until pH to 12 and extracted with DCM (3×20mL). The combined organic layers were washed with brine, dried over anhydrous Na₂SO₄, filtered, concentrated under reduced pressure, and purified by column chromatography to afford the compound **1** (100mg, 32%). MS: 506(M+H)⁺.

¹HNMR (DMSO-d6, 400MHz): δ 7.63 (s, 1H), 7.43-7.45 (d, 1H), 7.21-7.23 (d, 1H), 6.50-6.52 (d, 1H), 6.11 (s, 1H), 4.00-4.05 (m, 4H), 3.93-3.96 (dd, 2H), 2.93-3.29 (m, 4H), 1.54-1.98 (m, 4H).

### Example 2 Synthesis of compound 2

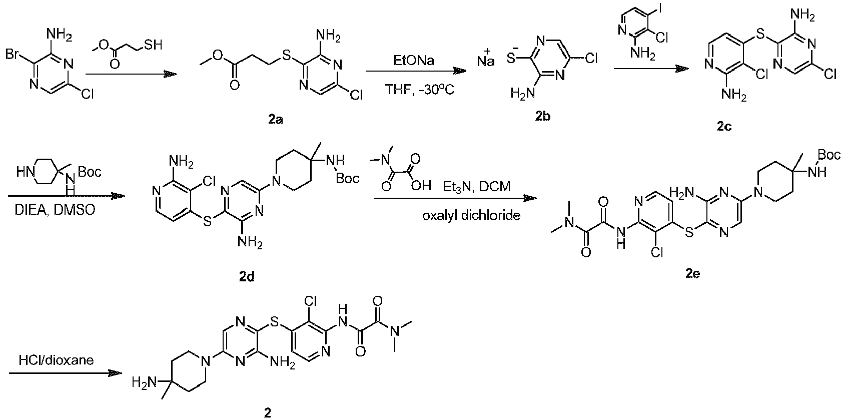

A mixture of 3-bromo-6-chloropyrazin-2-amine (20.02g, 96.05mmol), methyl 3-mercaptopropanoate (11.53g, 96.05mmol), DIEA (24.83g, 192.10mmol), Pd(OAc)₂ (0.30g, 1.34mmol), Xantphos (2.78g,4.8mmol) in dixoane (200mL) was heated to 95°C under N₂ for 18 hours. The reaction mixture was filtered, concentrated under reduced pressure, and purified by column chromatography to afford the compound **2a** (18.83g, 79%). MS: 248(M+H)⁺.

A solution of the compound **2a** (18.33g, 76.02mmol) in THF (150mL) was cooled to -30°C. Sodium ethoxide (6.72g, 98.82mmol) in ethanol (100mL) was added dropwise. The resulting mixture was stirred at -30 °C for 1 hours, then warmed to 25°C and stirred for another 2 hours. The volatiles were removed under reduced pressure and dissolved in DCM (100mL), the precipitate was filtered to give the compound **2b** as a brown solid (13.82g, 99%). MS: 162(M+H)⁺.

A mixture of the compound **2b** (9.98g, 54.36mmol), 3-chloro-4-iodopyridin-2-amine (13.83g, 54.36mmol), DIEA (14.04g, 108.72mmol), Pd₂(dba)₃ (1.00g, 1.09mmol), Xantphos (1.00g, 1.73mmol) in dioxane (200mL) was heated to 95°C under N₂ for 18 hours. The reaction mixture was filtered and concentrated under reduced pressure, the residue was dissolved in DCM (100mL), the precipitate was filtered to afford the compound **2c** as a brown solid (14.62g, 93.72%). MS: 288(M+H)⁺.

A mixture of the compound **2c** (2.31g, 8.05mmol), tert-butyl (4-methylpiperidin-4-yl)carbamate (3.45g, 16.10mmol), DIEA (3.12g, 24.15mmol) in DMSO (50mL) was stirred at 100°C for 3 hours. Water (100mL) was added to the reaction mixture and the precipitate was filtered to give the compound **2d** as a brown solid (2.19g, 58%). MS: 466(M+H)⁺.

To a solution of 2-(dimethylamino)-2-oxoacetic acid (350mg, 3mmol) in DCM (10mL) was added oxalyl dichloride (760mg, 6mmol) and DMF (2 drops). The resulting mixture was stirred at 20°C for 1 hour. The volatiles were removed under reduced pressure and the residue was dissolved in DCM (10mL), then the mixture was added to the solution of the compound **2d** (460mg, 1mmol) and TEA (1mL) in DCM (10mL). After completion of the reaction, the reaction mixture was concentrated in vacuo, the residue was purified by Pre-TLC to afford the compound **2e** as a yellow solid (142mg, 25%). MS: 565(M+H)⁺.

A mixture of the compound **2e** (142mg, 0.25mmol) in HCl/dioxane (10mL, 4M) was stirred for 1 hour. The precipitate was filtered to afford the compound **2** as a brown solid (70mg, 60%). MS: 465(M+H)⁺.

¹HNMR (DMSO-d6, 400MHz): δ 8.45-8.35 (m,2H), 8.06 (s, 1H), 7.71 (s, 1H), 6.45 (s, 1H), 4.06 (s, 6H), 3.38 (t, 2H), 2.93 (t, 2H), 2.86 (s, 2H), 1.84-1.71 (m, 4H), 1.39 (s, 3H).

### Example 3 Synthesis of compound 3

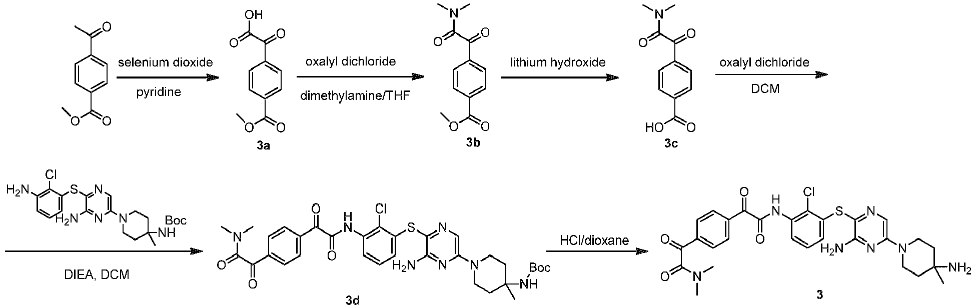

A mixture of methyl 4-acetylbenzoate (7.01g, 40.00mmol), selenium dioxide (8.95g, 80.00mmol) and pyridine (50mL) was heated to 100°C for 4 hours. Hydrochloric acid (70mL, 1M) was added to the reaction mixture to adjust pH=3, the aqueous solution was extracted with EA (70mL).The combined organic extracts were dried over anhydrous Na₂SO₄, concentrated in vacuo to afford the compound **3a** as a brown solid (6.12g, 74%). MS: m/z 207(M-H)⁻.

To a solution of the compound **3a** (2.51g, 12.07mmol) in DCM (30mL) was added oxalyl dichloride (15mL) and DMF (2 drops). The resulting mixture was stirred at 20°C for 1 hour. The volatiles were removed under reduced pressure and the residue was dissolved in DCM (10mL), then the mixture was added to the solution of dimethylamine/THF (10mL, 2M). After completion of the reaction, the reaction mixture was concentrated to afford the compound **3b** as a brown solid (2.35g, 83%). MS: 236(M+H)⁺.

A mixture of the compound **3b** (0.91g, 4.11mmol), lithium hydroxide monohydrate (0.85g, 20.55mmol), H₂O (10mL) in methanol (50mL) was stirred at 25°C for 1 hour. The volatiles were removed under reduced pressure and the residue was dissolved in hydrochloric acid (25mL,1M), the aqueous solution was extracted with EA (20mL×2).The combined organic extracts were washed with brine (50mL), dried over anhydrous Na₂SO₄, filtered and the volatiles were removed under reduced pressure to afford the compound **3c** as a brown solid (0.85g, 93%). MS: 222(M+H)⁺.

To a solution of the compound **3c** (0.85g, 3.85mmol) in DCM (10mL) was added oxalyl dichloride (8mL) and DMF (2 drops). The resulting mixture was stirred at 20°C for 1 hour. The volatiles were removed under reduced pressure and the residue was dissolved in DCM (10mL), then the mixture was added to the solution of tert-butyl (1-(6-amino-5-((3-amino-2-chlorophenyl)thio)pyrazin-2-yl)-4-methylpiperidin-4-yl)carbamate (200mg, 0.43mmol) and DIEA (5mL) in DCM (10mL). After completion of the reaction, the reaction mixture was concentrated in vacuo, the residue was purified by Pre-TLC to afford the compound **3d** as a yellow solid (170mg, 57 %). MS: 696(M+H)⁺.

To a solution of HCl/dioxane (1mL, 4M) was added the compound **3d** (170mg, 0.24mmol), the resulting mixture was stirred at 25°C for 1 hour and the precipitate was filtered to give the crude product (107mg).The crude product was purified by Pre-TLC to afford the compound **3** as a brown solid (35mg, 24%). MS: 596(M+H)⁺.

¹HNMR (DMSO-d6, 400MHz): δ 10.44 (s, 1H), 8.39 (s, 1H), 8.18 (d, 2H), 8.02 (d, 2H), 7.68 (s, 1H), 7.36 (d, 1H), 7.26 (t, 1H), 6.58 (d, 1H), 6.18 (s, 1H), 4.03 (d, 2H), 3.37 (d, 2H), 3.04 (s, 3H), 2.90 (s, 3H), 1.83-1.71 (m, 4H), 1.39 (s, 3H).

### Example 4 Synthesis of compound 4

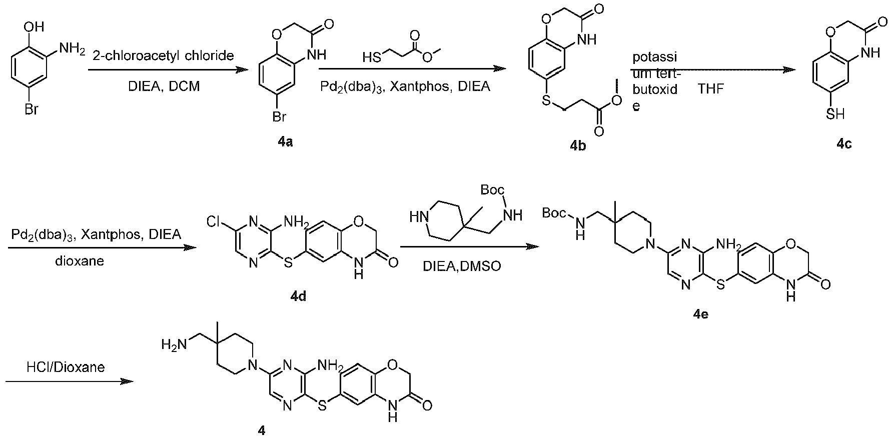

A mixture of 2-amino-4-bromophenol (10.15g, 354.29mmol), 2-chloroacetyl chloride (7.35g, 65.15mmol) and DCM (150mL) was cooled to 0°C. DIEA (35.06g, 271.45mmol) was added dropwise, the resulting mixture was stirred at 20°C for 5 hours. The reaction mixture was concentrated in vacuo and H₂O (70mL) was added, the precipitate was filtered to afford the compound **4a** as a red solid (7.61g,62%). MS: 228(M+H)⁺.

A solution of the compound **4a** (7.51g,33.09mmol), methyl 3-mercaptopropanoate (5.16g,43.02mmol), DIEA (8.55g,62.18mmol),Pd₂(dba)₃ (0.40g, 0.44mmol), Xantphos (0.40g, 0.69mmol) in dioxane (100mL) was heated to 100°C under N₂ for 8 hours. The reaction mixture was filtered and concentrated under reduced pressure, the residue was purified by column chromatography to afford the compound **4b** (5.12g, 58%). MS: 268(M+H)⁺.

A solution of the compound **4b** (3.75g, 14.04mmol) in THF (50mL) was cooled to -70°C, potassium tert-butoxide/THF (28mL, 28.08mmol) was added dropwise, the resulting mixture was stirred at -70 °C for 0.5 hour. Hydrochloric acid (15mL, 1mol/L) was added to the reaction mixture, the aqueous solution was extracted with EA (20mL×2). The combined organic extracts were washed with brine (50mL), dried over anhydrous Na₂SO₄, filtered and the volatiles were removed under reduced pressure to afford the compound **4c** as a red solid (1.57g, 62%). MS: 182(M+H)⁺.

A mixture of the compound **4c** (3.22g, 17.79mmol), 3-bromo-6-chloropyrazin-2-amine (3.68g, 17.79mmol), DIEA(4.60g, 35.58mmol), Pd₂(dba)₃ (0.15g, 0.16mmol), Xantphos (0.15g, 0.26mmol) in dioxane (30mL) was heated to 100°C under N₂ for 18 hours. The reaction mixture was filtered and concentrated under reduced pressure, the residue was dissolved in the solution of Hexane (10mL) and EA (10mL), the precipitate was filtered to afford the compound **4d** as a brown solid (2.88g, 52%). MS: 309(M+H)⁺.

A mixture of the compound **4d** (0.35g, 1.13mmol), tert-butyl ((4-methylpiperidin-4-yl)methyl)carbamate (0.39g, 1.70mmol), DIEA (0.36g, 2.83mmol) in DMSO (10mL) was stirred at 100°C for 2 hours. H₂O (10mL) was added to the reaction mixture, the aqueous solution was extracted with EA (10mL×2). The combined organic extracts were washed with brine (50mL), dried over anhydrous Na₂SO₄, filtered and the volatiles were removed under reduced pressure. The residue was purified by column chromatography to afford the compound **4e** (0.15g, 27%). MS: 501(M+H)⁺.

To a solution of HCl/Dixoane (1mL, 4mol/L) was added the compound **4e** (150mg, 0.30mmol). The resulting mixture was stirred at 25°C for 1 hour and the precipitate was filtered to afford the compound **4** as a white solid (60mg, 50%). MS: 401 (M+H)⁺.

¹HNMR(DMSO-d6, 400MHz): δ 7.5 (s, 1H), 6.87 (d, 1H), 6.75 (d, 1H), 6.73 (s, 1H), 6.00 (s, 1H), 4.52 (s, 2H), 3.78-3.74 (m, 2H), 3.30-3.27 (m, 2H), 2.39 (s, 2H), 1.43-1.39 (m, 2H), 1.27-1.25 (m, 2H), 0.91 (s, 3H).

### Example 5 Synthesis of compound 5

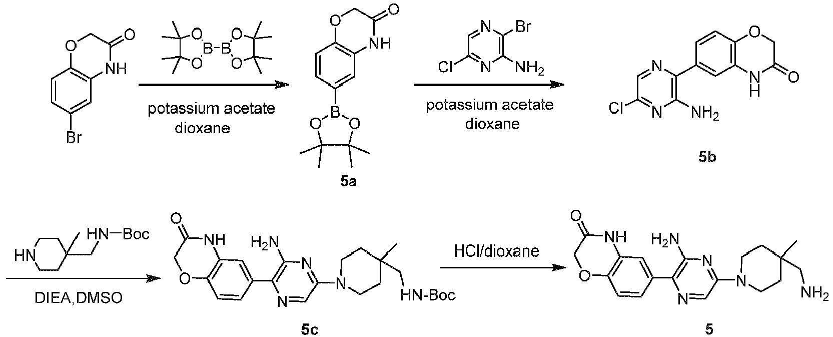

A solution of the compound **4a** (3.33g, 14.67mmol), 4,4,4',4',5,5,5',5'-octamethyl-2,2'-bi(1,3,2-dioxaborolane) (4.48g, 17.61mmol), potassium acetate (2.88g, 29.34mmol), Pd(dppf)Cl₂ 0.15g, 0.20mmol) in dioxane (40mL) was heated to 100°C under N₂ for 24 hours. The reaction mixture was filtered and concentrated under reduced pressure, the residue was purified by column chromatography to afford the compound **5a** (2.25g,56%). MS: 276(M+H)⁺.

A solution of the compound **5a** (2.20g, 8.00mmol), 3-bromo-5-chloropyrazin-2-amine (1.54g,7.27mmol), potassium acetate (2.00g, 14.54mmol), Pd(dppf)Cl₂ (0.15g, 0.20mmol), H₂O (2mL) in dioxane (40mL) was heated to 75°C under N₂ for 4 hours. Hexane (50mL) was added, the precipitate was filtered to afford the compound **5b** as a brown solid (0.79g, 36%). MS: 277(M+H)⁺.

A mixture of the compound **5b** (230mg, 0.83mmol), tert-butyl ((4-methylpiperidin-4-yl)methyl)carbamate (0.29g, 1.25mmol), DIEA (0.43g, 3.33mmol) in DMSO (10mL) was stirred at 100°C for 18 hours. H₂O (20mL) was added to the reaction mixture, the aqueous solution was extracted with EA (20mL×2). The combined organic extracts were washed with brine (20mL), dried over anhydrous Na₂SO₄, filtered and the volatiles were removed under reduced pressure, the residue was purified by column chromatography to afford the compound **5c** 157mg, 40%). MS: 469(M+H)⁺.

To a solution of HCl/dioxane (1mL, 4M) was added the compound **5c** (150mg, 0.34mmol). the resulting mixture was stirred at 25°C for 1 hour and the precipitate was filtered to afford the compound **5** as a brown solid (72mg, 56%). MS: 369 (M+H)⁺.

¹HNMR(DMSO-d6, 400MHz): δ 7.55 (s, 1H), 7.21 (d, 1H), 7.17 (d, 1H), 7.15 (d, 1H), 4.57 (s, 2H), 3.77 (t, 2H), 3.30 (t, 2H), 2.73 (s, 2H), 1.53-1.38 (m, 4H), 1.07 (s, 3H).

### Example 6 Synthesis of compound 6

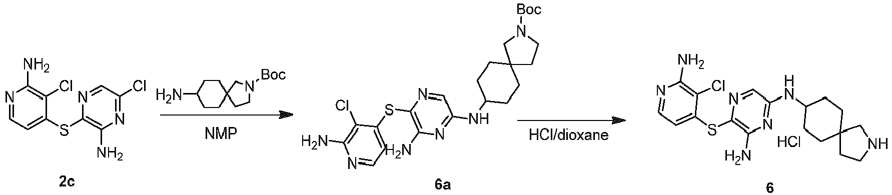

The oil bath was heated to 160 °C , a mixture of the compound **2c** (80mg, 0.28mmol) in NMP (5mL) was stirred at 160°C, then tert-butyl 8-amino-2-azaspiro[4.5]decane-2-Carboxylate (190mg, 0.75mmol) was added. 1.5 hours later. The reaction mixture was cooled and water (40mL) was added, the resulting mixture was extracted with EA (20mL×2). The combined organic extracts were washed with brine (30mL), dried over anhydrous Na₂SO₄ filtered and the volatiles were removed under reduced pressure. The residue was purified by Pre-TLC to afford the compound **6a** (15mg, 10.59%). MS: 506 (M+H)⁺.

A mixture of the compound **6a** (39mg, 0.08mmol), HCl/dioxane (4mol/L, 1mL), and dioxane (2mL) was stirred at 20°C for 1 hour. The mixture was concentrated under reduced pressure, the residue was added EA (5mL) and stirred for 5 mins. The solid was filtered to afford the compound 6 as an HCl salt (30mg, 84.76%). MS: 203.7 (M+2H)²⁺.

### Example 7 Synthesis of compound 7

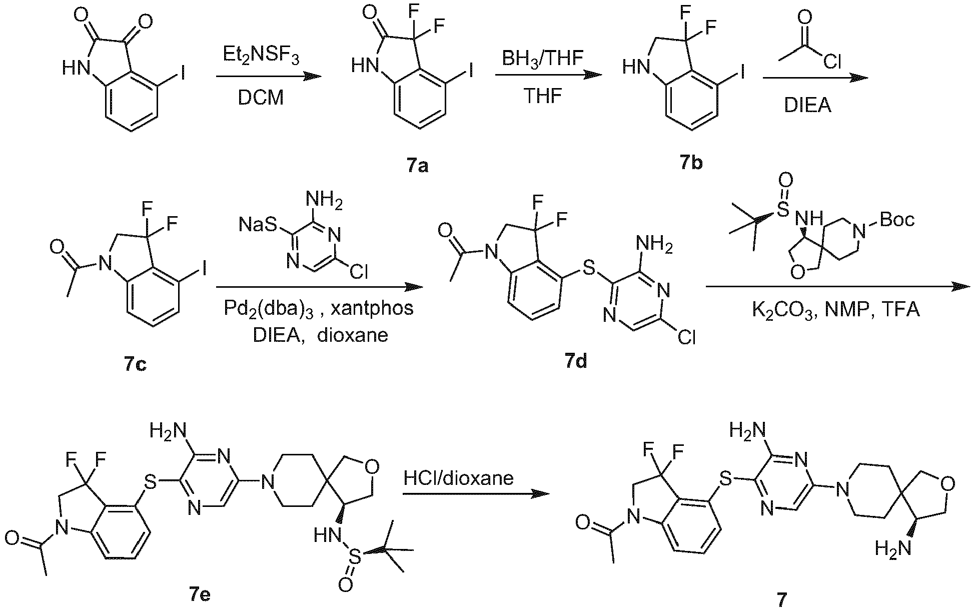

A solution of 4-iodoindoline-2,3-dione (35.00g, 128.19mmol) in DCM (300mL) was added diethylaminosulfurtrifluoride (62.00g, 387.12mmol) dropwise at 0°C. The mixture was stirred overnight at RT. After completion of the reaction, the mixture was dropwised into a solution of sodium hydrogen carbonate in water (85g/400mL), the two layers were separated and aqueous layer was extracted with DCM (150mL×2), The combined organic phases were dried over anhydrous Na₂SO₄ ,filtered and the volatiles were removed under reduced pressure. The residue was purified by column chromatography to afford the compound **7a** as a off white solid (24.01g, 63.4%). MS: 296(M+H)⁺.

A solution of the compound **7a** (24.00g, 81.34mmol) in THF (100mL) was stirred at 0°C, BH₃/THF (290mL, 1M) was added dropwise, the ice-water bath was removed after the adding was completed and the mixture was stirred at RT for another 1 hour, TLC showed the reaction was completed. The mixture was quenched with 10% citric acid solution (50mL) at 0°C,water (200mL) was added, extracted with EA (200mL×2), the organic phase was washed with brine (200mL×2), dried over anhydrous Na₂SO₄, filtered and the solvent was removed until about 300mL remained under reduced pressure, and compound 7b was used immediately for the next step without further purification. MS: 282(M+H)⁺.

A mixture of the compound **7b** in EA obtained from the last step and DIEA (19mL, 161.71mmol) was added acetyl chloride (12mL, 169.68mmol) dropwise at 0°C. After completion of the reaction, water was added (100mL), the organic layer was separated and aqueous layer was extracted with EA (50mL). The combined organic layers were washed with brine (200mL×2), dried over anhydrous Na₂SO₄, filtered and the volatiles were removed under reduced pressure, and the resultant residue was washed with EA-hexane=1:10 (80mL) to affford the compound **7c** (21.30g, 81.0%). MS: 324(M+H)⁺.

A mixture of the compound **7c** (10.00g, 30.95mol), sodium 3-amino-5-chloropyrazine-2-thiolate (6.28g, 34.21mmol), Pd₂(dba)₃ (1.40g, 1.55mmol), Xantphos (1.80g, 3.11mmol) and DIEA (8.00g, 62.13mmol) in dioxane (120mL) was stired at 70°C under N₂ for about 5 hours. The solution was cooled to RT and filtered, the filtrate was removed under reduced pressure, and the resultant residue was washed with EA (50mL), and filtrated to afford the compound **7d** (9.92g, 89.8%). MS: 357 (M+H)⁺.

A mixture of (S)-tert-butyl 4-((R)-1,1-dimethylethylsulfinamido)-2-oxa-8-azaspiro[4.5]decane-8-carboxylate (508mg, 1.41mmol) and TFA (1mL) in DCM (5mL)was stirred at RT for 1 hour, the solvent was removed under reduced pressure, the resultant residue was added K₂CO₃ (0.81g, 5.86mmol), NMP (5mL)and stirred for 5 min, then the compound **7d** (250mg, 0.70mmol) was added, the mixture was heated to 75°C for 2 hours, cooled and concentrated under reduced pressure, the residue was purified by column chromatography to afford the compound **7e** (84mg, 20.7%). MS: 581(M+H)⁺.

A solution of the compound **7e** (185mg, 0.32mmol) in 3mL of dioxane was added HCl/dioxane (1.2mL, 4M). The mixture was stirred at RT. After completion of the reaction, the solution was removed under reduced pressure and the residue was washed with EA (5mL) to afford the compound **7** (136mg, 82.8%) as an HCl salt. MS: 477 (M+H)⁺.

### Example 8 Synthesis of compound 8

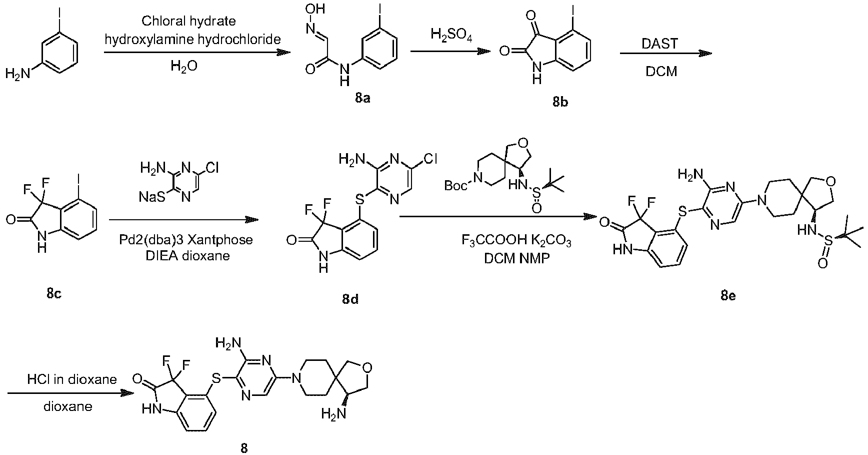

Chloral hydrate (50.02g) and Na₂SO₄ (350.21g) were dissolved in water (700mL) in a 3 L beaker and warmed to 35°C. A warm solution of the appropriate commercial aniline derivative (60.45g, 0.276mol) in water (200mL), and an aqueous solution of concentrated HCl (30mL) was added (a white precipitate of the amine sulfate was formed), followed by a warm solution of hydroxylamine hydrochloride (61.18g, 0.88mol) in water (275mL). The mixture was stirred by hand and heated on a hot plate (a thick paste formed at 75-70 °C) at 80-90 °C for 2 hours and then allowed to cool for 1 hour, by which time the temperature had fallen to 50°C and filtered. The pale cream product was washed by stirring with water (1L) and filtered. Drying overnight at 40 °C gave the compound **8a** (69.74g,87%). MS: 291(M+H)⁺.

Sulfuric acid (1L) was heated in a 3L beaker on a hot plate to 60°C and then removed. The compound **8a** was added in portion with stirring over 30 mins so that the temperature did not exceed 65°C. The mixture was then heated to 80°C for 15 mins, allowed to cool to 70°C, and cooled on ice. The solution was poured on to crushed ice (5L) and left to stand for 1 hour before filtering the orange-red precipitate. The product was washed by stirring with water (400mL) and filtered to give a mixture of the compound **8b** and 6-iodoindoline-2,3-dione. The crude product was dissolved in a solution of NaOH (20g) in water (200mL) at 60 °C and then acidified with acetic acid to pH=4.9. After standing 0.5 hour and cooling to 35°C, the compound **8b** precipitate was filtered and washed with water (50mL) (38.37g, 59%). MS: 274(M+H)⁺.

A mixture of the compound **8b** (2.31g, 8.46mmol), DAST (4.10g, 25.43mmol) and DCM (100mL) was stirred at RT for 24 hours. The mixture was quenched by addition of NaHCO₃ solution and then filtered to give the crude product, the crude product was washed with Hexane to afford the compound **8c** (2.14g,86%). MS: 294(M-H)⁺.

A mixture of the compound **8c** (1.01g, 3.42mmol), Pd₂(dba)₃ (100mg, 0.34mmol), Xantphos (100mg, 0.34mmol), DIEA (883mg, 6.84mmol) and dioxane (30mL) was stirred at 80°C for 30 mins. sodium 3-amino-5-chloropyrazine-2-thiolate (628mg, 3.42mmol) was added then stirred for another 3 hours at 80°C. The mixture was cooled and the volatiles were removed under reduced pressure, the residue was purified by column chromatography to afford the compound **8d** (545mg, 48%). MS: 329(M+H)⁺.

A mixture of (S)-tert-butyl 4-((R)-1,1-dimethylethylsulfinamido)-2-oxa-8-azaspiro[4.5]decane-8-carboxylate (1.18g,3.27mmol), TFA (5mL) and DCM (20mL) was stirred at room temperature for 2 hours. The volatiles were removed under reduced pressure. The residue was added the compound **8d** (542mg, 1.65mmol), K₂CO₃ (1.82g, 13.20mmol) and NMP (12mL) and stirred at 80°C for 10 hours. It was quenched by addition of water (40mL), then exacted with EA (30mL × 5), the combined organic phases were washed with brine (100mL), dried over anhydrous Na₂SO₄, filtered and the volatiles were removed under reduced pressure, the residue was purified by column chromatography to afford the compound **8e** (138mg, 15%). MS: 553(M+H)⁺.

The compound **8e** (138mg, 0.25mmol) was dissolved in dioxane (3mL) and stirred. A solution of HCl/dioxane (0.5mL, 4M) was added then the mixture was stirred at room temperature for 0.5 hour and the volatiles were removed under reduced pressure. The residue was washed with EA (10mL) and filtered to afford the compound **8** as a yellow solid, HCl salt. MS: 449(M+H)⁺.

### Example 9 Synthesis of compound 9

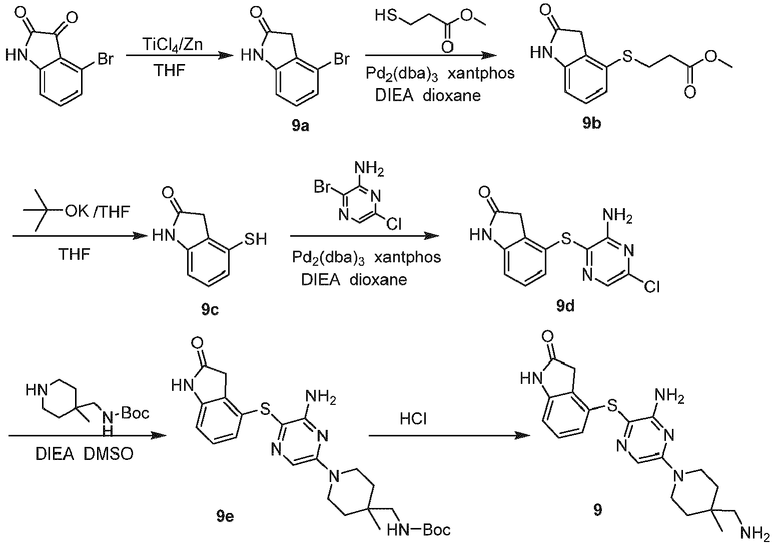

A mixture of Zn power (8.64g, 132.13mmol) and TiCl₄ (12.60g, 66.42mmol) in THF (100mL) was stirred at 80°C for 2 hours, then it was cooled to RT, a solution of 4-bromoindoline-2,3-dione (5.01g, 22.16mmol) in THF (100mL) was added dropwise under N₂ . After completion of the reaction, Hydrochloric acid solution (100mL, 3M) was added, the mixture was extracted with DCM (50mL×3), the organic phase was washed with brine (50mL×2), dried over anhydrous Na₂SO₄, filtered and the solvent was removed under reduced pressure, the residue was purified by column chromatography to afford the compound **9a** (2.63g, 56.0%). MS: 212(M+H)⁺.

A mixture of the compound **9a** (1.00g, 4.72mmol), methyl 3-mercaptopropanoate (1.13g, 9.40mmol), Pd₂(dba)₃ (0.15g, 0.16mmol), Xantphos (0.20g, 0.35mmol) and DIEA (1.23g, 9.52mmol) in dioxane (25mL) was stirred overnight at 100°C under N₂.the solvent was removed under reduced pressure, the residue was purified by column chromatography to afford the compound **9b** (0.73g, 61.5%). MS: 252(M+H)⁺.

A mixture of the compound **9b** (1.65g, 6.56mmol) in THF (50mL) was added t-BuOK/THF (15mL, 1M) dropwise at -70°C, After completion of the reaction, it was quenched with Hydrochloric acid solution (20mL, 1M) and extracted with EA (50mL×3), the organic phase was washed with brine (100mL×2), dried over anhydrous Na₂SO₄, filtered and the solvent was removed under reduced pressure, and the residue of the compound **9c** was used immediately for the next step without further purification (1.08g,100%). MS: 166 (M+H)⁺.

A mixture of the compound **9c** (1.0g, 6.54mmol), 3-bromo-6-chloropyrazin-2-amine (1.37g, 6.57mmol), Pd₂(dba)₃ (0.31g, 0.34mmol), Xantphos (0.40g, 0.69mmol) and DIEA (1.70g, 13.16mmol) in dioxane (80mL) was stirred at 100°C under N₂ for 5 hours. The mixture was cooled to RT and filtered, the filtrate was removed under reduced pressure, the residue was purified by column chromatography to afford the compound **9d** (0.53g, 27.7%). MS: 293 (M+H)⁺.

A mixture of the compound **9d** (146mg, 0.50mmol), tert-butyl ((4-methylpiperidin-4-yl)methyl)carbamate (240mg, 1.05mmol) and DIEA (203mg, 1.57mmol) in DMSO (5mL) was stired at 80°C , after the reaction was completed, cooled to RT, water (20mL) was added, the mixture was extracted with EA (20mL×2), the organic phase was washed with brine (50mL×2) , dried over anhydrous Na₂SO₄, filtered and the solvent was removed under reduced pressure, the residue was purified by column chromatography to afford the compound **9e** (99mg, 40.8 %). MS: 485(M+H)⁺.

A solution of the compound **9e** (24mg, 0.049mmol) in 5mL of DCM was bubbled into HCl gas at RT. After completion of the reaction, water (20mL) was added, the mixture was washed with EA (20mL×2), then the aqueous layer was adjusted to pH=11, extracted with DCM (20mL×2), dried over anhydrous Na₂SO₄, filtered and the filtrate was removed under reduced pressure to afford the compound 9 (16mg, 84.9%). MS: 385(M+H)⁺.

### Example 10 Synthesis of compound 10

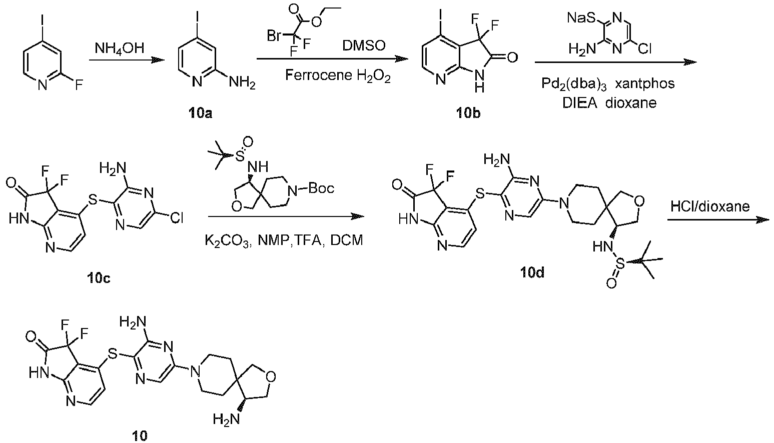

A mixture of 2-fluoro-4-iodopyridine (10.00g, 43.50mmol), ammonium hydroxide (10mL) in DMSO (20mL) was stirred at 100°C for 40 hours. H₂O (100mL) was added to the reaction mixture and the precipitate was filtered to afford the compound 10a as a brown solid (8.62g, 90%). MS: 221 (M+H)⁺.

To a solution of the compound **10a** (8.00g, 36.36mmol), ethyl 2-bromo-2,2-difluoroacetate (18.46g, 90.91mmol) and Ferrocene (0.68g, 3.64mmol) in DMSO (70mL) was added H₂O₂ (8mL) at -5°C. The resulting mixture was stirred at 25°C for 24 hours. H₂O (100mL) was added to the reaction mixture, the aqueous solution was extracted with EA (100mL×2). The combined organic extracts were washed with brine (50mL), dried over anhydrous Na₂SO₄, filtered and the volatiles were removed under reduced pressure in vacuo. The residue was purified by column chromatography to afford the compound **10b** as a yellow solid (3.41g, 32%). MS: 297(M+H)⁺.

A mixture of the compound **10b** (1.48g, 5.00mmol), sodium 3-amino-5-chloropyrazine-2-thiolate (0.92g, 5.00mmol) , DIEA (1.29g, 10.00mmol), Pd₂(dba)₃ (0.15g, 0.16mmol), Xantphos (0.15g, 0.26mmol) in dioxane (20mL) was heated to 95°C under N₂ for 18 hours. The reaction mixture was filtered and concentrated under reduced pressure, the residue was purified by column chromatography afford the compound **10c** (0.59g, 36%). MS: 330(M+H)⁺.

A mixture of (S)-tert-butyl 4-((R)-1,1-dimethylethylsulfinamido)-2-oxa-8-azaspiro[4.5]decane-8-carboxylate (361mg, 1.00mmol) , TFA (1mL) in DCM (5mL) was stirred at 25°C for 1 hour. The reaction mixture was concentrated under reduced pressure, the residue was dissolved in NMP (8mL), then the compound **10c** (330g, 1.00mmol), K₂CO₃ (1.10g, 8.00mmol) was added to mixture and stirred at 80°C for 1 hour. The reaction mixture was filtered and concentrated under reduced pressure, the residue was purified by Pre-TLC to afford the compound **10d** (80mg,14%). MS: 554(M+H)⁺.

To a solution of the compound **10d** (80mg, 0.14mmol) in DCM (10mL) was added HCl/dioxane(1mL, 4M). The resulting mixture was stirred at 25°C for 1 hour and the precipitate was filtered to afford the compound **10** as a brown solid (10mg, 16%). MS: 450 (M+H)⁺.

### Example 11 Synthesis of compound 11

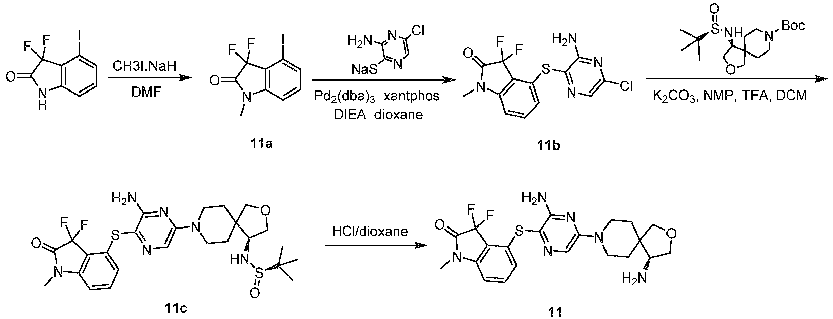

A mixture of the compound **7a** (2.30g, 7.80mmol), NaH (0.94g, 23.39mmol, 60%) in DMF (30mL) was stirred at 25°C for 0.5 hour. Methyl iodide (3.32g, 23.39mmol) was added to the reaction mixture and stirred at 25°C for 1 hour. Reaction was quenched with water (100mL), the aqueous solution was extracted with EA (100mL×2). The combined organic extracts were washed with brine (50mL), dried over anhydrous Na₂SO₄, filtered and the volatiles were removed under reduced pressure in vacuo. The residue was purified by column chromatography to afford the compound **11a** as a yellow solid (1.10g, 46%). MS: 310(M+H)⁺.

A mixture of the compound **11a** (1.10g, 3.56mmol), sodium 3-amino-5-chloropyrazine-2-thiolate (0.65g, 3.56mmol), DIEA (0.92g, 7.12mmol),Pd₂(dba)₃ (0.10g, 0.11 mmol), Xantphos (0.10g, 0.18mmol) in dioxane (30mL) was heated to 95°C under N₂ for 18 hours. The reaction mixture was concentrated under reduced pressure, the residue was purified by column chromatography to afford the compound **11b** (1.06g, 87%). MS: 343(M+H)⁺.

A mixture of (S)-tert-butyl 4-((R)-1,1-dimethylethylsulfinamido)-2-oxa-8-azaspiro[4.5]decane-8-carboxylate (200mg, 0.55mmol) , TFA (2mL) in DCM (10mL) was stirred at 25°C for 1 hour. The reaction mixture was concentrated under reduced pressure. The residue was dissolved in NMP (8mL), then the compound **11b** (190mg, 0.55mmol), K₂CO₃ (613mg, 4.44mmol) was added to mixture and stirred at 90°C for 24 hours. H₂O (50mL) was added to the reaction. The aqueous solution was extracted with EA (50mL×3), The combined organic extracts were washed with brine (50mL), dried over anhydrous Na₂SO₄, filtered and the volatiles were removed in vacuo, the residue was purified byPre-TLC to afford the compound **11c** as a yellow solid (50mg, 16%). MS: 567(M+H)⁺.

To a solution of the compound **11c** (50mg, 0.14mmol) in DCM (5mL) was added HCl/dioxane (1mL, 4M). The resulting mixture was stirred at 25°C for 1 hour. The reaction mixture was concentrated under reduced pressure, the residue was purified by Pre-TLC to afford the compound 11 (3mg, 7%). MS: 463 (M+H)⁺.

### Example 12 Synthesis of compound 12

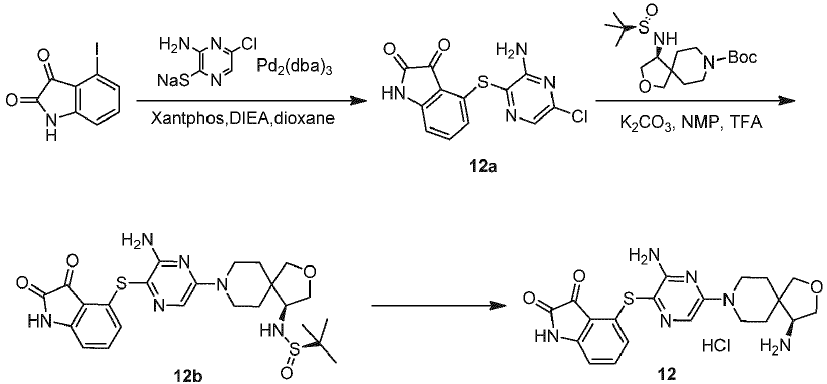

A mixture of 4-iodoindoline-2,3-dione (200mg, 0.73mmol), sodium 3-amino-5-chloropyrazine-2-thiolate (0.13g, 0.73mol), Pd₂(dba)₃ (20mg, 0.02mmol), Xantphos (20mg, 0.035mmol), DIEA (0.19g, 1.46mmol) in dioxane (10mL) was stirred at 95°C under N₂ for 2 hours. After completion of the reaction, the reaction mixture was concentrated under reduced pressure and the residue was purified by column chromatography to afford the compound **12a** (0.19g, 84.86%). MS: 307 (M+H)⁺.

A mixture of (S)-tert-butyl 4-((R)-1,1-dimethylethylsulfinamido)-2-oxa-8-azaspiro[4.5] decane-8-carboxylate (0.28g, 0.74mmol), TFA (1mL), and DCM (5mL) was stirred at 20°C for 1 hour. The mixture was concentrated under reduced pressure, and the residue was added DCM (10mL), concentrated under reduced pressure again. A mixture of the residue, K₂CO₃ (0.68g, 4.96mmol), the compound **12a** (0.19g, 0.62mmol) and NMP (5mL) was heated to 80°C for 18 hours. The reaction mixture was cooled and water (40mL) was added, the resulting mixture was extracted with EA (20mL×2).The combined organic extracts were washed with brine (30mL), dried over anhydrous Na₂SO₄ and evaporated to dryness. The residue was purified by Pre-TLC to afford the compound **12b** (18mg, 5.47 %). MS: 531 (M+H)⁺.

A mixture of the compound **12b** (39mg, 0.08mmol), HCl/dioxane (4mol/L, 1mL), and dioxane (2mL) was stirred at 20°C for 2 hours. The mixture was concentrated under reduced pressure, the residue was added EA (10mL) and stirred for 5 mins. The precipitate was filtered to afford the compound **12** (4mg, 25.47 %) as an HCl salt. MS: 427 (M+H)⁺.

### Example 13 Synthesis of compound 13

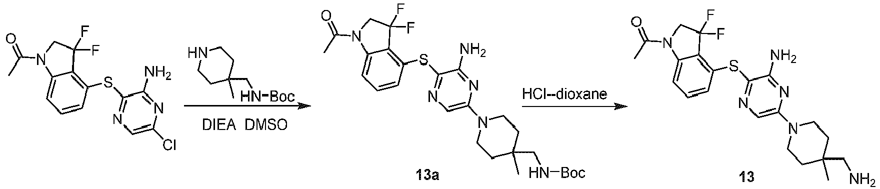

A mixture of the compound **7d** (80mg, 0.22mol), DIEA (101mg, 0.78mmol) and tert-butyl ((4-methylpiperidin-4-yl)methyl)carbamate (200mg, 0.88mmol) in DMSO (5mL) was stirred at 80°C , after the reaction was completed, the mixture was cooled to RT, water (20mL) was added, the mixture was extracted with EA (20mL×2), the organic phase was washed with brine (50mL×2) , dried over anhydrous Na₂SO₄, filtered and the solvent was removed under reduced pressure, the residue was purified by column chromatography to afford the compound **13a** (120mg, 100%). MS: 549(M+H)⁺.

A solution of the compound **13a** (120mg, 0.22mmol) in dioxane (4mL) was added HCl/dioxane (5mL, 4M). The mixture was stirred at RT under ultrasonic for about 5 mins. After completion of the reaction, the solution was removed under reduced pressure and the residue was washed with EA (5mL) to afford the compound **13** (85mg, 79.7%) as an HCl salt. MS: 449(M+H)⁺.

### Example 14 Synthesis of compound 14

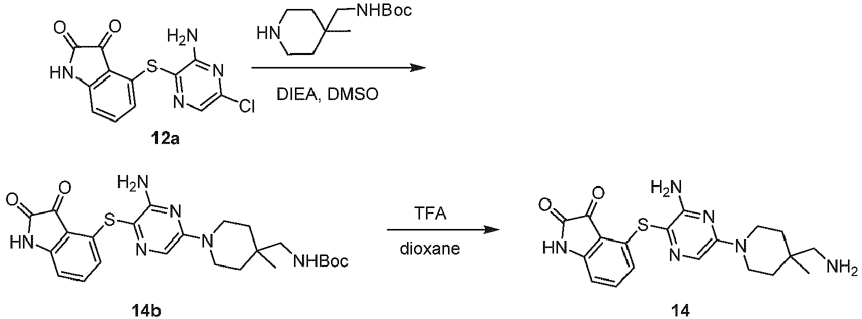

A mixture of the compound **12a** (0.11g, 0.36mmol), tert-butyl ((4-methylpiperidin-4-yl)methyl)Carbamate (0.25g, 1.08mmol), DIEA (93mg, 0.72mmol) and DMSO (10mL) was heated to 80°C for 17 hours. The reaction mixture was cooled and water (50mL) was added, the resulting mixture was extracted with EA (30mL×2). The combined organic extracts were washed with brine (60mL), dried over anhydrous Na₂SO₄, filtered and the solvent was removed under reduced pressure. The residue was purified by Pre-TLC to afford the compound **14b** (0.13g, 72.43%). MS: 499 (M+H)⁺.

A mixture of the compound **14b** (0.13g,0.26mmol), TFA (1mL) and DCM (5mL) was stirred at 20°C for 2 hours. The mixture was concentrated under reduced pressure, the residue was added EA (10mL), stirred for 5min.The precipitate was filtered to afford the compound **14** (25mg, 18.76%) as an TFA salt. MS: 399 (M+H)⁺.

| Ex No | Chemical Name | Structure | Physical Data (MS) (M+H)⁺ |
|---|---|---|---|
| 15 | 5-((2-amino-3-chloropyridin-4-yl)thio)-6-( 4-(aminomethy1)-4-methylpiperidin-1-yl)p yrazin-2-amine | 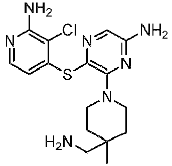 | 380 |
| 16 | N¹-(4-((3-amino-5-(4-(aminomethyl)-4-me thylpiperidin-1 -yl)pyrazin-2-yl)thio)-3 -chl oropyridin-2-yl)-N2,N2-dimethyloxalamid e | 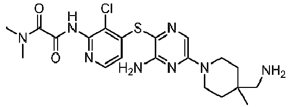 | 479 |
| 17 | tert-butyl ((1-(6-amino-5-((2-amino-3-chloropyridin -4-yl)thio)pyrazin-2-yl)-4-methylpiperidin-4-yl)methyl)carbamate | 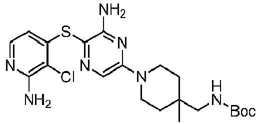 | 480 |
| 18 | (S)-N1-(4-((3-amino-5-(4-amino-2-oxa-8-azaspiro[4.5]decan-8-yl)pyrazin-2-yl)thio) -3-chloropyridin-2-yl)-N2,N2-dimethylox alamide | 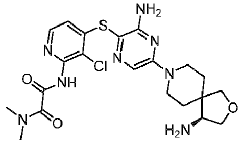 | 507 |
| 19 | (S)-N¹-(3-((3-amino-5-(4-amino-2-oxa-8-a zaspiro[4.5]decan-8-yl)pyrazin-2-yl)thio)-2-chlorophenyl)-N²,N²-dimethyloxalamide | 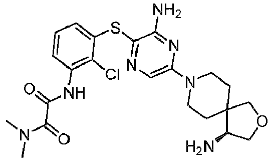 | 506 |
| 20 | N¹-(3-((3-amino-5-(4-(aminomethyl)-4-me thylpiperidin-1-yl)pyrazin-2-yl)thio)-2-chl orophenyl)-N²,N²-dimethyloxalamide | 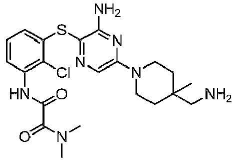 | 478 |
| 21 | N-(3-((3-amino-5-(4-(aminomethyl)-4-met hylpiperidin-1-yl)pyrazin-2-yl)thio)-2-chlo rophenyl)-3-(2-(dimethylamino)-2-oxoacet yl)benzamide | 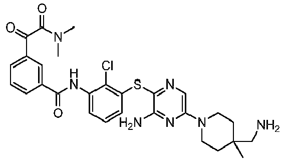 | 582 |
| 22 | 2-(3-(3-(3-((3-amino-5-(4-(aminomethyl)-4-methylpiperidin-1-yl)pyrazin-2-yl)thio)-2-chlorophenyl)ureido)phenyl)-N,N-dimet hyl-2-oxoacetamide | 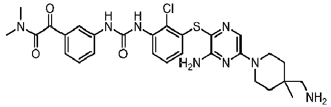 | 597 |
| 23 | 2-(4-(3 -(3 -((3 -amino-5-(4-(aminomethyl)-4-methylpipendin-1-yl)pyrazin-2-yl)thio)-2-chlorophenyl)ureido)phenyl)-N,N-dimet hyl-2-oxoacetamide | 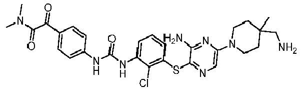 | 597 |
| 24 | 6-(4-(aminomethyl)-4-methylpiperidin-1-y 1)-3-((3,4-dihydro-2H-benzo[b][1,4]oxazin -6-yl)thio)pyrazin-2-amine | 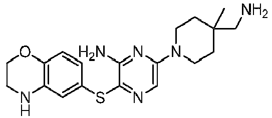 | 387 |
| 25 | tert-butyl (1-(6-amino-5-((2-amino-3-chloropyridin-4-yl)thio)pyrazin-2-yl)-4-methylpiperidin-4-yl)carbamate | 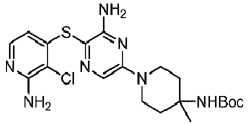 | 466 |
| 26 | tert-butyl (1-(5-((2-acrylamido-3-chloropyridin-4-yl) thio)-6-aminopyrazin-2-yl)-4-methylpiperi din-4-yl)carbamate | 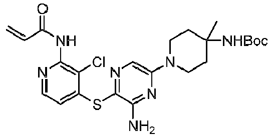 | 520 |
| 27 | tert-butyl (1-(6-amino-5-((3-chloro-2-(2-(dimethyla mino)-2-oxoacetamido)pyridin-4-yl)thio)p yrazin-2-yl)-4-methylpiperidin-4-yl)carba mate | 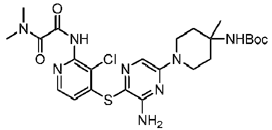 | 565 |
| 28 | tert-butyl (1-(6-amino-5-((2-chloro-3-(2-(dimethyla mino)-2-oxoacetamido)phenyl)thio)pyrazi n-2-yl)-4-methylpiperidin-4-yl)carbamate | 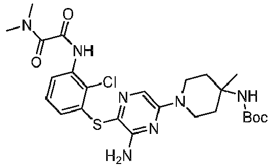 | 564 |
| 29 | N¹-(3-((3-amino-5-(4-amino-4-methylpipe ridin-1 -yl)pyrazin-2-yl)thio)-2-chlorophen yl)-N²,N²⁻dimethyloxalamide | 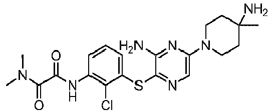 | 464 |
| 30 | tert-butyl (1-(6-amino-5-((3-amino-2-chlorophenyl)t hio)pyrazin-2-yl)-4-methylpiperidin-4-yl)c arbamate | 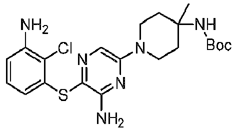 | 465 |
| 31 | N-(3-((3-amino-5-(4-amino-4-methylpiper idin-1-yl)pyrazin-2-yl)thio)-2-chloropheny 1)-2-oxo-2-(p-tolyl)acetamide | 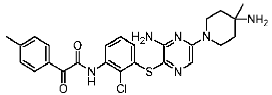 | 511 |
| 32 | tert-butyl (1-(5-((3-acrylamido-2-chlorophenyl)thio) -6-aminopyrazin-2-yl)-4-methylpiperidin-4-yl)carbamate | 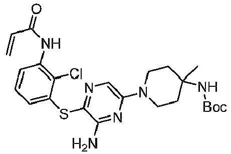 | 519 |
| 33 | 6-(3-amino-5-(4-amino-4-methylpiperidin-1-yl)pyrazin-2-yl)-2H-benzo[b][1,4]oxazi n-3(4H)-one | 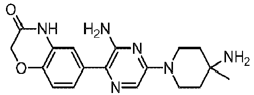 | 355 |
| 34 | N-(4-((3-amino-5-(4-amino-4-methylpiper idin-1-yl)pyrazin-2-yl)thio)-3-chloropyridi n-2-yl)-4-(2-(dimethylamino)-2-oxoacetyl) benzamide | 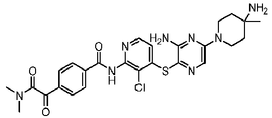 | 569 |
| 35 | N-(4-((3-amino-5-(4-(aminomethyl)-4-met hylpiperidin-1-yl)pyrazin-2-yl)thio)-3-chlo ropyridin-2-yl)-4-(2-(dimethylamino)-2-ox oacetyl)benzamide | 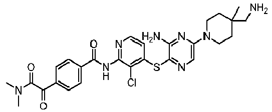 | 583 |
| 36 | N-(3-((3-amino-5-(4-(aminomethyl)-4-met hylpiperidin-1-yl)pyrazin-2-yl)thio)-2-chlo rophenyl)-4-(2-(dimethylamino)-2-oxoacet yl)benzamide | 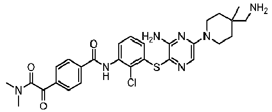 | 582 |
| 37 | 5-((2-amino-3-chloropyridin-4-yl)thio)-N2 -cyclohexylpyrazine-2,6-diamine | 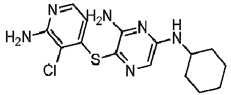 | 351 |
| 38 | (S)-8-(5-((1H-pyrrolo[2,3-b]pyridin-4-yl)t hio)-6-aminopyrazin-2-yl)-2-oxa-8-azaspir o[4.5]decan-4-amine | 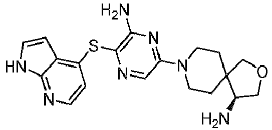 | 398 |
| 39 | (S)-8-(6-amino-5-((3,3-dimethylindolin-4-yl)thio)pyrazin-2-yl)-2-oxa-8-azaspiro[4.5 ]decan-4-amine | 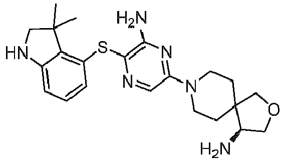 | 427 |
| 40 | (S)-8-(6-amino-5-((3-fluoro-1H-indol-4-yl )thio)pyrazin-2-yl)-2-oxa-8-azaspiro[4.5]d ecan-4-amine | 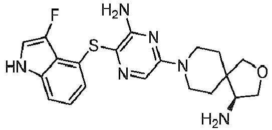 | 415 |
| 41 | 5-((2-amino-3-chloropyridin-4-yl)thio)-N2 -(4-(aminomethyl)-4-methylcyclohexyl)py razine-2,6-diamine | 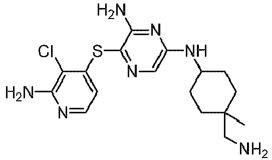 | 394 |
| 42 | (S)-8-(5-((1H-indol-4-yl)thio)-6-aminopyr azin-2-yl)-2-oxa-8-azaspiro[4.5]decan-4-a mine | 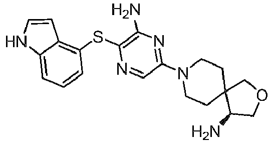 | 397 |
| 43 | (S)-1-(4-((3-amino-5-(4-amino-2-oxa-8-az aspiro[4.5]decan-8-yl)pyrazin-2-yl)thio)-1 H-indol-1-yl)ethanone | 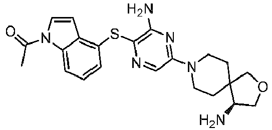 | 439 |
| 44 | 5-((2-amino-3-chloropyridin-4-yl)thio)-N2 -(4-amino-4-methylcyclohexyl)pyrazine-2, 6-diamine | 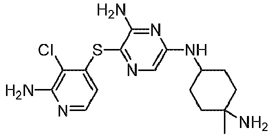 | 380 |
| 45 | (S)-6-((3-amino-5-(4-amino-2-oxa-8-azasp iro[4.5]decan-8-yl)pyrazin-2-yl)thio)-2H-b enzo[b][1,4]oxazin-3(4H)-one | 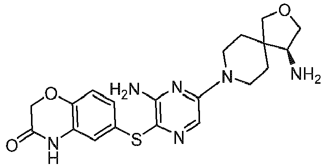 | 429 |
| 46 | (S)-4-((3-amino-5-(4-amino-2-oxa-8-azasp iro[4.5]decan-8-yl)pyrazin-2-yl)thio)-1,3,3 -trimethylindolin-2-one | 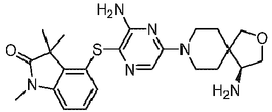 | 455 |
| 47 | (4S)-8-(6-amino-5-((3-fluoroindolin-4-yl)t hio)pyrazin-2-yl)-2-oxa-8-azaspiro[4.5]de can-4-amine | 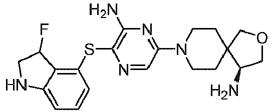 | 417 |
| 48 | 1-(4-((3-amino-5-((S)-4-amino-2-oxa-8-az aspiro[4.5]decan-8-yl)pyrazin-2-yl)thio)-3 - fluoro-3-methylindolin-1-yl)ethanone | 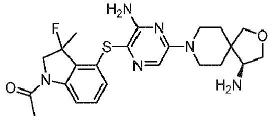 | 473 |
| 49 | (S)-8-(6-amino-5-((3,3-difluoroindolin-4-y 1)thio)pyrazin-2-yl)-2-oxa-8-azaspiro[4.5] decan-4-amine | 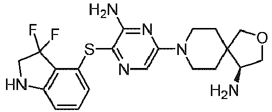 | 435 |
| 50 | 1-(4-((3-amino-5-((S)-4-amino-2-oxaspiro [4.5]decan-8-yl)pyrazin-2-yl)thio)-3-meth ylindolin-1-yl)ethanone | 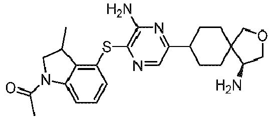 | 454 |
| 51 | (S)- 8-(6-amino-5-((8-chloro-4,4-difluoro-1,2,3,4-tetrahydroquinolin-5-yl)thio)pyrazi n-2-yl)-2-oxa-8-azaspiro[4.5]decan-4-ami ne | 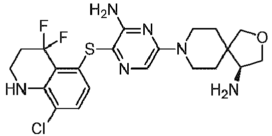 | 483 |
| 52 | (4S)-8-(6-amino-5-((8-chloro-4-fluoro-1,2, 3,4-tetrahydroquinolin-5-yl)thio)pyrazin-2 -yl)-2-oxa-8-azaspiro[4.5]decan-4-amine | 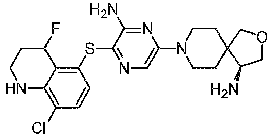 | 465 |
| 53 | (S)-8-(6-amino-5-((3,3-difluoro-1-methyli ndolin-4-yl)thio)pyrazin-2-yl)-2-oxa-8-aza spiro [4.5]decan-4-amine | 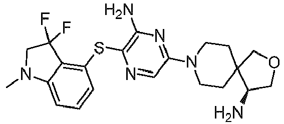 | 449 |
| 54 | (S)-6-((3-amino-5-(4-amino-2-oxa-8-azasp iro[4.5]decan-8-yl)pyrazin-2-yl)thio)-3,3-d ifluoroindolin-2-one | 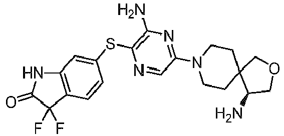 | 449 |
| 55 | 4-((3-amino-5-((S)-4-amino-2-oxa-8-azasp iro[4.5]decan-8-yl)pyrazin-2-yl)thio)-3-flu oroindolin-2-one | 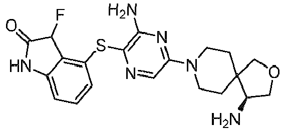 | 431 |
| 56 | (S)-8-(6-amino-5-((3,3-difluoro-2,3-dihydr obenzofuran-4-yl)thio)pyrazin-2-yl)-2-oxa -8-azaspiro[4.5]decan-4-amine | 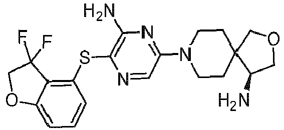 | 436 |
| 57 | (S)-8-(6-amino-5-((4,4-difluorochroman-5 -yl)thio)pyrazin-2-yl)-2-oxa-8-azaspiro[4. 5]decan-4-amine | 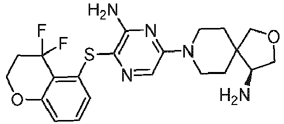 | 450 |
| 58 | 4-((3-amino-5-((S)-4-amino-2-oxa-8-azasp iro[4.5]decan-8-yl)pyrazin-2-yl)thio)-3-flu oro-1-methyl-3-(trifluoromethyl)indolin-2-one | 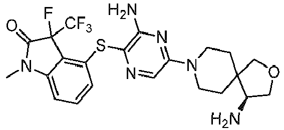 | 513 |
| 59 | (S)-6-((3-amino-5-(4-amino-2-oxa-8-azasp iro[4.5]decan-8-yl)pyrazin-2-yl)thio)-7-chl oroindolin-2-one | 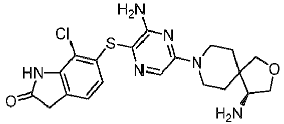 | 447 |
| 60 | (S)-8-(6-amino-5-((5-chloro-3,4-dihydro-2 H-benzo[b][1,4]oxazin-6-yl)thio)pyrazin-2 -yl)-2-oxa-8-azaspiro[4.5]decan-4-amine | 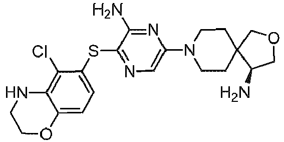 | 449 |
| 61 | (S)-7-((3-amino-5-(4-amino-2-oxa-8-azasp iro[4.5]decan-8-yl)pyrazin-2-yl)thio)-8-chl oro-3,4-dihydroquinolin-2(1H)-one | 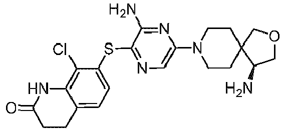 | 461 |
| 62 | (S)-6-((3-amino-5-(4-amino-2-oxa-8-azasp iro[4.5]decan-8-yl)pyrazin-2-yl)thio)-5-chl oro-2H-benzo[b][1,4]oxazin-3(4H)-one | 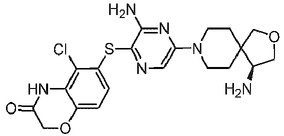 | 463 |
| 63 | (S)-2-(3-((3-amino-5-(4-amino-2-oxa-8-az aspiro[4.5]decan-8-yl)pyrazin-2-yl)thio)-2 -chlorophenyl)-N,N-dimethyl-2-oxoaceta mide | 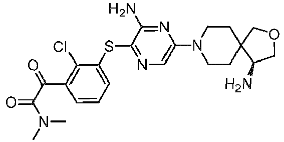 | 491 |
| 64 | 4-((3-amino-5-(4-(aminomethyl)-4-methyl piperidin- 1-yl)pyrazin-2-yl)thio)-3,3-diflu oro-1H-pyrrolo[2,3-b]pyridin-2(3H)-one | 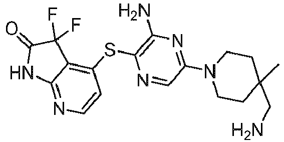 | 422 |
| 65 | (S)-4-((3-amino-5-(4-amino-2-oxa-8-azasp iro[4.5]decan-8-yl)pyrazin-2-yl)thio)-1H-b enzo[d]imidazol-2(3H)-one | 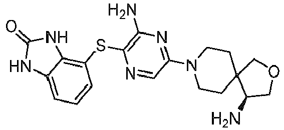 | 414 |
| 66 | (S)-4-((3-amino-5-(4-amino-2-oxa-8-azasp iro[4.5]decan-8-yl)pyrazin-2-yl)thio)-1,3-d imethyl-1H-benzo[d]imidazol-2(3H)-one | 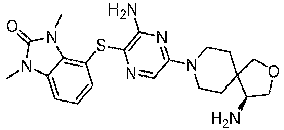 | 442 |
| 67 | (S)-8-(6-amino-5-((2,2-difluoro-2,3-dihydr o-1H-benzo [d]imidazol-4-yl)thio)pyrazin-2-yl)-2-oxa-8-azaspiro[4.5]decan-4-amine | 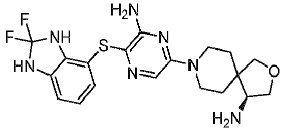 | 436 |
| 68 | (S)-8-(6-amino-5-((2,2-difluoro-1,3-dimet hyl-2,3-dihydro-1H-benzo[d]imidazol-4-yl )thio)pyrazin-2-yl)-2-oxa-8-azaspiro[4.5]d ecan-4-amine | 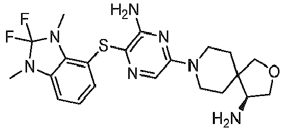 | 464 |
| 69 | (4S)-8-(6-amino-5-((1-amino-3,3-difluoro-2,3-dihydro-1H-inden-4-yl)thio)pyrazin-2-yl)-2-oxa-8-azaspiro[4.5]decan-4-amine | 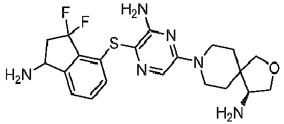 | 449 |
| 70 | (S)-5-((3-amino-5-(4-amino-2-oxa-8-azasp iro[4.5]decan-8-yl)pyrazin-2-yl)thio)-2-me thyl-[1,2,4]triazolo[4,3-a]pyridin-3(2H)-o ne | 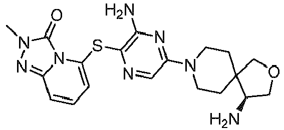 | 429 |
| 71 | (S)-8-(6-amino-5-((3,3-difluoro-2-methyl-2,3-dihydro-[1,2,4]triazolo[4,3-a]pyridin-5 -yl)thio)pyrazin-2-yl)-2-oxa-8-azaspiro[4. 5]decan-4-amine | 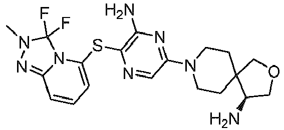 | 451 |
| 72 | (S)-1-(4-((3-amino-5-(4-amino-2-oxa-8-az aspiro[4.5]decan-8-yl)pyrazin-2-yl)thio)in dolin-1-yl)ethanone | 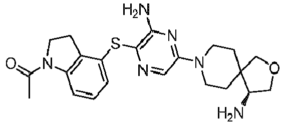 | 441 |
| 73 | 1'-(6-amino-5-((2-amino-3-chloropyridin-4-yl)thio)pyrazin-2-yl)hexahydrospiro[cyc lopenta[b]furan-5,4'-piperidin]-4-amine | 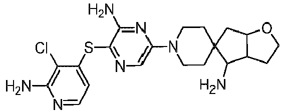 | 448 |
| 74 | 1'-(6-amino-5-((2-amino-3-chloropyridin-4-yl)thio)pyrazin-2-yl)spiro[bicyclo[3.1.0] hexane-3,4'-piperidin]-2-amine | 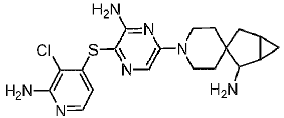 | 418 |
| 75 | 1'-amino-1-(6-amino-5-((2-amino-3-chloro pyridin-4-yl)thio)pyrazin-2-yl)tetrahydros piro[piperidine-4,2'-pyrrolizin]-3'(1'H)-one | 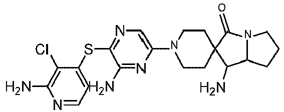 | 461 |
| 76 | 1'-(6-amino-5-((2-amino-3-chloropyridin-4-yl)thio)pyrazin-2-yl)spiro[bicyclo[3.1.0] hexane-2,4'-piperidin]-3-amine | 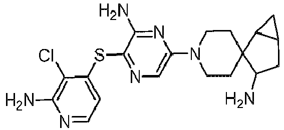 | 418 |

### PHARMACOLOGICAL TESTING

### Example A. Phosphatase Assay (single dose inhibition)

### Assay Protocol:

For single dose inhibition assays using 6,8-difluoro-4-methylumbelliferyl phosphate (DiFMUP) as a substrate, SHP2 samples (diluted to 0.5 nM in reaction buffer) were incubated with dPEG8 peptide for 30 min in reaction buffer[60 mM 3,3-dimethyl glutarate (pH7.2), 75 mM NaCl, 75 mM KCl, and 1 mM EDTA, 0.05% Tween 20, 2mM dithiothreitol (DTT) ] to active the PTP. DMSO [0.5% (v/v)] or compounds (100nM) were added to the mixture and incubated for 30 min at room temperature. Reactions were initiated by the addition of DiFMUP (12 µM; total reaction volume of 100 µL), and the fluorescence (excitation at 340 nm, emission at 450 nm) of the resulting solutions was measured on a 2104-0020 EnVision Xcite Multilabel Reader (PerkinElmer) after 30min. The experiment is carried out in triplicate. The value for the control sample (DMSO) was set to 100%, and the values for the compound-treated samples were expressed as activity relative to the control sample. The inhibition of SHP2 by compounds of the invention were shown in table 1.

**Table 1**

| Example | SHP2 inhibition(%) | Example | SHP2 inhibition(%) |
|---|---|---|---|
| 3@0.1µM | 60 | 7@0.1µM | 79 |
| 8@0.1µM | 76 | 10@0.1µM | 81 |
| 20@0.1µM | 57 | 29@0.1µM | 47 |
| 31 @0.1µM | 53 | 32 @0.1µM | 49 |
| 34 @0.1µM | 37 | 35 @0.1µM | 30 |
| 38 @0.1µM | 30 | 40 @0.1µM | 71 |
| 42@0.1µM | 58 | 43@0.1µM | 43 |
| 58 @0.1µM | 61 | 72@0.1µM | 31 |
| 74@0.1µM | 75 | 75@0.1µM | 35 |

### Example B.Phosphatase Assays (IC50)

IC₅₀ values were estimated using 6,8-difluoro-4-methylumbelliferyl phosphate (DiFMUP) as a substrate, SHP2 samples (diluted to 0.5 nM in reaction buffer) were incubated with dPEG8 peptide for 30 min in reaction buffer[60 mM 3,3-dimethyl glutarate (pH7.2), 75 mM NaCl, 75 mM KCl, and 1 mM EDTA, 0.05% Tween 20, 2mM dithiothreitol (DTT) ] to active the PTP. DMSO [0.5% (v/v)] or compounds (concentrations ranging from 0.3 nM to 1 µM) were added to the mixture and incubated for 30 min at room temperature. Reactions were initiated by the addition of DiFMUP (12 µM; total reaction volume of 100 µL), and the fluorescence (excitation at 340 nm, emission at 450 nm) of the resulting solutions was measured on a 2104-0020 EnVision Xcite Multilabel Reader (PerkinElmer) after 30min. The IC₅₀ results of the compounds of the invention were shown by table 2.

**Table 2**

| Example | IC₅₀(nM) | Example | IC₅₀(nM) |
|---|---|---|---|
| 7@ | 25.8 | 8@ | 21.4 |
| 10@ | 30 | 40@ | 40.6 |
| 74@ | 8.3 | SHP099 | 84 |

### Example C. Cell Proliferation Assay

KYSE-520 (1500 cells/well) were plated onto 96-well plates in 100µL medium (RPMI-1640 containing 3% FBS for KYSE-520 cells, Gibco). For drug treatment, compounds of the invention at various concentrations were added 24 hours after cell plating. At day 8, 50µL MTS/PMS reagents (Promega/Sigma) were added, and the absorbance value was determined according to the supplier's instruction (Promega). The IC₅₀ results of the compounds of the invention were shown by table 3.

**Table 3**

| Example | IC₅₀((µM) | Example | IC₅₀((µM) |
|---|---|---|---|
| 1 | 15.94 | 7 | 2.17 |
| 8 | 26.38 | 11 | 12.04 |
| 13 | 20.57 | 74 | 3.38 |

The compounds of the present invention are preferably formulated as pharmaceutical compositions administered by a variety of routes. Most preferably, such compositions are for oral administration. Such pharmaceutical compositions and processes for preparing the same are well known in the art. See, e.g., REMINGTON: THE SCIENCE AND PRACTICE OF PHARMACY (A. Gennaro, et al, eds., 19th ed., Mack Publishing Co., 1995). The compounds of Formula I are generally effective over a wide dosage range.

For example, dosages per day normally fall within the range of about 1 mg to about 200 mg total daily dose, preferably 1 mg to 150 mg total daily dose, more preferably 1 mg to 50 mg total daily dose. In some instances dosage levels below the lower limit of the aforesaid range may be more than adequate, while in other cases still larger doses may be employed. The above dosage range is not intended to limit the scope of the invention in any way. It will be understood that the amount of the compound actually administered will be determined by a physician, in the light of the relevant circumstances, including the condition to be treated, the chosen route of administration, the actual compound or compounds administered, the age, weight, and response of the individual patient, and the severity of the patient's symptoms.

The invention is also described by reference to the following clauses:
1. A compound of Formula I or a pharmaceutically acceptable salt: 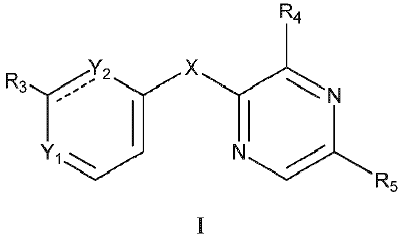
   X is absent, O, S, SO, S(O)₂, C(O), C(O)R₁₁, CR₁₁R₁₂, or -NR₁₁; and each R₁₁ and R₁₂ is independently -H, halogen, -NH₂, -CN, -OH, -NO₂, carbonyl, =O, oxo, carboxyl, substituted or unsubstituted C₁₋₆alkoxy, or substituted or unsubstituted C₁₋₆alkyl;
   Y₁ is Nor CR₁;
   Y₂ is N or CR₂;
   each R₁ and R₂ is independently -H, halogen, -CN, -OH, -NH₂, -N₃, -NO₂, substituted or unsubstituted C₁₋₆alkoxy, or substituted or unsubstituted C₁₋₆alkyl; or
   R₁ combines with R₃, or R₂ combines with R₃, to form a 5-10 member heteroaryl, 5-10 member carbocyclic or 5-10 member heterocyclic ring, wherein each of the ring systems is independently optionally substituted with halogen, -CN, -OH, -NR₈R₉, -N₃, -NO₂, carbonyl, =O, oxo, substituted or unsubstituted C₁₋₆alkyl, substituted or unsubstituted C₁₋₆alkoxy, or C(O)R₈; or
   R₃ is -H, halogen, -CN, -OH, -N₃, -NO₂, -NR₈R₉, -N(R₈)(CH₂)ₚNR₈R₉, -N(R₈)(CH₂)ₚR₈, -N(R₈)GpR₈, -N(R₈)GₚNR₈R₉, -N(R₈)(C=O)_{q}R₈, -N(R₈)(C=O)_{q}NR₈R₉, -N(R₈)(C=O)_{q}GₚR₈, -N(R₈)(C=O)_{q}GₚNR₈R₉, -N(R₈)(C=O)_{q}Gₚ(C=O)_{q}R₈, -N(R₈)(C=O)_{q}Gₚ(C=O)_{q}NR₈R₉, -N(R₈)(C=O)_{q}N(R₈)(C=O)_{q}R₈, -N(R₈)(C=O)_{q}N(R₈) (C=O)_{q}NR₈R₉, -N(R₈)(C=O)_{q}N(R₈)G_{q}(C=O)ₚR₈, -N(R₈)(C=O)_{q}N(R₈)Gₚ(C=O)_{q}NR₈R₉, C(O)_{q}R₈, C(O)OR₈, C(O)NH₂, C(O)NHR₈, C(O)NR₈R₉, C₁₋₆alkyl, C₆₋₁₀aryl, arylalkyl, alkoxy, heteroaryl, heterocyclic, or carbocyclic; and each of which may be optionally substituted; and each p and q is independently 0, 1, 2 or 3;
   each G is independently C₆₋₁₀ary, C₃₋₈carbocyclic or C₅₋₁₀heteroaryl; and each of which may be optionally substituted;
   R₄ is -H, halogen, -CN, -OH, -NR₈R₉, -N₃, -NO₂, substituted or unsubstituted C₁₋₆alkoxy, substituted or unsubstituted C₁₋₆alkyl, C₅₋₁₈heterocyclic or C₅₋₁₈carbocyclic; wherein each of the ring systems is independently optionally substituted with halogen, -CN, -OH, -NO₂, carbonyl, =O, oxo, substituted or unsubstituted C₁₋₆alkyl, substituted or unsubstituted C₁₋₆alkoxy, -NR₈R₉ or -CH₂NR₈R₉;
   R₅ is -H, halogen, -CN, -OH, -NR₈R₉, -N₃, -NO₂, C₁₋₆alkyl, C₁₋₆alkoxy, C₆₋₁₀aryl, C₆₋₁₀arylalkyl, C₆₋₁₀heteroaryl, C₅₋₁₈heterocyclic or C₅₋₁₈carbocyclic; and each of which is independently optionally substituted;
   each R₈ and R₉ is independently -H, halogen, -CN, -OH, -N₃, -NO₂, C₁₋₆alkyl, C₁₋₆alkoxy, C₂₋₆alkenyl, NH(C₁₋₆alkyl), N(C₁₋₆alkyl)₂, C₅₋₁₀heterocyclicor C₅₋₁₀carbocyclic; and each of which may be independently optionally substituted.
2. The compound of clause 1, wherein R₁ is -H, -F, -Cl, -Br, -I, -CN, -OH, -NH₂, substituted or unsubstituted C₁₋₃alkoxy, or substituted or unsubstituted C₁₋₃alkyl.
3. The compound of clause 1 or 2, wherein R₁ is -H, -F, -Cl, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.
4. The compound of any one of clauses 1-3, wherein R₂ is -H, -F, -Cl, -Br, -I, -CN, -OH, -NH₂, substituted or unsubstituted C₁₋₃alkoxy, or substituted or unsubstituted C₁₋₃alkyl.
5. The compound of any one of clauses 1-4, wherein R₂ is -H, -F, -Cl, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.
6. The compound of any one of clauses 1-5, wherein R₃ is -H, -F, -Cl, -Br, -CN, -OH, -NO₂, -NR₈R₉, -N(R₈)(CH₂)ₚNR₈R₉, -N(R₈)(CH₂)ₚR₈, -N(R₈)GpR₈, -N(R₈)GₚNR₈R₉, -N(R₈)(C=O)_{q}R₈, -N(R₈)(C=O)_{q}NR₈R₉, -N(R₈)(C=O)_{q}GₚR₈, -N(R₈)(C=O)_{q}GₚNR₈R₉, -N(R₈)(C=O)_{q}Gₚ(C=O)_{q}R₈, -N(R₈)(C=O)_{q}Gₚ(C=O)_{q}NR₈R₉, -N(R₈)(C=O)_{q}N(R₈)(C=O)_{q}R₈, -N(R₈)(C=O)_{q}N(R₈) (C=O)_{q}NR₈R₉, -N(R₈)(C=O)_{q}N(R₈)G_{q}(C=O)ₚR₈, -N(R₈)(C=O)_{q}N(R₈)Gₚ(C=O)_{q}NR₈R₉, C(O)_{q}R₈, C(O)OR₈, C(O)NH₂, C(O)NHR₈, C(O)NR₈R₉, C₁₋₆alkyl or C₆₋₁₀aryl; and each of which may be optionally substituted with halogen, -CN, -OH, -NH₂, -N₃, -NO₂, substituted or unsubstituted C₁₋₆alkyl, or substituted or unsubstituted C₁₋₆alkoxy.
7. The compound of any one of clauses 1-6, wherein R₃ is -H, -F, -Cl, -Br, -NR₈R₉, -N(R₈)(CH₂)ₚNR₈R₉, -N(R₈)(CH₂)ₚR₈, -N(R₈)GpR₈, -N(R₈)GₚNR₈R₉, -N(R₈)(C=O)_{q}R₈, -N(R₈)(C=O)_{q}NR₈R₉, -N(R₈)(C=O)_{q}GₚR₈, -N(R₈)(C=O)_{q}GₚNR₈R₉, -N(R₈)(C=O)_{q}Gₚ(C=O)_{q}R₈, -N(R₈)(C=O)_{q}Gₚ(C=O)_{q}NR₈R₉, -N(R₈)(C=O)_{q}N(R₈)(C=O)_{q}R₈, -N(R₈)(C=O)_{q}N(R₈) (C=O)_{q}NR₈R₉, -N(R₈)(C=O)_{qN}(R₈)G_{q}(C=O)ₚR₈, -N(R₈)(C=O)_{qN}(R₈)Gₚ(C=O)_{q}NR₈R₉ or C(O)_{q}R₈, and each of which may be optionally substituted with -F, -Cl, -Br, -I, -CN, -OH, -NH₂, -N₃, -NO₂, substituted or unsubstituted C₁₋₃alkyl, or substituted or unsubstituted C₁₋₃alkoxy.
8. The compound of any one of clauses 1-7, wherein R₃ is -H, -NR₈R₉, -N(R₈)(C=O)_{q}R₈, -N(R₈)(C=O)_{q}NR₈R₉, -N(R₈)(C=O)_{q}GₚR₈, -N(R₈)(C=O)_{q}Gₚ(C=O)_{q}NR₈R₉, -N(R₈)(C=O)_{q}N(R₈)Gₚ(C=O)_{q}NR₈R₉ or C(O)_{q}R₈; and each of which may be optionally substituted with -F, -Cl, -Br, -NHmethyl, -NHethyl, -NHpropyl, -NHisopropyl, -NHOCH₃, -NHOCH₂CH₃, -NHCH₂OCH₃, -NHOCH₂CH₂CH₃, -NHCH₂OCH₂CH₃, -NHCH₂CH₂OCH₃, -NHOCH(CH₃)₂, -NHCH(OCH₃)₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with -F, -Cl, -Br or -I.
9. The compound of any one of clauses 1-8, wherein each G is independently 6-membered aryl, 7-membered aryl, 8-membered aryl, 3-membered carbocyclic, 4-membered carbocyclic, 5-membered carbocyclic, 6-membered carbocyclic or 7-membered carbocyclic; and each of which may be optionally substituted with halogen, -CN, -OH, -NH₂, -N₃, -NO₂, substituted or unsubstituted C₁₋₆alkyl, or substituted or unsubstituted C₁₋₆alkoxy.
10. The compound of any one of clauses 1-9, wherein each G is independently 6-membered aryl, 7-membered aryl, 8-membered aryl, 3-membered carbocyclic, 4-membered carbocyclic, 5-membered carbocyclic or 6-membered carbocyclic; and each of which may be optionally substituted with -F, -Cl, -Br, -CN, -OH, -NH₂, -NO₂, substituted or unsubstituted C₁₋₃alkyl, or substituted or unsubstituted C₁₋₃alkoxy.
11. The compound of any one of clauses 1-10, wherein each G is independently phenyl, and which may be optionally substituted with -F, -Cl, -OH, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with -F, -Cl, -Br or -I.
12. The compound of any one of clauses 1-11, wherein each G is independently 5-membered heteroaryl, 6-membered heteroaryl, 7-membered heteroaryl or 8-membered heteroaryl; and each of which contains 1, 2, 3 or 4 heteroatoms select from N, O or S, and may be optionally substituted with halogen, -CN, -OH, -NH₂, -N₃, -NO₂, substituted or unsubstituted C₁₋₆alkyl, or substituted or unsubstituted C₁₋₆alkoxy.
13. The compound of any one of clauses 1-12, wherein p is 0 or 1.
14. The compound of any one of clauses 1-13, wherein q is 1 or 2.
15. The compound of any one of clauses 1-14, wherein, R₃ is -NH₂, 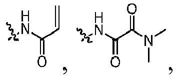 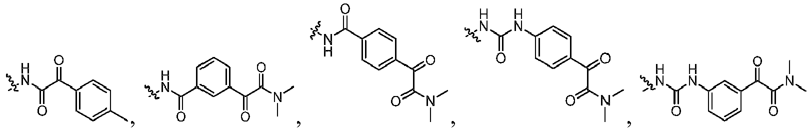 or 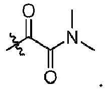
16. The compound of any one of clauses 1-15, wherein R₁ is combines with R₃, or R₂ is combines with R₃, to form a 5-10 member heteroaryl or 5-10 member heterocyclic ring, wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy, or C(O)R₈.
17. The compound of any one of clauses 1-16, wherein R₁ is combines with R₃, or R₂ is combines with R₃, to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring; wherein each of the ring systems contains 1, 2, 3 or 4 heteroatoms select from N, O or S, and is optionally substituted with -F, -Cl, -Br, -I, -CN, -OH, -NH₂, carbonyl, =O, oxo, substituted or unsubstituted C₁₋₃alkyl, substituted or unsubstituted C₁₋₃alkoxy or C(O)R₈.
18. The compound of any one of clauses 1-17, wherein R₁ is combines with R₃ to form a 5-10 member heteroaryl or a 5-10 member heterocyclic ring; wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy or C(O)R₈.
19. The compound of any one of clauses 1-18, wherein R₁ is combines with R₃ to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms select from N, O or S, and is optionally substituted with -F, -Cl, -Br, -I, -CN, -OH, -NH₂, carbonyl, =O, oxo, substituted or unsubstituted C₁₋₃alkyl, substituted or unsubstituted C₁₋₃alkoxy or C(O)R₈.
20. The compound of any one of clauses 1-19, wherein R₁ is combines with R₃ to form a 5-membered heteroaryl, 6-membered heteroaryl, 5-membered heterocyclic ring or 6-membered heterocyclic ring, wherein each of the ring systems contains 1 or 2 heteroatoms select from N or O, and is optionally substituted with -F, -Cl, -Br, -OH, -NH₂, -NHmethyl, -NHethyl, -NHpropyl, -NHisopropyl, -NHOCH₃, carbonyl, =O, oxo, methyl, ethyl, propyl, isopropyl, methoxy, CHF₂, CH₂F, CF₃ or C(O)R₈; and each methyl, ethyl, propyl, isopropyl, methoxy, CHF₂, CH₂F or C(O)R₈ is independently optionally substituted with -F, -Cl, -Br or -I.
21. The compound of any one of clauses 1-17, wherein R₂ is combines with R₃ to form a 5-10 member heteroaryl or 5-10 member heterocyclic ring, wherein each of the ring systems is optionally substituted with halogen, -CN, -OH, -NH₂, carbonyl, =O, oxo, substituted or unsubstituted C₁₋₆alkyl, substituted or unsubstituted C₁₋₆alkoxy or C(O)R₈.
22. The compound of any one of clauses 1-17 or 21, wherein R₂ is combines with R₃ to form a 5-membered heteroaryl, 6-membered heteroaryl, 7-membered heteroaryl, 8-membered heteroaryl, 5-membered heterocyclic ring, 6-membered heterocyclic ring, 7-membered heterocyclic ring or 8-membered heterocyclic ring, wherein each of the ring systems contains 1, 2, 3 or 4 heteroatoms select from N, O or S, and is optionally substituted with -F, -Cl, -Br, -I, -CN, -OH, -NH₂, carbonyl, =O, oxo, substituted or unsubstituted C₁₋₃alkyl, substituted or unsubstituted C₁₋₃alkoxy or C(O)R₈.
23. The compound of any one of clauses 1-22, wherein R₄ is -H, -F, -Cl, -Br, -CN, -OH, -NR₈R₉, substituted or unsubstituted C₁₋₆alkyl, substituted or unsubstituted C₁₋₆alkoxy, C₅₋₁₈heterocyclic or C₅₋₁₀carbocyclic, wherein each of the ring systems is optionally substituted with -F, -Cl, -Br, -CN, -OH, -NO₂, carbonyl, =O, oxo, substituted or unsubstituted C₁₋₆alkyl, substituted or unsubstituted C₁₋₆alkoxy, -NH₂, -NH-C₁₋₆alkyl, -NH-C₁₋₆alkoxy, -C₁₋₆alkylene-NH₂ or -C₁₋₆alkylene-NH-C₁₋₆alkyl.
24. The compound of any one of clauses 1-23, wherein R₄ is H, -F, -Cl, -CN, -OH, -NH₂, substituted or unsubstituted C₁₋₃alkyl, substituted or unsubstituted C₁₋₃alkoxy, 5-membered heterocyclic containing 1, 2 or 3 heteroatoms select from N or O, 6-membered heterocyclic containing 1, 2 or 3 heteroatoms select from N or O, 5-membered carbocyclic or 6-membered carbocyclic; wherein each of the ring systems is optionally substituted with -F, -Cl, -Br, -CN, -OH, carbonyl, =O, oxo, substituted or unsubstituted C₁₋₃alkyl, substituted or unsubstituted C₁₋₃alkoxy, -NH₂, -NH-C₁₋₃alkyl, -NH-C₁₋₃alkoxy, -C₁₋₃alkylene-NH₂ or -C₁₋₃alkylene-NH-C₁₋₃alkyl.
25. The compound of any one of clauses 1-24, wherein R₄ is -Cl, -NH₂, methyl or piperidinyl, wherein the ring system is optionally substituted with methyl, -NH₂ or -CH₂NH₂.
26. The compound of any one of clauses 1-25, wherein R₅ is -H, -F, -Cl, -Br, -I, -NR₈R₉, C₁₋₃alkyl, C₁₋₃alkoxy, C₆₋₉aryl, C₆₋₉arylalkyl, C₆₋₉heteroaryl, C₆₋₁₇heterocyclic or C₆₋₁₇carbocyclic, wherein each of which is independently optionally substituted with halogen, -CN, -OH, -N₃, -NO₂, -NH₂, carbonyl, =O, oxo, substituted or unsubstituted C₁₋₆alkyl, substituted or unsubstituted C₁₋₆alkoxy, substituted or unsubstituted (CH₂)ₖNR₈R₉, substituted or unsubstituted (CH₂)ₖNHC(O)OR₈ or C(O)R₈; and k is 0, 1 or 2.
27. The compound of clause 26, wherein the C₆₋₉ heteroaryl contains 1, 2, 3 or 4 heteroatoms select from N, O or S, and the C₆₋₉heteroaryl is 6-membered heteroaryl, 7-membered heteroaryl, 8-membered heteroaryl or 9-membered heteroaryl; the C₆₋₁₇heterocyclic contains 1, 2, 3, 4, 5, or 6 heteroatoms select from N, O, or S, and the C₆₋₁₇heterocyclic is 6-membered heterocyclic, 7-membered heterocyclic, 8-membered heterocyclic, 9-membered heterocyclic, 10-membered heterocyclic, 11-membered heterocyclic, 12-membered heterocyclic, 13-membered heterocyclic, 14-membered heterocyclic, 15-membered heterocyclic, 16-membered heterocyclic or 17-membered heterocyclic.
28. The compound of any one of clauses 1-27, wherein R₅ is -F, -Cl, -Br, -NR₈R₉, methyl, ethyl, propyl, isopropyl, methoxy, ethoxy, propoxy, isopropoxy, 6-membered aryl, 7-membered aryl, 8-membered aryl, 9-membered aryl, 6-membered arylalkyl, 7-membered arylalkyl, 8-membered arylalkyl, 9-membered arylalkyl, 6-membered heteroaryl, 7-membered heteroaryl, 8-membered heteroaryl, 9-membered heteroaryl, 6-membered heterocyclic, 7-membered heterocyclic, 8-membered heterocyclic, 9-membered heterocyclic, 10-membered heterocyclic, 11-membered heterocyclic, 12-membered heterocyclic, 13-membered heterocyclic, 14-membered heterocyclic, 15-membered heterocyclic, 16-membered heterocyclic, 6-membered carbocyclic, 7-membered carbocyclic, 8-membered carbocyclic, 9-membered carbocyclic, 10-membered carbocyclic, 11-membered carbocyclic, 12-membered carbocyclic, 13-membered carbocyclic, 14-membered carbocyclic, 15-membered carbocyclic or 16-membered carbocyclic; and each heteroaryl contains 1, 2 or 3 heteroatoms select from N, O or S, and each heterocyclic contains 1, 2, 3, or 4 heteroatoms select from N, O or S; wherein each of which is independently optionally substituted with -F, -Cl, -Br, -I, -CN, -OH, -NO₂, -NH₂, carbonyl, =O, oxo, substituted or unsubstituted C₁₋₃alkyl, substituted or unsubstituted C₁₋₃alkoxy, substituted or unsubstituted (CH₂)ₖNR₈R₉, substituted or unsubstituted (CH₂)ₖNHC(O)OR₈, or substituted or unsubstituted C(O)R₈; and k is 0, 1 or 2.
29. The compound of any one of clauses 1-28, wherein:
   R₅ is 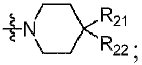
   each R₂₁ and R₂₂ is independently halogen, C₁₋₃alkyl, -NH₂, -C₁₋₃alkylene-NH₂, -C₁₋₃alkylene-NH-C₁₋₃alkyl, -C₁₋₃alkylene-N(C₁₋₃alkyl)₂, -NHBoc or -CH₂NHBoc;
   or R₂₁ and R₂₂ together with the carbon atom to which they are both attached form a 5-10 member heteroaryl, 5-10 member carbocyclic or a 5-10 member heterocyclic ring, wherein each of the ring system is optionally substituted with halogen, -CN, -OH, carbonyl, =O, oxo, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH₂, C₁₋₃alkoxy or C₁₋₃alkyl.
30. The compound of clause 29, wherein, R₂₁ and R₂₂ together with the carbon atom to which they are both attached form a 5-membered heteroaryl, 6-membered heteroaryl, 7-membered heteroaryl, 8-membered heteroaryl, 9-membered heteroaryl, 10-membered heteroaryl, 5-membered heterocyclic ring, 6-membered heterocyclic ring, 7-membered heterocyclic ring, 8-membered heterocyclic ring, 9-membered heterocyclic ring or 10-membered heterocyclic ring; wherein each of the ring system contains 1, 2 or 3 heteroatoms select from N, O or S, and is independently optionally substituted with halogen, -CN, -OH, carbonyl, =O, oxo, -NH₂, C₁₋₃alkoxy or C₁₋₃alkyl.
31. The compound of any one of clauses 1-30, wherein X is O, S or absent.
32. The compound of any one of clauses 1-31, wherein Y₁ is N and Y₂ is CR₂.
33. The compound of any one of clauses 1-32, wherein Y₂ is N and Y₁ is CR₁.
34. The compound of any one of clauses 1-33, wherein R₄ is -NH₂.
35. The compound of any one of clauses 1-34, wherein R₅ is -NH₂, 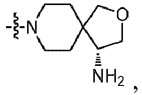 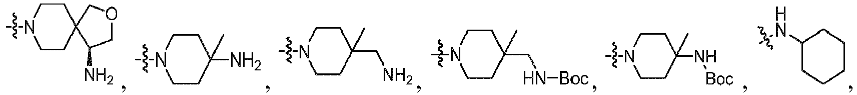 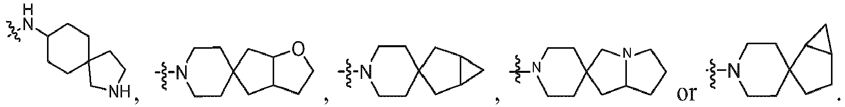
36. The compound of any one of clauses 1-35, wherein each R₈ and R₉ is independently -H, halogen, CN, -OH, -NO₂, -NH₂, -C₁₋₆alkylene-NH₂, -C₁₋₆alkylene-NH-C₁₋₆alkyl, -C₁₋₆alkylene-N(C₁₋₆alkyl)₂, -NHBoc, -CH₂NHBoc; -NH-C₁₋₆alkyl, -N(C₁₋₆alkyl)₂, -NH-C₁₋₆alkoxy, -N(C₁₋₆alkoxy)₂, C₁₋₆alkyl, C₁₋₆alkoxy, C₂₋₆alkenyl, C₂₋₆alkylnyl, C₅₋₁₀heterocyclic or C₅₋₁₀carbocyclic; each of which may be optionally substituted.
37. The compound of any one of clauses 1-36, wherein each R₈ and R₉ is independently -H, -F, -Cl, -CN, -OH, -NO₂, -NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, C₁₋₄alkyl, C₁₋₃alkoxy, C₂₋₃alkenyl, C₂₋₃alkylnyl, 5-membered heterocyclic, 6-membered heterocyclic, 7-membered heterocyclic, 8-membered heterocyclic, 9-membered heterocyclic, 10-membered heterocyclic, 5-membered carbocyclic, 6-membered carbocyclic, 7-membered carbocyclic, 8-membered carbocyclic, 9-membered carbocyclic, 10-membered carbocyclic, 5-membered carbocyclic, 6-membered carbocyclic, 7-membered carbocyclic, 8-membered carbocyclic, 9-membered carbocyclic, or 10-membered carbocyclic; and each of which may be independently optionally substituted with halogen, -CN, -OH, -NH₂, -N₃, -NO₂, -C₁₋₆alkylene-NH₂, -C₁₋₆alkylene-NH-C₁₋₆alkyl, -C₁₋₆alkylene-N(C₁₋₆alkyl)₂, -NHBoc, -CH₂NHBoc; -NH-C₁₋₆alkyl, -N(C₁₋₆alkyl)₂, -NH-C₁₋₆alkoxy, -N(C₁₋₆alkoxy)₂, substituted or unsubstituted C₁₋₆alkyl, or substituted or unsubstituted C₁₋₆alkoxy; and each heterocyclic contains 1, 2, 3 or 4 heteroatoms select from N, O or S.
38. The compound of any one of clauses 1-37, wherein each R₈ and R₉ is independently -H, methyl, tert-butyl, -CH=CH₂, N(CH₃)₂, 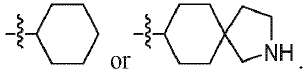
39. The compound of any one of clauses 1-38, wherein the compound is of Formula II: 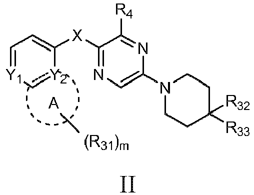 wherein,
   X is absent or S;
   Y₁ is N or CR₂₅;
   Y₂ is N or C;
   R₂₅ is H, halogen, C₁₋₃alkyl, C₁₋₃alkoxy, C₂₋₃alkenyl or C₂₋₃alkylnyl;
   ring 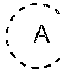 is 5-8 member heteroaryl containing 1,2,3 or 4 heteroatoms select form N, O or S, 5-8 member carbocyclic or 5-8 member heterocyclic ring containing 1,2,3 or 4 heteroatoms select form N, O or S;
   R₃₁ is -H, halogen, -OH, -NH₂, -(C=O)C₁₋₃alkyl, -CN, -NO₂, carbonyl, =O, oxo, carboxyl, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy;
   m is 0, 1, 2, 3 or 4;
   R₄ is -H, halogen, -NH₂, substituted or unsubstituted C₁₋₃alkoxy, or substituted or unsubstituted C₁₋₃alkyl;
   each R₃₂ and R₃₃ is independently -H, halogen, -OH, -NH₂, -CN, -NO₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy;
   or R₃₂ and R₃₃ together with the carbon atom to which they are both attached form a 5-8 member heteroaryl containing 1, 2 or 3 heteroatoms select from N, O or S, or 5-8 member heterocyclic ring containing 1, 2 or 3 heteroatoms select from N, O or S, wherein each of the ring systems is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy.
40. The compound of clause 39, wherein ring 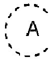 is 5-membered heteroaryl, 6-membered heteroaryl, 7-membered heteroaryl, 5-membered heterocyclic ring, 6-membered heterocyclic ring, 7-membered heterocyclic ring, 5-membered carbocyclic, 6-membered carbocyclic, 7-membered carbocyclic or 8-membered carbocyclic; and each the ring systems contains 1, 2 or 3 heteroatoms select form N, O or S.
41. The compound of clause 40 or 41, wherein ring 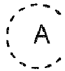 is 5-membered heterocyclic ring containing 1, 2 or 3 heteroatoms select form N or O, 6-membered heterocyclic ring containing lor 2 heteroatoms select form N or O or 5-membered carbocyclic.
42. The compound of any one of clauses 39-41, wherein R₃₁ is -H, -F, -Cl, -Br, -I, -OH, -NH₂, carbonyl, =O, oxo, -(CO)C₁₋₃alkyl, -C₁₋₃alkylene-NH₂, -NHBoc, -CH₂NHBoc, -NH-C₁₋₃alkyl, -N(C₁₋₃alkyl)₂, substituted or unsubstituted C₁₋₃alkyl, or substituted or unsubstituted C₁₋₃alkoxy.
43. The compound of any one of clauses 39-42, wherein R₃₁ is -F, -COCH₃, carbonyl, =O, oxo, -CH₃ or -CF₃.
44. The compound of clauses 39-43, wherein R₃₂ and R₃₃ together with the carbon atom to which they are both attached to form a 5-membered heteroaryl, 6-membered heteroaryl, 7-membered heteroaryl, 5-membered heterocyclic ring, 6-membered heterocyclic ring or 7-membered heterocyclic ring; wherein each of the ring systems contains 1, 2 or 3 heteroatoms select from N, O or S, and is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NH-C₁₋₃alkoxy, -N(C₁₋₃alkoxy)₂, substituted or unsubstituted C₁₋₃alkyl, or substituted or unsubstituted C₁₋₃alkoxy.
45. The compound of any one of clauses 39-44, wherein R₃₂ and R₃₃ together with the carbon atom to which they are both attached to form a 5-membered heterocyclic ring, 6-membered heterocyclic ring or 7-membered heterocyclic ring; wherein each of the ring system containing 1 or 2 heteroatoms independently select from O or N, and is optionally substituted with halogen, -CN, -OH, -NH₂, carbonyl, =O, oxo, carboxyl, -N₃, -NO₂, C₁₋₃alkyl or C₁₋₃alkoxy.
46. The compound of any one of clauses 39-45, wherein R₃₂ and R₃₃ together with the carbon atom to which they are both attached to form a 5-membered heterocyclic ring; and the heterocyclic ring contains 1 heteroatoms selected from O or N, and is optionally substituted with -F, -Cl, -OH, -NH₂, carbonyl, =O, oxo, methyl or methoxy.
47. The compound of any one of clauses 39-43, wherein each R₃₂ and R₃₃ is independently -CH₂NH₂, -CH₂NHBoc or methyl.
48. The compound of any one of clauses 39-47, wherein Y₁ is N.
49. The compound of any one of clauses 39-48, wherein Y₂ is C.
50. The compound of any one of clauses 39-49, wherein R₂₅ is -H or -Cl.
51. The compound of any one of clauses 39-50, wherein R₄ is -NH₂.
52. The compound of any one of clauses 1-38, wherein the compound is of Formula III: 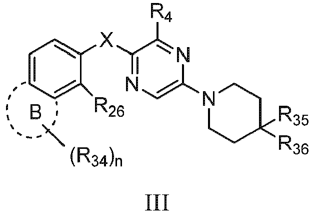 wherein,
   X is absent or S;
   R₂₆ is -H, halogen, substituted or unsubstituted C₁₋₃alkyl, or substituted or unsubstituted C₁₋₃alkoxy;
   ring 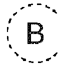 is 5-8 member heteroaryl or 5-8 member heterocyclic ring; and each the ring system independently contains 1, 2, 3 or 4 heteroatoms select form N, O or S;
   R₃₄ is -H, halogen, -OH, -NR₃₅R₃₆, -CN, -NO₂, carbonyl, =O, oxo, substituted or unsubstituted C₁₋₃alkyl, or substituted or unsubstituted C₁₋₃alkoxy;
   n is 0, 1, 2 or 3;
   R₄ is -H, halogen, -NH₂, substituted or unsubstituted C₁₋₆alkoxy, or substituted or unsubstituted C₁₋₆ alkyl;
   each R₃₅ and R₃₆ is independently -H, halogen, -OH, -NH₂, -CN, -NO₂,-CH₂NH₂, substituted or unsubstituted C₁₋₃alkyl, or substituted or unsubstituted C₁₋₃alkoxy;
   or R₃₅ and R₃₆ together with the carbon atom to which they are both attached to form a 5-8 member heteroaryl or 5-8 member heterocyclic ring, wherein each of the ring system independently contains 1, 2 or 3 heteroatoms select from N, O or S, and is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NHC₁₋₃alkoxy, -N(C₁₋₃alkoxy)₂, substituted or unsubstituted C₁₋₃alkyl, or substituted or unsubstituted C₁₋₃alkoxy.
53. The compound of clause 52, wherein ring 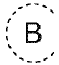 is 5-membered heteroaryl, 6-membered heteroaryl, 7-membered heteroaryl, 5-membered heterocyclic ring, 6-membered heterocyclic ring or 7-membered heterocyclic ring; and each of the ring system independently contains 1, 2 or 3 heteroatoms select form N, O or S.
54. The compound of clause 52 or 53, wherein ring 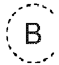 is 5-membered heterocyclic ring or 6-membered heterocyclic ring; and each of the ring system independently contains 1 or 2 heteroatoms select form N or O.
55. The compound of any one of clauses 52-54, wherein R₃₅ and R₃₆ together with the carbon atom to which they are both attached to form a 5-membered heteroaryl, 6-membered heteroaryl, 7-membered heteroaryl, 5-membered heterocyclic ring, 6-membered heterocyclic ring or 7-membered heterocyclic ring; wherein each of the ring system is independently contains 1, 2 or 3 heteroatoms select from N, O or S, and is optionally substituted with halogen, -CN, -OH, -NH₂, carbonyl, =O, oxo, -CH₂NH₂, -C₁₋₃alkylene-NH₂, -C₁₋₃alkylene-NH-C₁₋₃alkyl, -C₁₋₃alkylene-N(C₁₋₃alkyl)₂, -NHBoc, -CH₂NHBoc; -NH-C₁₋₃alkyl, -N(C₁₋₃alkyl)₂, -NHC₁₋₃alkoxy, -N(C₁₋₃alkoxy)₂, substituted or unsubstituted C₁₋₃alkyl, or substituted or unsubstituted C₁₋₃alkoxy.
56. The compound of any one of clauses 52-55, wherein R₃₅ and R₃₆ together with the carbon atom to which they are both attached to form a 5-membered heterocyclic ring, 6-membered heterocyclic ring or 7-membered heterocyclic ring; wherein each of the ring system is independently contains 1 or 2 heteroatoms independently select from O or N, and is optionally substituted with halogen, -CN, -OH, -NH₂, carbonyl, =O, oxo, carboxyl, -N₃, -NO₂,C₁₋₃alkyl or C₁₋₃alkoxy.
57. The compound of any one of clauses 52-56, wherein R₃₅ and R₃₆ together with the carbon atom to which they are both attached to form a 5-membered heterocyclic ring; wherein the ring system contains 1 heteroatom independently select from O or N, and is optionally substituted with F, -Cl, -OH, -NH₂, carbonyl, =O, oxo, methyl or methoxy.
58. The compound of any one of clauses 52-57, wherein R₂₆ is -H or -Cl.
59. The compound of any one of clauses 52-58, wherein R₄ is -NH₂.
60. The compound of any one of clauses 52-59, wherein R₃₄ is -F, -COCH₃, carbonyl, =O, oxo, -CH₃ or -CF₃.
61. The compound of any one of clauses 1-60, wherein each substituted or unsubstituted C₁₋₆alkyl is independently C₁₋₆alkyl, or C₁₋₆alkyl substituted with halogen, -OH, -CN, NH₂, -NO₂,carbonyl, =O, oxo, -C₁₋₆alkylene-NH₂, -C₁₋₆alkylene-NH-C₁₋₆alkyl. or -C₁₋₆alkylene-N(C₁₋₆alkyl)₂; each substituted or unsubstituted C₁₋₆alkoxy is independently C₁₋₆alkoxy, or C₁₋₆alkoxy substituted with halogen, -OH, -CN, NH₂, -NO₂, carbonyl, =O, oxo, -C₁₋₆alkylene-NH₂, -C₁₋₆alkylene-NH-C₁₋₃alkyl or -C₁₋₆alkylene-N(C₁₋₆alkyl)₂.
62. The compound of any one of clauses 1-61, wherein each substituted or unsubstituted C₁₋₃alkyl is independently C₁₋₃alkyl, or C₁₋₃alkyl substituted with halogen, -OH, -CN, NH₂, -NO₂, carbonyl, =O, oxo, -C₁₋₃alkylene-NH₂, -C₁₋₃alkylene-NH-C₁₋₃alkyl, or -C₁₋₃alkylene-N(C₁₋₃alkyl)₂; each substituted or unsubstituted C₁₋₃alkoxy is independently C₁₋₃alkoxy, or C₁₋₃alkoxy substituted with halogen, -OH, -CN, NH₂, -NO₂, carbonyl, =O, oxo, -C₁₋₃alkylene-NH₂, -C₁₋₃alkylene-NH-C₁₋₃alkyl, or -C₁₋₃alkylene-N(C₁₋₃alkyl)₂-
63. The compound of any one of clauses 1-62, wherein each C₁₋₆alkyl is independently methyl, ethyl, propyl, isopropyl, cyclopropyl, n-butyl, isobutyl, tert-buyl, n-pentyl, neopentyl, isopentyl, cyclopentyl, n-hexyl or cyclohexyl.
64. The compound of any one of clauses 1-63, wherein each C₁₋₃alkyl is independently methyl, ethyl, propyl, isopropyl or cyclopropyl.
65. The compound of any one of clauses 1-64, wherein each C₁₋₃alkoxy is independently methoxy, ethoxy, propoxy, isopropoxy or cyclopropyloxy.
66. The compound of any one of clauses 1-65, wherein each C₂₋₃alkenyl is independently -CH=CH₂, -CH₂-CH=CH₂ or -CH=CH-CH₃.
67. The compound of any one of clauses 1-66, wherein each C₂₋₃alkylnyl is independently -C≡CH, -CH₂-C≡CH or -C≡C-CH₃.
68. The compound of any one of clauses 1-67, wherein each halogen is independently -F, -Cl, -Br or -I.
69. The compound of any one of clauses 1-68, wherein each heterocyclic group and each carbocyclic group includes single ring, spiral ring, bridge ring, fused ring and all various combinations of spiral ring, bridge ring, and/or fused ring.
70. The compound of clause 69, the said single ring includes the said spiral ring includes 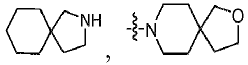 ; and the said various combinations of spiral ring, bridge ring, and/or fused ring include 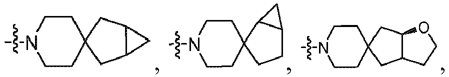 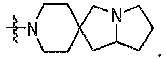
71. The compound of clause 1, wherein the compound is
   1) (S)-N1-(3-((3-amino-5-(4-amino-2-oxa-8-azaspiro[4.5]decan-8-yl)pyrazin-2-yl)thio)-2-chlorophenyl)-N2,N2-dimethyloxalamide hydrochloride;
   2) N¹-(4-((3-amino-5-(4-amino-4-methylpiperidin-1-yl)pyrazin-2-yl)thio)-3-chloropyrid in-2-yl)-N²,N²-dimethyloxalamide;
   3) N-(3-((3-amino-5-(4-amino-4-methylpiperidin-1-yl)pyrazin-2-yl)thio)-2-chloropheny 1)-4-(2-(dimethylamino)-2-oxoacetyl)benzamide;
   4) 6-((3-amino-5-(4-(aminomethyl)-4-methylpiperidin-1-yl)pyrazin-2-yl)thio)-2H-benz o[b][1,4]oxazin-3(4H)-one;
   5) 6-(3 -amino-5-(4-(aminomethyl)-4-methylpiperidin-1-yl)pyrazin-2-yl)-2H-benzo[b] [1 ,4] oxazin-3 (4H)-one;
   6) 5-((2-amino-3-chloropyridin-4-yl)thio)-N2-(2-azaspiro[4.5]decan-8-yl)pyrazine-2,6-diamine;
   7) (S)-1-(4-((3-amino-5-(4-amino-2-oxa-8-azaspiro[4.5]decan-8-yl)pyrazin-2-yl)thio)-3 ,3-difluoroindolin-1-yl)ethanone;
   8) (S)-4-((3-amino-5-(4-amino-2-oxa-8-azaspiro[4.5]decan-8-yl)pyrazin-2-yl)thio)-3,3-difluoroindolin-2-one;
   9) 4-((3-amino-5-(4-(aminomethyl)-4-methylpiperidin-1-yl)pyrazin-2-yl)thio)indolin-2-one;
   10) (S)-4-((3-amino-5-(4-amino-2-oxa-8-azaspiro[4.5]decan-8-yl)pyrazin-2-yl)thio)-3,3-difluoro-1H-pyrrolo[2,3-b]pyridin-2(3H)-one;
   11) (R)-N-((S)-8-(6-amino-5-((3,3-difluoro-1-methyl-2-oxoindolin-4-yl)thio)pyrazin-2-y 1)-2-oxa-8-azaspiro[4.5]decan-4-yl)-2-methylpropane-2-sulfinamide;
   12) (S)-4-((3-amino-5-(4-amino-2-oxa-8-azaspiro[4.5]decan-8-yl)pyrazin-2-yl)thio)indol ine-2,3-dione hydrochloride;
   13) tert-butyl ((1-(5-((1-acetyl-3,3-difluoroindolin-4-yl)thio)-6-aminopyrazin-2-yl)-4-methylpiperidin-4-yl)methyl)carbamate;
   14) 4-((3-amino-5-(4-(aminomethyl)-4-methylpiperidin-1-yl)pyrazin-2-yl)thio)indoline-2 ,3-dione;
   15) 5-((2-amino-3-chloropyridin-4-yl)thio)-6-(4-(aminomethyl)-4-methylpiperidin-1-yl)p yrazin-2-amine;
   16) N¹-(4-((3 -amino-5 -(4-(aminomethyl)-4-methylpiperidin-1-yl)pyrazin-2-yl)thio)-3 -chl oropyridin-2-yl)-N2,N2-dimethyloxalamide ;
   17) tert-butyl ((1-(6-amino-5-((2-amino-3-chloropyridin-4-yl)thio)pyrazin-2-yl)-4-methylpiperidin-4-yl)methyl)carbamate;
   18) (S)-N1-(4-((3-amino-5-(4-amino-2-oxa-8-azaspiro[4.5]decan-8-yl)pyrazin-2-yl)thio)-3 -chloropyridin-2-yl)-N2,N2-dimethyloxalamide;
   19) (S)-N¹-(3-((3-amino-5-(4-amino-2-oxa-8-azaspiro[4.5]decan-8-yl)pyrazin-2-yl)thio)-2-chlorophenyl)-N²,N²-dimethyloxalamide;
   20) N¹-(3-((3-amino-5-(4-(aminomethyl)-4-methylpiperidin-1-yl)pyrazin-2-yl)thio)-2-chl orophenyl)-N²,N²-dimethyloxalamide;
   21) N-(3-((3-amino-5-(4-(aminomethyl)-4-methylpiperidin-1-yl)pyrazin-2-yl)thio)-2-chl orophenyl)-3-(2-(dimethylamino)-2-oxoacetyl)benzamide;
   22) 2-(3-(3-(3-((3-amino-5-(4-(aminomethyl)-4-methylpiperidin-1-yl)pyrazin-2-yl)thio)-2-chlorophenyl)ureido)phenyl)-N,N-dimethyl-2-oxoacetamide;
   23) 2-(4-(3-(3-((3-amino-5-(4-(aminomethyl)-4-methylpiperidin-1-yl)pyrazin-2-yl)thio)-2-chlorophenyl)ureido)phenyl)-N,N-dimethyl-2-oxoacetamide;
   24) 6-(4-(aminomethyl)-4-methylpiperidin-1-yl)-3-((3,4-dihydro-2H-benzo[b][1,4]oxazi n-6-yl)thio)pyrazin-2-amine;
   25) tert-butyl (1-(6-amino-5-((2-amino-3-chloropyridin-4-yl)thio)pyrazin-2-yl)-4-methylpiperidin-4-yl)carbamate;
   26) tert-butyl (1-(5-((2-acrylamido-3-chloropyridin-4-yl)thio)-6-aminopyrazin-2-yl)-4-methylpiperidin-4-yl)carbamate;
   27) tert-butyl (1-(6-amino-5-((3-chloro-2-(2-(dimethylamino)-2-oxoacetamido)pyridin-4 -yl)thio)pyrazin-2-yl)-4-methylpiperidin-4-yl)carbamate;
   28) tert-butyl (1-(6-amino-5-((2-chloro-3-(2-(dimethylamino)-2-oxoacetamido)phenyl) thio)pyrazin-2-yl)-4-methylpiperidin-4-yl)carbamate;
   29) N¹-(3-((3-amino-5-(4-amino-4-methylpiperidin-1-yl)pyrazin-2-yl)thio)-2-chlorophen yl)-N²,N²-dimethyloxalamide;
   30) tert-butyl (1-(6-amino-5-((3-amino-2-chlorophenyl)thio)pyrazin-2-yl)-4-methylpiperidin-4-yl)carbamate;
   31) N-(3-((3-amino-5-(4-amino-4-methylpiperidin-1-yl)pyrazin-2-yl)thio)-2-chloropheny l)-2-oxo-2-(p-tolyl)acetamide;
   32) tert-butyl (1-(5-((3-acrylamido-2-chlorophenyl)thio)-6-aminopyrazin-2-yl)-4-methylpiperidin-4-yl)carbamate;
   33) 6-(3-amino-5-(4-amino-4-methylpiperidin-1-yl)pyrazin-2-yl)-2H-benzo[b][1,4]oxazi n-3(4H)-one;
   34) N-(4-((3-amino-5-(4-amino-4-methylpiperidin-1-yl)pyrazin-2-yl)thio)-3-chloropyridi n-2-yl)-4-(2-(dimethylamino)-2-oxoacetyl)benzamide;
   35) N-(4-((3-amino-5-(4-(aminomethyl)-4-methylpiperidin-1-yl)pyrazin-2-yl)thio)-3-chl oropyridin-2-yl)-4-(2-(dimethylamino)-2-oxoacetyl)benzamide;
   36) N-(3-((3-amino-5-(4-(aminomethyl)-4-methylpiperidin-1-yl)pyrazin-2-yl)thio)-2-chl orophenyl)-4-(2-(dimethylamino)-2-oxoacetyl)benzamide;
   37) 5-((2-amino-3-chloropyridin-4-yl)thio)-N2-cyclohexylpyrazine-2,6-diamine;-3062-B
   38) (S)-8-(5-((1H-pyrrolo[2,3-b]pyridin-4-yl)thio)-6-aminopyrazin-2-yl)-2-oxa-8-azaspir o[4.5]decan-4-amine;
   39) (S)-8-(6-amino-5-((3,3-dimethylindolin-4-yl)thio)pyrazin-2-yl)-2-oxa-8-azaspiro[4.5 ]decan-4-amine;
   40) (S)-8-(6-amino-5-((3-fluoro-1H-indol-4-yl)thio)pyrazin-2-yl)-2-oxa-8-azaspiro[4.5]d ecan-4-amine;
   41) 5-((2-amino-3-chloropyridin-4-yl)thio)-N2-(4-(aminomethyl)-4-methylcyclohexyl)p yrazine-2,6-diamine;
   42) (S)-8-(5-((1H-indol-4-yl)thio)-6-aminopyrazin-2-yl)-2-oxa-8-azaspiro[4.5]decan-4-a mine;
   43) (S)-1-(4-((3-amino-5-(4-amino-2-oxa-8-azaspiro[4.5]decan-8-yl)pyrazin-2-yl)thio)-1 H-indol-1-yl)ethanone;
   44) 5-((2-amino-3-chloropyridin-4-yl)thio)-N2-(4-amino-4-methylcyclohexyl)pyrazine-2 ,6-diamine;
   45) (S)-6-((3-amino-5-(4-amino-2-oxa-8-azaspiro[4.5]decan-8-yl)pyrazin-2-yl)thio)-2H-benzo[b][1,4]oxazin-3 (4H)-one;
   46) (S)-4-((3-amino-5-(4-amino-2-oxa-8-azaspiro[4.5]decan-8-yl)pyrazin-2-yl)thio)-1,3, 3-trimethylindolin-2-one;
   47) (4S)-8-(6-amino-5-((3-fluoroindolin-4-yl)thio)pyrazin-2-yl)-2-oxa-8-azaspiro[4.5]de can-4-amine;
   48) 1-(4-((3-amino-5-((S)-4-amino-2-oxa-8-azaspiro[4.5]decan-8-yl)pyrazin-2-yl)thio)-3 -fluoro-3-methylindolin-1-yl)ethanone;
   49) (S)-8-(6-amino-5-((3,3-difluoroindolin-4-yl)thio)pyrazin-2-yl)-2-oxa-8-azaspiro[4.5] decan-4-amine;
   50) 1-(4-((3-amino-5-((S)-4-amino-2-oxaspiro[4.5]decan-8-yl)pyrazin-2-yl)thio)-3-meth ylindolin-1-yl)ethanone;
   51) (S)-8-(6-amino-5-((8-chloro-4,4-difluoro-1,2,3,4-tetrahydroquinolin-5-yl)thio)pyrazi n-2-yl)-2-oxa-8-azaspiro[4.5]decan-4-amine;
   52) (4S)-8-(6-amino-5-((8-chloro-4-fluoro-1,2,3,4-tetrahydroquinolin-5-yl)thio)pyrazin-2 -yl)-2-oxa-8-azaspiro[4.5]decan-4-amine;
   53) (S)-8-(6-amino-5-((3,3-difluoro-1-methylindolin-4-yl)thio)pyrazin-2-yl)-2-oxa-8-aza spiro[4.5]decan-4-amine;
   54) (S)-6-((3-amino-5-(4-amino-2-oxa-8-azaspiro[4.5]decan-8-yl)pyrazin-2-yl)thio)-3,3-difluoroindolin-2-one;
   55) 4-((3-amino-5-((S)-4-amino-2-oxa-8-azaspiro[4.5]decan-8-yl)pyrazin-2-yl)thio)-3-fl uoroindolin-2-one;
   56) (S)-8-(6-amino-5-((3,3-difluoro-2,3-dihydrobenzofuran-4-yl)thio)pyrazin-2-yl)-2-ox a-8-azaspiro[4.5]decan-4-amine;
   57) (S)-8-(6-amino-5-((4,4-difluorochroman-5-yl)thio)pyrazin-2-yl)-2-oxa-8-azaspiro[4. 5]decan-4-amine;
   58) 4-((3-amino-5-((S)-4-amino-2-oxa-8-azaspiro[4.5]decan-8-yl)pyrazin-2-yl)thio)-3-fl uoro-1-methyl-3-(trifluoromethyl)indolin-2-one;
   59) (S)-6-((3-amino-5-(4-amino-2-oxa-8-azaspiro[4.5]decan-8-yl)pyrazin-2-yl)thio)-7-ch loroindolin-2-one;
   60) (S)-8-(6-amino-5-((5-chloro-3,4-dihydro-2H-benzo[b][1,4]oxazin-6-yl)thio)pyrazin-2-yl)-2-oxa-8-azaspiro[4.5]decan-4-amine;
   61) (S)-7-((3-amino-5-(4-amino-2-oxa-8-azaspiro[4.5]decan-8-yl)pyrazin-2-yl)thio)-8-ch loro-3,4-dihydroquinolin-2(1H)-one;
   62) (S)-6-((3-amino-5-(4-amino-2-oxa-8-azaspiro[4.5]decan-8-yl)pyrazin-2-yl)thio)-5-ch loro-2H-benzo[b][1,4] oxazin-3 (4H)-one;
   63) (S)-2-(3-((3-amino-5-(4-amino-2-oxa-8-azaspiro[4.5]decan-8-yl)pyrazin-2-yl)thio)-2 -chlorophenyl)-N,N-dimethyl-2-oxoacetamide;
   64) 4-((3-amino-5-(4-(aminomethyl)-4-methylpiperidin-1-yl)pyrazin-2-yl)thio)-3,3-diflu oro-1H-pyrrolo[2,3-b]pyridin-2(3H)-one;
   65) (S)-4-((3-amino-5-(4-amino-2-oxa-8-azaspiro[4.5]decan-8-yl)pyrazin-2-yl)thio)-1H-benzo[d]imidazol-2(3H)-one;
   66) (S)-4-((3-amino-5-(4-amino-2-oxa-8-azaspiro[4.5]decan-8-yl)pyrazin-2-yl)thio)-1,3-dimethyl-1H-benzo[d]imidazol-2(3H)-one;
   67) (S)-8-(6-amino-5-((2,2-difluoro-2,3-dihydro-1H-benzo[d]imidazol-4-yl)thio)pyrazin-2-yl)-2-oxa-8-azaspiro[4.5]decan-4-amine;
   68) (S)-8-(6-amino-5-((2,2-difluoro-1,3-dimethyl-2,3-dihydro-1H-benzo[d]imidazol-4-yl )thio)pyrazin-2-yl)-2-oxa-8-azaspiro[4.5]decan-4-amine;
   69) (4S)-8-(6-amino-5-((1-amino-3,3-difluoro-2,3-dihydro-1H-inden-4-yl)thio)pyrazin-2 -yl)-2-oxa-8-azaspiro[4.5]decan-4-amine;
   70) (S)-5-((3-amino-5-(4-amino-2-oxa-8-azaspiro[4.5]decan-8-yl)pyrazin-2-yl)thio)-2-m ethyl-[1,2,4]triazolo[4,3-a]pyridin-3(2H)-one;
   71) (S)-8-(6-amino-5-((3,3-difluoro-2-methyl-2,3-dihydro-[1,2,4]triazolo[4,3-a]pyridin-5 -yl)thio)pyrazin-2-yl)-2-oxa-8-azaspiro[4.5]decan-4-amine;
   72) (S)-1-(4-((3-amino-5-(4-amino-2-oxa-8-azaspiro[4.5]decan-8-yl)pyrazin-2-yl)thio)in dolin-1-yl)ethanone;
   73) 1'-(6-amino-5-((2-amino-3-chloropyridin-4-yl)thio)pyrazin-2-yl)hexahydrospiro[cycl openta[b]furan-5,4'-piperidin]-4-amine;
   74) 1'-(6-amino-5-((2-amino-3-chloropyridin-4-yl)thio)pyrazin-2-yl)spiro[bicyclo[3.1.0] hexane-3,4'-piperidin]-2-amine;
   75) 1'-amino-1-(6-amino-5-((2-amino-3-chloropyridin-4-yl)thio)pyrazin-2-yl)tetrahydros piro[piperidine-4,2'-pyrrolizin]-3'(1'H)-one;
   76) 1'-(6-amino-5-((2-amino-3-chloropyridin-4-yl)thio)pyrazin-2-yl)spiro[bicyclo[3.1.0] hexane-2,4'-piperidin]-3-amine.
72. A pharmaceutical composition comprising at least one compound as defined in any one of clauses 1-71 and at least one pharmaceutically acceptable excipient.
73. The pharmaceutical composition according to clause 72, wherein, the said compound in a weight ratio to the said excipient within the range from about 0.0001 to about 10.
74. Use of a pharmaceutical composition of as defined in clause 72 or 73 for the preparation of a medicament.
75. The use according to clause 74 for the preparation of a medicament for the treatment or prevention of cancer, cancer metastasis, cardiovascular disease, an immunological disorder or an ocular disorder.
76. Use of at least one compound of any one of clauses 1-71 is to prepare of a medicament.
77. The use according to clause 76 for the preparation of a medicament for the treatment or prevention of cancer, cancer metastasis, cardiovascular disease, an immunological disorder or an ocular disorder.
78. At least one compound for use as defined in any one of clauses 1-71, which is for use in the treatment of cancer, the prevention of cancer metastasis or the treatment of cardiovascular disease, an immunological disorder or an ocular disorder.
79. Use, in the manufacture of a medicament for use as an inhibitor of SHP2, of at least one compound as defined in any one of clauses 1-71.
80. A method of treating a patient having a condition which is mediated by the activity of SHP2, said method comprising administering to the patient a therapeutically effective amount of at least one compound as defined in any one of clauses 1-71, or a pharmaceutically acceptable salt thereof.
81. The method according tc clause 80 wherein the condition mediated by the activity of SHP2 is cancer.
82. The method according to clause 80, wherein the condition mediated by the activity of SHP2 is noonan syndrome, leopard syndrome, juvenile myelomonocytic leukemias, liver cancer, neuroblastoma, melanoma, squamous-cell carcinoma of the head and neck, acute myeloid leukemia, breast cancer, esophageal cancer, lung cancer, colon cancer, head cancer, gastric carcinoma,neuroblastoma, anaplastic large-cell lymphoma and glioblastoma.
83. At least one compound as defined in any one of clauses 1-71 or a pharmaceutically acceptable salt thereof for use as a medicament.
84. At least one compound according to any one of clauses 1-71 or a pharmaceutically acceptable salt thereof for use in the treatment of cancer.
85. A method of treating cancer selected from the group consisting of noonan syndrome, leopard syndrome, juvenile myelomonocytic leukemias, liver cancer, neuroblastoma, melanoma, squamous-cell carcinoma of the head and neck, acute myeloid leukemia, breast cancer, esophageal cancer, lung cancer, colon cancer, head cancer, gastric carcinoma,neuroblastoma, anaplastic large-cell lymphoma and glioblastoma in a mammal comprising administering to a mammal in need of such treatment an effective amount of at least one compound as defined in any one of clauses 1-71 or a pharmaceutically acceptable salt thereof.

## Claims

1. A compound of Formula I or a pharmaceutically acceptable salt: 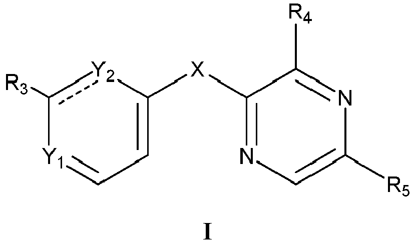
X is absent, O, S, SO, S(O)₂, C(O), C(O)R₁₁, CR₁₁R₁₂, or -NR₁₁; and each R₁₁ and R₁₂ is independently -H, halogen, -NH₂, -CN, -OH, -NO₂, carbonyl, =O, oxo, carboxyl, substituted or unsubstituted C₁₋₆alkoxy, or substituted or unsubstituted C₁₋₆alkyl;
Y₁ is N or CR₁;
Y₂ is N or CR₂;
each R₁ and R₂ is independently -H, halogen, -CN, -OH, -NH₂, -N₃, -NO₂, substituted or unsubstituted C₁₋₆alkoxy, or substituted or unsubstituted C₁₋₆alkyl; or
R₁ combines with R₃, or R₂ combines with R₃, to form a 5-10 member heteroaryl, 5-10 member carbocyclic or 5-10 member heterocyclic ring, wherein each of the ring systems is independently optionally substituted with halogen, -CN, -OH, -NR₈R₉, -N₃, -NO₂, carbonyl, =O, oxo, substituted or unsubstituted C₁₋₆alkyl, substituted or unsubstituted C₁₋₆alkoxy, or C(O)R₈; or
R₃ is -H, halogen, -CN, -OH, -N₃, -NO₂, -NR₈R₉, -N(R₈)(CH₂)ₚNR₈R₉, -N(R₈)(CH₂)ₚR₈, -N(R₈)GₚR₈, -N(R₈)GₚNR₈R₉, -N(R₈)(C=O)_{q}R₈, -N(R₈)(C=O)₄NR₈R₉, -N(R₈)(C=O)_{q}GₚR₈, - N(R₈)(C=O)_{q}GₚNR₈R₉, -N(R₈)(C=O)_{q}Gₚ(C=O)_{q}R₈, -N(R₈)(C=O)_{q}G_{P}(C=O)_{q}NR₈R₉, - N(R₈)(C=O)qN(R₈)(C=O)_{q}R₈, -N(R₈)(C=O)_{q}N(R₈) (C=O)_{q}NR₈R₉, - N(R₈)(C=O)_{q}N(R₈)G_{q}(C=O)ₚR₈, -N(R₈)(C=O)_{q}N(R₈)Gₚ(C=O)_{q}NR₈R₉, C(O)_{q}R₈, C(O)OR₈, C(O)NH₂, C(O)NHR₈, C(O)NR₈R₉, C₁₋₆alkyl, C₆₋₁₀aryl, arylalkyl, alkoxy, heteroaryl, heterocyclic, or carbocyclic; and each of which may be optionally substituted; and each p and q is independently 0, 1, 2 or 3;
each G is independently C₆₋₁₀aryl, C₃₋₈carbocyclic or C₅₋₁₀heteroaryl; and each of which may be optionally substituted;
R₄ is -H, halogen, -CN, -OH, -NR₈R₉, -N₃, -NO₂, substituted or unsubstituted C₁₋₆alkoxy, substituted or unsubstituted C₁₋₆alkyl, C₅₋₁₈heterocyclic or C₅₋₁₈carbocyclic; wherein each of the ring systems is independently optionally substituted with halogen, -CN, -OH, - NO₂, carbonyl, =O, oxo, substituted or unsubstituted C₁₋₆alkyl, substituted or unsubstituted C₁₋₆alkoxy, -NR₈R₉ or -CH₂NR₈R₉;
R₅ is -H, halogen, -CN, -OH, -NR₈R₉, -N₃, -NO₂, C₁₋₆alkyl, C₁₋₆alkoxy, C₆₋₁₀aryl, C₆₋₁₀arylalkyl, C₆₋₁₀heteroaryl, C₅₋₁₈heterocyclic or C₅₋₁₈carbocyclic; and each of which is independently optionally substituted;
each R₅ and R₉ is independently -H, halogen, -CN, -OH, -N₃, -NO₂, C₁₋₆alkyl, C₁₋₆alkoxy, C₂₋₆alkenyl, NH(C₁₋₆alkyl), N(C₁₋₆alkyl)₂, C₅₋₁₀heterocyclic or C₅₋₁₀carbocyclic; and each of which may be independently optionally substituted.

2. The compound of claim 1, wherein R₁ is -H, -F, -Cl, -Br, -I, -CN, -OH, -NH₂, substituted or unsubstituted C₁₋₃alkoxy, or substituted or unsubstituted C₁₋₃alkyl; preferably wherein R₁ is -H, -F, -Cl, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.

3. The compound of claim 1 or 2, wherein R₂ is -H, -F, -Cl, -Br, -I, -CN, -OH, -NH₂, substituted or unsubstituted C₁₋₃alkoxy, or substituted or unsubstituted C₁₋₃alkyl; preferably wherein R₂ is -H, -F, -Cl, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with halogen, OH or NH₂.

4. The compound of any one of claims 1-3, wherein R₃ is:
A) -H, -F, -Cl, -Br, -CN, -OH, -NO₂, -NR₈R₉, -N(R₈)(CH₂)ₚNR₈R₉, -N(R₈)(CH₂)ₚR₈,-N(R₈)GₚR₈, -N(R₈)GₚNR₈R₉, -N(R₈)(C=O)_{q}R₈, -N(R₈)(C=O)₄NR₈R₉, -N(P₈)(C=O)_{q}GₚR₈, - N(R₈)(C=O)_{q}GₚNR₈R₉, -N(R₈)(C=O)_{q}Gₚ(C=O)_{q}R₈, -N(R₈)(C=O)_{q}G_{P}(C=O)_{q}NR₈R₉, - N(R₈)(C=O)_{q}N(R₈)(C=O)_{q}R₈, -N(R₈)(C=O)_{q}N(R₈) (C=O)_{q}NR₈R₉, - N(R₈)(C=O)_{q}N(R₈)G_{q}(C=O)ₚR₈, -N(R₈)(C=O)_{q}N(R₈)Gₚ(C=O)_{q}NR₈R₉, C(O)₄R₈, C(O)OR₈, C(O)NH₂, C(O)NER₈, C(O)NR₈R₉, C₁₋₆alkyl or C₆₋₁₀aryl; and each of which may be optionally substituted with halogen, -CN, -OH, -NH₂, -N₃, -NO₂, substituted or unsubstituted C₁₋₆alkyl, or substituted or unsubstituted C₁₋₆alkoxy;
B) -H, -F, -Cl, -Br, -NR₈R₉, -N(R₈)(CH₂)ₚNR₈R₉, -N(R₈)(CH₂)ₚR₈,-N(R₈)GₚR₈, - N(R₈)GₚNR₈R₉, -N(R₈)(C=O)_{q}R₈, -N(R₈)(C=O)₄NR₈R₉, -N(R₈)(C=O)_{q}GₚR₈, - N(R₈)(C=O)_{q}GₚNR₈R₉, -N(R₈)(C=O)_{q}Gₚ(C=O)_{q}P₈, -N(R₈)(C=O)_{q}G_{P}(C=O)_{q}NR₈R₉, - N(R₈)(C=O)_{q}N(R₈)(C=O)_{q}R₈, -N(R₈)(C=O)_{q}N(R₈) (C=O)_{q}NR₈R₉, - N(R₈)(C=O)_{q}N(R₈)G_{q}(C=O)ₚR₈, -N(R₈)(C=O)_{q}N(R₈)Gₚ(C=O)_{q}NR₈R₉ or C(O)₄R₈, and each of which may be optionally substituted with -F, -Cl, -Br, -I, -CN, -OH, -NH₂, -N₃, -NO₂, substituted or unsubstituted C₁₋₃alkyl, or substituted or unsubstituted C₁₋₃alkoxy;
C) -H, -NR₈R₉, -N(R₈)(C=O)_{q}R₈, -N(R₈)(C=O)_{q}NR₈R₉, -N(R₈)(C=O)_{q}GₚR₈, - N(R₈)(C=O)_{q}Gₚ(C=O)_{q}NR₈R₉, -N(R₈)(C=O)_{q}N(P₈)Gₚ(C=O)_{q}NR₈R₉ or C(O)_{q}R₈; and each of which may be optionally substituted with -F, -Cl, -Br, -NHmethyl, -NHethyl, -NHpropyl, - NHisopropyl, -NHOCH₃, -NHOCH₂CH₃, -NHCH₂OCH₃, -NHOCH₂CH₂CH₃, - NHCH₂OCH₂CH₃, -NHCH₂CH₂OCH₃, -NHOCH(CH₃)₂, -NHCH(OCH₃)₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with -F, -Cl, -Br or -I; or
D) -NH₂,

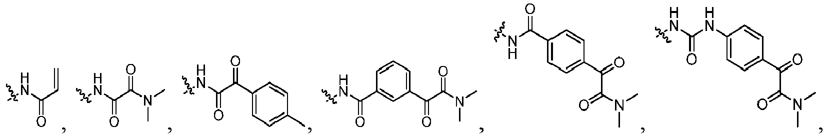

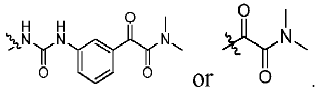

5. The compound of any one of claims 1-4, wherein each G is independently:
A) 6-membered aryl, 7-membered aryl, 8-membered aryl, 3-membered carbocyclic, 4-membered carbocyclic, 5-membered carbocyclic, 6-membered carbocyclic or 7-membered carbocyclic; and each of which may be optionally substituted with halogen, -CN, -OH, -NH₂, -N₃, -NO₂, substituted or unsubstituted C₁₋₆alkyl, or substituted or unsubstituted C₁₋₆alkoxy;
B) 6-membered aryl, 7-membered aryl, 8-membered aryl, 3-membered carbocyclic, 4-membered carbocyclic, 5-membered carbocyclic or 6-membered carbocyclic; and each of which may be optionally substituted with -F, -Cl, -Br, -CN, -OH, -NH₂, -NO₂, substituted or unsubstituted C₁₋₃alkyl, or substituted or unsubstituted C₁₋₃alkoxy;
C) phenyl, and which may be optionally substituted with -F, -Cl, -OH, -NH₂, methyl, ethyl, propyl, isopropyl, methoxy, ethoxy, propoxy or isopropoxy; and each methyl, ethyl, propyl, isopropyl, methoxy, ethoxy, propoxy or isopropoxy is independently optionally substituted with -F, -Cl, -Br or -I; or
D) 5-membered heteroaryl, 6-membered heteroaryl, 7-membered heteroaryl or 8-membered heteroaryl; and each of which contains 1, 2, 3 or 4 heteroatoms select from N, O or S, and may be optionally substituted with halogen, -CN, -OH, -NH₂, -N₃, -NO₂, substituted or unsubstituted C₁₋₆alkyl, or substituted or unsubstituted C₁₋₆alkoxy.

6. The compound of any one of claims 1-5, wherein p is 0 or 1.

7. The compound of any one of claims 1-6, wherein q is 1 or 2.

8. The compound of any one of claims 1-7, wherein R₄ is:
A) -H, -F, -Cl, -Br, -CN, -OH, -NR₈R₉, substituted or unsubstituted C₁₋₆alkyl, substituted or unsubstituted C₁₋₆alkoxy, C₅₋₁₈heterocyclic or C₅₋₁₀carbocyclic, wherein each of the ring systems is optionally substituted with -F, -Cl, -Br, -CN, -OH, -NO₂, carbonyl, =O, oxo, substituted or unsubstituted C₁₋₆alkyl, substituted or unsubstituted C₁₋₆alkoxy, -NH₂, -NH-C₁₋₆alkyl, -NH-C₁₋₆alkoxy, -C₁₋₆alkylene-NH₂ or -C₁₋₆alkylene-NH-C₁₋₆alkyl;
B) H, -F, -Cl, -CN, -OH, -NH₂, substituted or unsubstituted C₁₋₃alkyl, substituted or unsubstituted C₁₋₃alkoxy, 5-membered heterocyclic containing 1, 2 or 3 heteroatoms select from N or O, 6-membered heterocyclic containing 1, 2 or 3 heteroatoms select from N or O, 5-membered carbocyclic or 6-membered carbocyclic; wherein each of the ring systems is optionally substituted with -F, -Cl, -Br, -CN, -OH, carbonyl, =O, oxo, substituted or unsubstituted C₁₋₃alkyl, substituted or unsubstituted C₁₋₃alkoxy, -NH₂, -NH-C₁₋₃alkyl, -NH-C₁₋₃alkoxy, -C₁₋₃alkylene-NH₂ or -C₁₋₃alkylene-NH-C₁₋₃alkyl; or
C) -Cl, -NH₂, methyl or piperidinyl, wherein the ring system is optionally substituted with methyl, -NH₂ or -CH₂NH₂.

9. The compound of any one of claims 1-8, wherein R₅ is:
A) -H, -F, -Cl, -Br, -I, -NR₈R₉, C₁₋₃alkyl, C₁₋₃alkoxy, C₆₋₉aryl, C₆₋₉arylalkyl, C₆₋₉heteroaryl, C₆₋₁₇heterocyclic or C₆₋₁₇carbocyclic, wherein each of which is independently optionally substituted with halogen, -CN, -OH, -N₃, -NO₂, -NH₂, carbonyl, =O, oxo, substituted or unsubstituted C₁₋₆alkyl, substituted or unsubstituted C₁₋₆alkoxy, substituted or unsubstituted (CH₂)ₖNR₈R₉, substituted or unsubstituted (CH₂)ₖNHC(O)OR₈ or C(O)R₈; and k is 0, 1 or 2; optionally wherein the C₆₋₉ heteroaryl contains 1, 2, 3 or 4 heteroatoms select from N, O or S, and the C₆₋₉heteroaryl is 6-membered heteroaryl, 7-membered heteroaryl, 8-membered heteroaryl or 9-membered heteroaryl; the C₆₋₁₇heterocyclic contains 1, 2, 3, 4, 5, or 6 heteroatoms select from N, O, or S, and the C₆₋₁₇heterocyclic is 6-membered heterocyclic, 7-membered heterocyclic, 8-membered heterocyclic, 9-membered heterocyclic, 10-membered heterocyclic, 11-membered heterocyclic, 12-membered heterocyclic, 13-membered heterocyclic, 14-membered heterocyclic, 15-membered heterocyclic, 16-membered heterocyclic or 17-membered heterocyclic; or
B) -F, -Cl, -Br, -NR₈R₉, methyl, ethyl, propyl, isopropyl, methoxy, ethoxy, propoxy, isopropoxy, 6-membered aryl, 7-membered aryl, 8-membered aryl, 9-membered aryl, 6-membered arylalkyl, 7-membered arylalkyl, 8-membered arylalkyl, 9-membered arylalkyl, 6-membered heteroaryl, 7-membered heteroaryl, 8-membered heteroaryl, 9-membered heteroaryl, 6-membered heterocyclic, 7-membered heterocyclic, 8-membered heterocyclic, 9-membered heterocyclic, 10-membered heterocyclic, 11-membered heterocyclic, 12-membered heterocyclic, 13-membered heterocyclic, 14-membered heterocyclic, 15-membered heterocyclic, 16-membered heterocyclic, 6-membered carbocyclic, 7-membered carbocyclic, 8-membered carbocyclic, 9-membered carbocyclic, 10-membered carbocyclic, 11-membered carbocyclic, 12-membered carbocyclic, 13-membered carbocyclic, 14-membered carbocyclic, 15-membered carbocyclic or 16-membered carbocyclic; and each heteroaryl contains 1, 2 or 3 heteroatoms select from N, O or S, and each heterocyclic contains 1, 2, 3, or 4 heteroatoms select from N, O or S; wherein each of which is independently optionally substituted with -F, -Cl, -Br, -I, -CN, -OH, -NO₂, -NH₂, carbonyl, =O, oxo, substituted or unsubstituted C₁₋₃alkyl, substituted or unsubstituted C₁₋₃alkoxy, substituted or unsubstituted (CH₂)ₖNR₈R₉, substituted or unsubstituted (CH₂)ₖNHC(O)OR₈, or substituted or unsubstituted C(O)R₈; and k is 0, 1 or 2.

10. The compound of any one of claims 1-9, wherein X is O, S or absent.

11. The compound of any one of claims 1-10, wherein Y₁ is N and Y₂ is CR₂.

12. The compound of any one of claims 1-11, wherein Y₂ is N and Y₁ is CRi.

13. The compound of any one of claims 1-12, wherein R₄ is -NH₂.

14. The compound of any one of claims 1-10, wherein each R₈ and R₉ is independently - H, halogen, CN, -OH, -NO₂, -NH₂, -C₁₋₆alkylene-NH₂, -C₁₋₆alkylene-NH-C₁₋₆alkyl, -C₁-₆alkylene-N(C₁₋₆alkyl)₂, -NHBoc, -CH₂NHBoc; -NH-C₁₋₆alkyl, -N(C₁₋₆alkyl)₂, -NH-C₁₋₆alkoxy, -N(C₁₋₆alkoxy)₂, C₁₋₆alkyl, C₁₋₆alkoxy, C₂₋₆alkenyl, C₂₋₆alkylnyl, C₅₋₁₀heterocyclic or C₅₋₁₀carbocyclic; each of which may be optionally substituted;
optionally wherein each R₅ and R₉ is independently -H, -F, -Cl, -CN, -OH, -NO₂, -NH₂, -C₁₋₃alkylene-NH₂, -C₁₋₃alkylene-NH-C₁₋₃alkyl, -C₁₋₃alkylene-N(C₁₋₃alkyl)₂, -NHBoc, - CH₂NHBoc; -NH-C₁₋₃alkyl, -N(C₁₋₃alkyl)₂, -NH-C₁₋₃alkoxy, -N(C₁₋₃alkoxy)₂, C₁₋₄alkyl, C₁₋₃alkoxy, C₂₋₃alkenyl, C₂₋₃alkylnyl, 5-membered heterocyclic, 6-membered heterocyclic, 7-membered heterocyclic, 8-membered heterocyclic, 9-membered heterocyclic, 10-membered heterocyclic, 5-membered carbocyclic, 6-membered carbocyclic, 7-membered carbocyclic, 8-membered carbocyclic, 9-membered carbocyclic, 10-membered carbocyclic, 5-membered carbocyclic, 6-membered carbocyclic, 7-membered carbocyclic, 8-membered carbocyclic, 9-membered carbocyclic, or 10-membered carbocyclic; and each of which may be independently optionally substituted with halogen, -CN, -OH, -NH₂, -N₃, -NO₂, -C₁₋₆alkylene-NH₂, -C₁₋₆alkylene-NH-C₁₋₆alkyl, -C₁₋₆alkylene-N(C₁₋₆alkyl)₂, -NHBoc, - CH₂NHBoc; -NH-C₁₋₆alkyl, -N(C₁₋₆alkyl)₂, -NH-C₁₋₆alkoxy, -N(C₁₋₆alkoxy)₂, substituted or unsubstituted C₁₋₆alkyl, or substituted or unsubstituted C₁₋₆alkoxy;and each heterocyclic contains 1, 2, 3 or 4 heteroatoms select from N, O or S.
